# EUROPEAN PATENT SPECIFICATION

(11) **EP 0 692 025 B1**
(45) Date of publication and mention of the grant of the patent: **17.10.2001**
(21) Application number: 94912292.3
(22) Date of filing: 23.03.1994
(51) Int. Cl.: C12N 15/10, C12N 15/81, C12Q 1/68, C12N 1/19, C07K 14/39

(54) **YEAST CELLS ENGINEERED TO PRODUCE PHEROMONE SYSTEM PROTEIN SURROGATES, AND USES THEREFOR**
HEFE ZELLEN SO KONSTRUIERT, DASS SIE PROTEINSURROGATE DES PHEROMENSYSTEMS PRODUZIEREN UND ANWENDUNGEN DAFUER
CELLULES DE LEVURE TRAITEES POUR PRODUIRE DES SUBSTITUTS DE PROTEINES DU SYSTEME DE PHEROMONES, ET LEURS EMPLOIS

(30) Priority: 31.03.1993 US 41431; 31.01.1994 US 190328
(43) Date of publication of application: 17.01.1996
(60) Divisional application: 98202997.7
(73) Proprietor: Cadus Pharmaceutical Corporation, New York, NY 10128 (US)
(72) Inventor: FOWLKES, Dana, Merriman 90 Green Street, New York, NY 10012 (US); BROACH, Jim 360 East 88th Street, New York, NY 10128 (US); MANFREDI, John 666 Greenwich Street, New York, NY 10014 (US); KLEIN, Christine 666 Greenwich Street, New York, NY 10014 (US); MURPHY, Andrew, J., Montclair, NJ 07043 (US); PAUL, Jeremy, Palisades, NY 10964 (US); TRUEHEART, Joshua, South Nyack, NY 10960 (US)
(74) Representative: Price, Vincent Andrew
(86) International application number: US9403143
(87) International publication number: WO9423025

(56) References cited:
- WO-A-92/05244
- SCIENCE vol. 250 , October 1990 , LANCASTER, PA US pages 121 - 123 KLIM KING ET AL. 'Control of yeast mating signal transduction by a mammalian beta2-adrenergic receptor and Gs alpha subunit'
- CELL vol. 66 , 20 September 1991 , CAMBRIDGE, NA US pages 1197 - 1206 D. J. LEW ET AL. 'Isolation of three novel human cyclins by rescue of G1 cyclin (Cln) function in yeast'
- CELL vol. 65 , 17 May 1991 , CAMBRIDGE, NA US pages 691 - 699 YUE XIONG ET AL. 'Human D-type cyclin'
- PROCEEDINGS OF THE NATIONAL ACADEMY OF SCIENCES OF USA vol. 89 , October 1992 , WASHINGTON US pages 9410 - 9414 M. WITHEWAY ET AL. 'Dominant negative selection of heterologous genes: Isolation of Candida albicans genes that interfere with Saccharomyces cerevisiae mating factor-induced cell cycle arrest'
- JOURNAL OF CELLULAR BIOCHEMISTRY vol. 18B , February 1994 page 224 J. MANFREDI ET AL. 'Autocrine stimulation of yeast through human G-coupled receptors'
- A. KOFF ET AL.,: 'HUMAN CYCLIN E, A NEW CYCLIN THAT INTERACTS WITH TWO MEMBERS OF THE CDC2 GENE FAMILY' CELL vol. 66, 1991, pages 1217 - 1228
- D.A. HUGHES ET AL.,: 'COMPLEMENTATION OF BYR1 IN FISSION YEAST BY MAMMALIAN MAP KINASE REQUIRES COEXPRESSION OF RAF KINASE' NATURE vol. 364, 1993, pages 394 - 352

## Description

### BACKGROUND OF THE INVENTION

### Field of the Invention

The present invention relates to the screening of drugs, especially random peptides, in yeast cells for the ability to interact with proteins involved in the post-translational modification, transport of and response to yeast pheromones or substitutes therefor.

### Description of the Background Art

### Drug Screening.

The identification of biological activity in new molecules has historically been accomplished through the use of in *vitro* assays or whole animals. Intact biological entities, either cells or whole organisms, have been used to screen for antibacterial, anti-fungal, anti-parasitic and anti-viral agents in *vitro.* Cultured mammalian cells have also been used in screens designed to detect potential therapeutic compounds. A variety of bioassay endpoints are exploited in mammalian cell screens including the stimulation of growth or differentiation of cells, changes in cell motility, the production of particular metabolites, the expression of specific proteins within cells, altered protein function, and altered conductance properties. Cytotoxic compounds used in cancer chemotherapy have been identified through their ability to inhibit the growth of tumor cells in *vitro* and *in vivo*. In addition to cultures of dispersed cells, whole tissues have served in bioassays, as in those based on the contractility of muscle.

*In vitro* testing is a preferred methodology in that it permits the design of high-throughput screens: small quantities of large numbers of compounds can be tested in a short period of time and at low expense. Optimally, animals are reserved for the latter stages of compound evaluation and are not used in the discovery phase; the use of whole animals is labor-intensive and extremely expensive.

Microorganisms, to a much greater extent than mammalian cells and tissues, can be easily exploited for use in rapid drug screens. Yeast provide a particularly attractive test system; extensive analysis of this organism has revealed the conservation of structure and function of a variety of proteins active in basic cellular processes in both yeast and higher eukaryotes.

The search for agonists and antagonists of cellular receptors has been an intense area of research aimed at drug discovery due to the elegant specificity of these molecular targets. Drug screening has been carried out using whole cells expressing functional receptors and, recently, binding assays employing membrane fractions or purified receptors have been designed to screen compound libraries for competitive ligands. Duke University, WO92/05244 (April 2, 1992) describes the expression of mammalian G protein-coupled receptors in yeast and a means of identifying agonists and antagonists of those receptors using that organism.

In addition, yeast are, of course, used in the discovery of antifungal compounds; Etienne et al. (1990) describe the use of *Saccharomyces cerevisiae* mutant strains, made highly sensitive to a large range of antibiotics, for the rapid detection of antifungals.

### Yeast Pheromone System Proteins and Their Metabolic Function

Haploid yeast cells are able not only to grow vegetatively, but also to mate to form a diploid cell. The two mating types ("sexes") of haploid cells are designated **a** and α. The **a** cells produce the dodecapeptide **a**-factor, and the α cells, the tridecapeptide α-factor. Because **a**-factor and α-factor elicit a mating response in the yeast cell of the opposite "sex", they are called "pheromones". These pheromones, as well as other proteins specifically involved in the production or transport of, or response to, pheromones, are considered "pheromone system proteins".

The gene encoding **a**-factor pheromone, like the α-factor receptor gene, is an **a** cell-specific gene; **a** cell-specific genes are only expressed in **a** cells. The gene encoding α-factor pheromone, like the **a**-factor receptor gene, is an α cell-specific gene; α cell-specific genes are only expressed in α cells. Other yeast genes belong to a haploid-specific gene set and are expressed in haploid cells **(a** cells or α cells) but not in diploid **(a**/α) cells. In addition, there exists a diploid cell-specific gene set, including those genes involved in sporulation.

In eukaryotic cells, RNA polymerase II promoters contain a specific sequence (the TATA box) to which the transcription factor TFIID (TATA binding protein or TBP) binds. An active transcription initiation complex includes TFIID, accessory initiation proteins, and RNA Pol II. As in higher eukaryotic cells, the TATA box is an essential control sequence in yeast promoters. Yeast TATA-box-binding protein (TBP) was identified by its ability to substitute in function for mammalian TFIID [Buratowski et al., Nature 334, 37 (1988); Cavallini et al., Nature 334, 77 (1988)]. With only a few apparent exceptions [transcription of some glycolytic enzyme genes, see Struhl, Mol. Cell. Biol. 6, 3847 (1986) and Ogden et al., Mol. Cell Biol. 6, 4335 (1986)] transcription of yeast genes requires the proximal TATA box element and TFIID binding for initiation of transcription. Also required for efficient transcription are gene-specific activator proteins; the precise mechanism whereby these gene-specific regulatory proteins influence transcription has not been completely elucidated.

MCM1p (encoded in the MCM1 gene) is a non-cell-type-specific transcription factor in yeast. MCM1p acts alone or in concert with other regulatory proteins to control expression of **a**- and α- cell specific genes. Yeast mating type loci encode the regulatory proteins that contribute to the control of cell type-specific expression. These proteins are Mat**a**1p (encoded by the MAT**a** gene) and Matα1p and Matα2p (encoded by the MATα locus). MCM1p activates transcription of **a**-specific genes by binding to an upstream activation sequence (UAS) located in the control region of **a**-specific genes. Matα1p and MCM1p interact to enhance each other's binding to specific UAS binding sites to activate α-cell-specific gene transcription in α-cells. Matα2p associates with MCM1p to repress **a**-specific gene transcription in α-cells. In diploid (**a**/α) cells, Matα1p and Matα2p associate to repress the transcription of haploid-specific genes. The Matα1p/Matα2p regulatory entity is found only in diploid cells.

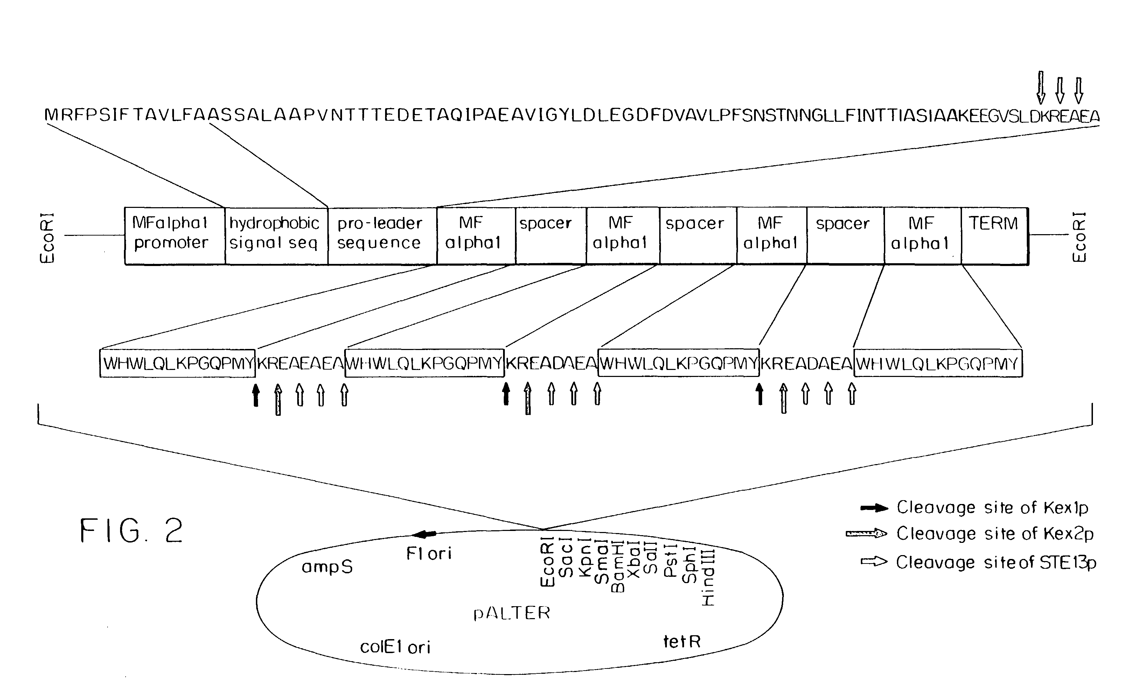

Yeast contain two genes encoding the α-factor pheromone, MFα1 and MFα2. Analysis of yeast bearing mutations in these sequences indicates that MFα1 gives rise to the majority of α-factor produced by cells. Expression occurs at a higher level from MFα1 than from MFα2 (Kurjan, Mol. Cell. Biol. 5, 787 (1985). The MFα1 gene of yeast encodes a 165 aa precursor protein containing an 85 aa leader sequence at the N-terminus. The leader includes a 19 aa signal sequence and a 66 aa sequence which contains sites for the addition of three oligosaccharide side chains (Kurjan and Herskowitz, Cell 39, 933 (1982); Singh et al. Nuc. Acids Res. 11, 4049 (1983); Julius et al. Cell 36, 309 (1984). Four tandem copies of the 13 aa α-factor are present in the C-terminal portion of the precursor; 6-8 aa spacer peptides precede the α-factor sequences (see Figure 2).

After translocation of the nascent α-factor polypeptide to the ER, the signal sequence is cleaved from the precursor protein to yield pro-α-factor (Waters et al. J. Biol. Chem. 263, 6209 (1988). The core N-linked carbohydrate is added to three sites in the N-terminus of pro-α-factor (Emter et al. Biochem. Biophys. Res. Commun. 116, 822 (1983); Julius et al. Cell 36, 309 (1984); Julius et al. Cell 37, 1075 (1984). Additional glycosylation occurs in the Golgi prior to cleavage of pro-α-factor by the KEX2 endopeptidase. This enzyme cleaves within each of the spacer repeats leaving a Lys-Arg sequence attached to the C-terminus of α-factor peptide (Julius et al. Cell 37, 1075 (1984). The Lys-Arg sequence is removed by the action of the KEX-1 protease (Dmochowska et al. Cell 50, 573 (1987). The additional spacer residues present at the N-terminus of α-factor peptide are removed by the dipeptidyl aminopeptidase encoded by STE13 (Julius et al. Cell 32, 839 (1983). Four α-factor peptides are released from each precursor protein via the proteolytic processing outlined above and the mature α-factor is secreted from the cell.

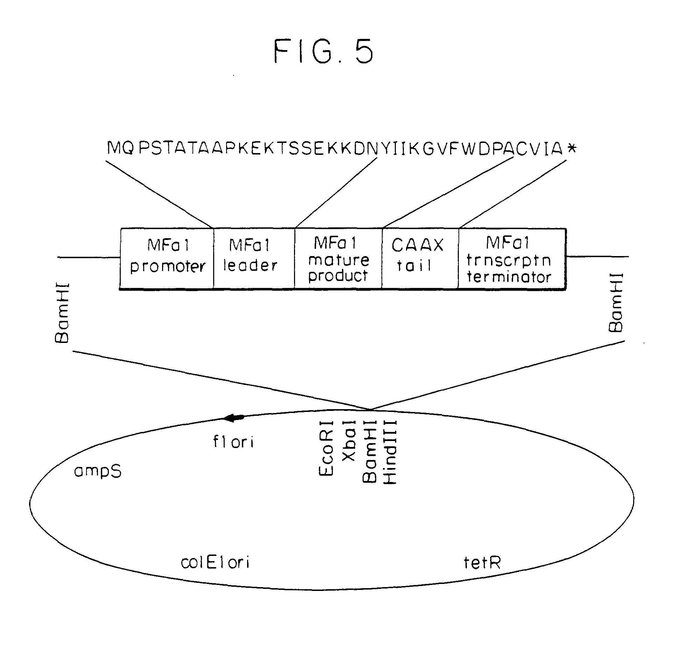

Precursors of the 12 aa mature **a**-factor peptide are encoded in the MF**a**1 and MF**a**2 genes and are 36 aa and 38 aa residues, respectively (for schematic of MF**a**1 gene see Figure 5). The precursors contain one copy of **a**-factor and the products of the two genes differ in sequence at one amino acid. The two forms of **a-**factor are produced in equal amounts by **a** cells (Manney et al. in *Sexual interactions in eukaryotic microbes,* p21, Academic Press, New York (1981).

Processing of **a**-factor entails a process that differs in every detail from that of α-factor. The processing of **a**-factor begins in the cytosol and involves the farnesylation of the C-terminal cysteine residue near the carboxyl terminus (-CVIA) by a farnesyl transferase (Schafer et al. Science 245, 379 (1989); Schafer et al. Science 249, 1133 (1990). The α and β subunits of the farnesyl transferase are encoded by the RAM2 and RAM1 genes, respectively (He et al. Proc. Natl. Acad. Sci. 88, 11373 (1991). Subsequent to farnesylation is the proteolytic removal of the three amino acids that are C-terminal to the modified cysteine by a membrane-bound endoprotease. Next, the carboxy-terminal farnesylated cysteine residue is modified further: the carboxyl group is methylated by the product of the STE14 gene. STE14p is a membrane-bound S-farnesyl-cysteine carboxyl methyl transferase (Hrycyna et al. EMBO. J. 10, 1699 (1991). The mechanisms of the N-terminal processing of **a**-factor have not been elucidated. After processing of the precursors is complete, mature a-factor is transported to the extracellular space by the product of the STE6 gene (Kuchler et. al. EMBO J. 8, 3973 (1989), an ATP-binding cassette (ABC) transporter.

In normal S. cerevisiae (budding yeast) **a** cells, the α-factor binds the G protein-coupled membrane receptor STE2. The G protein dissociates into the G_{α} and G_{βγ} subunits, and the G_{βγ} binds an unidentified effector, which in turn activates a number of genes. STE20, a kinase, activates STE5, a protein of unknown function. STES activates STE11 kinase, which stimulates STE7 kinase, which induces the KSS1 and/or FUS3 kinases. These switch on expression of the transcription factor STE12. STE12 stimulates expression of a wide variety of genes involved in mating, including FUS1 (cell fusion), FAR1 (cell-cycle arrest), STE2 (the receptor), MFA1 (the pheromone), SST2 (recovery), KAR3 (nuclear fusion) and STE6 (pheromone secretion). Other genes activated by the pathway are CHS1, AGα1, and KAR3. The multiply tandem sequence TGAAACA has been recognized as a "pheromone response element" found in the 5'-flanking regions of many of the genes of this pathway.

One of the responses to mating pheromone is the transient arrest of the yeast cell in the G1 phase of the cell cycle. This requires that all three G1 cyclins (CLN1, CLN2, CLN3) be inactivated. It is believed that FUS3 inactivates CLN3, and FAR1 inhibits CLN2. (The product responsible for inactivating CLN1 is unknown).

The growth arrest is terminated by a number of different mechanisms. First, the α-factor receptor is internalized following binding of the pheromone, resulting in a transient decrease in the number of pheromone binding sites. Second, the C-terminal tail of the receptor is phosphorylated consequent to ligand binding, resulting in uncoupling of the receptor from the transducing G proteins. Third, pheromone-induced increases in expression of GPA1p (the Gα-subunit of the heterotrimeric G protein) increase the level of the α subunit relative to the G_{β} and G_{γ} subunits, resulting in reduction in the level of free G_{βγ} and consequent inactivation of the pheromone response pathway. Additional mechanisms include induction of the expression of SST2 and BAR1 and phosphorylation of the α subunit (perhaps by SVG1).

Signaling is inhibited by expression of a number of genes, including CDC36, CDC39, CDC72, CDC73, and SRM1. Inactivation of these genes leads to activation of the signaling pathway.

A similar pheromone signaling pathway may be discerned in α cells, but the nomenclature is different in some cases (e.g., STE3 instead of STE2).

Other yeast also have G protein-mediated mating factor response pathways. For example, in the fission yeast S. pombe, the M factor binds the MAP3 receptor, or the P-factor the MAM2 receptor. The dissociation of the G protein activates a kinase cascade (BYR2, BYR1, SPK1), which in turn stimulates a transcription factor (STE11). However, in S. combe, the Gα subunit transmits the signal, and there are of course other differences in detail.

### Pheromone Pathway Mutants

The effects of spontaneous and induced mutations in pheromone pathway genes have been studied. These include the α-factor (MFα1 and MFα2) genes, see Kurjan, Mol. Cell. Biol., 5:787 (1985); the **a**-factor (MF**a**1 and MF**a**2) genes, see Michaelis and Herskowitz, Mol. Cell. Biol. 8:1309 (1988); the pheromone receptor (STE2 and STE3) genes, see Mackay and Manney, Genetics, 76:273 (1974), Hartwell, J. Cell. Biol., 85:811 (1980), Hagen, et al., P.N.A.S. (USA), 83:1418 (1986); the FAR1 gene, see Chang and Herskowitz, Cell, 63:999 (1990); and the SST2 gene, see Chan and Otte, Mol. Cell. Biol., 2:11 (1982).

### Expression of Foreign Proteins in Yeast Cells

A wide variety of foreign proteins have been produced in S. *cerevisiae,* either solely in the yeast cytoplasm or through exploitation of the yeast secretory pathway (Kingsman et al. TIBTECH 5, 53 (1987). These proteins include, as examples, insulin-like growth factor receptor (Steube et al. Eur. J. Biochem. 198, 651 (1991), influenza virus hemagglutinin (Jabbar et al. Proc. Natl. Acad. Sci. 82, 2019 (1985), rat liver cytochrome P-450 (Oeda et al. DNA 4, 203 (1985) and functional mammalian antibodies (Wood et al. Nature 314, 446 (1985). Use of the yeast secretory pathway is preferred since it increases the likelihood of achieving faithful folding, glycosylation and stability of the foreign protein. Thus, expression of heterologous proteins in yeast often involves fusion of the signal sequences encoded in the genes of yeast secretory proteins (e.g., α-factor pheromone or the SUC2 [invertase] gene) to the coding region of foreign protein genes.

A number of yeast expression vectors have been designed to permit the constitutive or regulated expression of foreign proteins. Constitutive promoters are derived from highly expressed genes such as those encoding metabolic enzymes like phosphoglycerate kinase (PGK1) or alcohol dehydrogenase I (ADH1) and regulatable promoters have been derived from a number of genes including the galactokinase (GAL1) gene. In addition, supersecreting yeast mutants can be derived; these strains secrete mammalian proteins more efficiently and are used as "production" strains to generate large quantities of biologically active mammalian proteins in yeast (Moir and Davidow, Meth. in Enzymol. 194, 491 (1991).

A variety of heterologous proteins have been expressed in yeast cells as a means of generating the quantity of protein required for commercial use or for biochemical study (Kingsman et al. TIBTECH 5, 53 (1987). In addition, a number of mammalian proteins have been expressed in yeast in order to determine whether the proteins (1) will functionally substitute for cognate proteins normally expressed within that organism or (2) will interact with accessory yeast proteins to accomplish a specific function. Thus it has been determined that a human TBP with altered binding specificity will function to initiate transcription in yeast [Strubin and Struhl, Cell 68, 721 (1992)]. In addition, mammalian steroid hormone receptors [Metzger et al. (1988); Schena and Yamamoto (1988)] and human p53 [Scharer and Iggo, Nuc. Acids Res. 20, 1539 (1992)] were shown to activate transcription in yeast.

Expression in yeast of the *gag-pol* gene of HIV-1 results in the processing of the *gag* protein precursor to yield the products which normally arise within the virion; processing in yeast, as in the virus, is due to the action of the protease encoded within the *gag-pol* gene (Kramer et al. Science 231,1580 (1986).

A number of mammalian ABC transporters have been expressed in yeast to determine their ability to substitute for yeast Ste6p in the transport of pheromone. The mammalian proteins thus far tested include human Mdr1 (Kuchler and Thorner, Proc. Natl. Acad. Sci. 89, 2302 (1992)) and murine Mdr3 (Raymond et al. Science 256, 232 (1992), proteins involved in multidrug resistance; in addition, a chimeric protein containing human CFTR (cystic fibrosis transmembrane conductance regulator) and yeast STE6 sequence has been shown to transport **a**-factor pheromone in yeast (Teem et al. Cell 73, 335 (1993).

An **a** cell may be engineered to produce the **a**-factor receptor, and an α cell to make α-factor receptor. Nakayama, et al., EMBO J., 6:249-54 (1987); Bender and Sprague, Jr., Genetics 121: 463-76 (1989).

Heterologous G protein-coupled receptors have been functionally expressed in S. cerevisiae. Marsh and Hershkowitz, Cold Spring Harbor Symp., Quant. Biol., 53: 557-65 (1988) replaced the S. cerevisiae STE2 with its homologue from S. Kluyven. More dramatically, a mammalian beta-adrenergic receptor and Gα subunit have been expressed in yeast and found to control the yeast mating signal pathway. King, et al., Science, 250: 121-123 (1990).

Duke University, WO92/05244 (April 2, 1992) describes a transformed yeast cell which is incapable of producing a yeast G protein α subunit, but which has been engineered to produce both a mammalian G protein α subunit and a mammalian receptor which is "coupled to" (i.e., interacts with) the aforementioned mammalian G protein α subunit. Specifically, Duke reports expression of the human beta-2 adrenergic receptor (hβAR), a seven transmembrane receptor (STR), in yeast, under control of the GAL1 promoter, with the hβAR gene modified by replacing the first 63 base pairs of coding sequence with 11 base pairs of noncoding and 42 base pairs of coding sequence from the STE2 gene. (STE2 encodes the yeast α-factor receptor). Duke found that the modified hβAR was functionally integrated into the membrane, as shown by studies of the ability of isolated membranes to interact properly with various known agonists and antagonists of hβAR. The ligand binding affinity for yeast-expressed hβAR was said to be nearly identical to that observed for naturally produced hβAR.

Duke co-expressed a rat G protein α subunit in the same cells, yeast strain 8C, which lacks the cognate yeast protein. Ligand binding resulted in G protein-mediated signal transduction.

Duke teaches that these cells may be used in screening compounds for the ability to affect the rate of dissociation of Gα from G*β*γ in a cell. For this purpose, the cell further contains a pheromone-responsive promoter (e.g. BAR1 or FUS1), linked to an indicator gene (e.g. HIS3 or LacZ). The cells are placed in multi-titer plates, and different compounds are placed in each well. The colonies are then scored for expression of the indicator gene.

Duke's yeast cells do not, however, actually produce the compounds to be screened. As a result, only a relatively small number of compounds can be screened, since the scientist must ensure that a given group of cells is contacted with only a single, known compound.

Yeast have been engineered to express foreign polypeptide variants to be tested as potential antagonists of mammalian receptors. Libraries encoding mutant glucagon molecules were generated through random misincorporation of nucleotides during synthesis of oligonucleotides containing the coding sequence of mammalian glucagon. These libraries were expressed in yeast and culture broths from transformed cells were used in testing for antagonist activity on glucagon receptors present in rat hepatocyte membranes (Smith et al. 1993).

Drugs which overcome the multiple drug resistance (MDR) of cancer cells may be identified by using transformed yeast cells expressing P-glycoprotein (Suntory Ltd., patent application JP 2257873 entitled "Multiple drug resistance-relating gene-comprises P-glycoprotein accumulated in cell membrane part of transformed yeast"). The drugs were not produced by the yeast cells in question.

A yeast strain has been derived to allow the identification of inhibitors of protein farnesyltransferase which exhibit activity against mammalian Ras and which may therefore function as antitumor drugs (Hara et al. 1993).

### Genetic Markers in Yeast Strains

Yeast strains that are auxotrophic for histidine (HIS3) are known, see Struhl and Hill), Mol. Cell. Biol., 7:104 (1987); Fasullo and Davis, Mol. Cell. Biol., 8:4370 (1988). The HIS3 (imidazoleglycerol phosphate dehydratase) gene has been used as a selective marker in yeast. See Sikorski and Heiter, Genetics, 122:19 (1989); Struhl, et al., P.N.A.S. 76:1035 (1979); and, for FUS1-HIS3 fusions, see Stevenson, et al., Genes Dev., 6:1293 (1992).
*Peptide Libraries.* Peptide libraries are systems which simultaneously display, in a form which permits interaction with a target, a highly diverse and numerous collection of peptides. These peptides may be presented in solution (Houghten), or on beads (Lam), chips (Fodor), bacteria (Ladner USP 5,223,409), spores (Ladner USP '409), plasmids (Cull) or on phage (Scott, Devlin, Cwirla, Felici, Ladner '409). Many of these systems are limited in terms of the maximum length of the peptide or the composition of the peptide (e.g., Cys excluded). Steric factors, such as the proximity of a support, may interfere with binding. Usually, the screening is for binding in vitro to an artificially presented target, not for activation or inhibition of a cellular signal transduction pathway in a living cell. While a cell surface receptor may be used as a target, the screening will not reveal whether the binding of the peptide caused an allosteric change in the conformation of the receptor.

Ladner, USP 5,096,815 describes a method of identifying novel proteins or polypeptides with a desired DNA binding activity. Semi-random ("variegated") DNA encoding a large number of different potential binding proteins is introduced, in expressible form, into suitable host cells. The target DNA sequence is incorporated into a genetically engineered operon such that the binding of the protein or polypeptide will prevent expression of a gene product that is deleterious to the gene under selective conditions. Cells which survive the selective conditions are thus cells which express a protein which binds the target DNA. While it is taught that yeast cells may be used for testing, bacterial cells are preferred. The interactions between the protein and the target DNA occur only in the cell, not in the periplasm, and the target is a nucleic acid, and not a pheromone system protein surrogate.

Substitution of random peptide sequences for functional domains in cellular proteins permits some determination of the specific sequence requirements for the accomplishment of function. Though the details of the recognition phenomena which operate in the localization of proteins within cells remain largely unknown, the constraints on sequence variation of mitochondrial targeting sequences and protein secretion signal sequences have been elucidated using random peptides (Lemire et al., J. Biol. Chem. 264, 20206 (1989) and Kaiser et al. Science 235, 312 (1987), respectively).

### SUMMARY OF THE INVENTION

The invention is defined by the claims appended hereto.

In the present invention, a yeast cell is engineered to express an exogenous protein which is, however, capable of substituting for a yeast protein which is involved in the post-translational modification, transport, recognition or signal transduction of a yeast pheromone, sufficiently, to be able, at least under some circumstances, to carry out that role of the yeast protein. For the sake of convenience, these yeast proteins will be referred co as "pheromone system proteins" (PSP), and their cognate non-yeast proteins as PSP surrogates.

The pheromone system of a yeast cell is thus subverted so that the response of the cell to a yeast pheromone is at least partially determined by the activity of the surrogate PSP. In a preferred embodiment, the cognate yeast PSP is not produced in functional form, so that the response is essentially entirely dependent on the activity of the surrogate PSP.

Such yeast cells may be used to identify drugs which inhibit or activate, to a detectable degree, the ability of the surrogate to substitute for the cognate yeast PSP. To screen for an inhibitor, a normally functional surrogate is expressed, and the presence of an inhibitor is indicated by a depression of the cellular response. To screen for an activator, a surrogate functional in yeast, or one normally not functional in yeast but which is activatable (the latter is preferred, to minimize background) is used, and the activator is detected through its elevation of the cellular response.

In a preferred embodiment, the candidate drug is a peptide, and the yeast cell is engineered to express the candidate drug as well as the surrogate PSP.

Another consideration is that with wild-type yeast cells, to achieve pheromone secretion and response, both α and **a** cells must be provided. In some preferred embodiments, α cells are engineered to express α-factor receptor or **a** cells are engineered to express **a**-factor receptor. These modified cells may be considered "autocrine" because they are "self-stimulatory". Cells which express both a surrogate for the pheromone receptor, and a heterologous peptide agonist for the surrogate receptor, are also considered "autocrine", because they will respond to the co-produced agonist.

The classes of PSPs are numerous. However, some examples may be helpful.
*Farnesyltransferases and carboxymethyltransferases.* After expression, **a**-factor is farnesylated by RAM1p and RAM2p and carboxymethylated by Ste14p. These modifications are required for activity.

RAM1p and RAM2p are homologous to the subunits of the heterodimeric mammalian farnesyltransferase, which itself is necessary for membrane association of mammalian Ras proteins. If a yeast cell is engineered to express the mammalian farnesyltransferase, it may be used to identify drugs which interact with that enzyme by determining whether a functional **a**-factor is produced. Similarly, Ste14p is homologous to mammalian carboxymethyltransferases, which play regulatory roles in controlling the function of low molecular weight G proteins (Ras, Rho, Rab).
*Proteases.* The PSP may be a yeast protease, such as KEX2, STE13 or KEX1. Yeast α-factor pheromone is generated through the controlled and limited proteolysis of precursor proteins by these proteases. A yeast cell may be engineered to express an inactive precursor of yeast α-factor in which a peptide linker, corresponding to the cleavage site of a surrogate non-yeast protease, is incorporated so that cleavage will liberate mature α-factor (or its functional homologue). For example, the PSP surrogate may be HIV protease, with the cleavage site of HIV protease being substituted for the yeast protease cleavage sites in the α-factor precursor. The precursor and the HIV protease are co-expressed in the yeast cell. Proteolysis by HIV protease will give rise to production of mature α-factor and initiation of signal transduction. This system may be used to identify inhibitors of HIV protease.

Preferably, unlike yeast cells occurring in nature, the yeast cell is engineered not only to express the α-factor precursor, but also the α-factor receptor, so that a single haploid type of yeast is all that is required to conduct the assay.

*ABC Transporters.* Ste6 is the yeast ABC transporter necessary for the export of **a**-factor. The yeast cell is engineered to express both **a**-factor and a foreign ABC transporter. This transporter may be one which is not, by itself, able to transport **a**-factor, but which in the presence of a drug of interest, is capable of doing so, or it may be one which is already functional.

Preferably, the yeast cell is engineered to express not only **a**-factor, but also the **a**-factor receptor.

*G Protein-Coupled Receptors.* The PSP may be a yeast pheromone receptor. The surrogate is a non-yeast, G protein-coupled receptor. In order to achieve coupling to the pheromone signal transduction pathway, it may be necessary to provide a foreign or chimeric G_{α} or G_{βγ} subunit.

The engineered yeast cell may be used to screen for agonists as well as antagonists. When used to screen for agonists, it is preferable that the yeast pheromone not be produced in functional form.

*Protein Kinases.* The PSP may be a protein kinase, such as the FUS1, KSS1, STE11 or STE7 proteins, which participate in the cellular response to pheromones. The PSP surrogate would be, e.g., a mammalian, mitogen-activated protein kinase. Yeast cells engineered to express the surrogate protein kinase could be used to screen for activators or inhibitors thereof.

*Cyclins.* The PSP may be a cyclin, such as CLN1, CLN2 or CLN3. The cyclins regulate the progression of cells through the cell cycle. The human C, D1 and E cyclins are capable of substituting functionally for the CLN proteins of yeast. Inhibitors of mammalian cyclins may be useful in cancer chemotherapy. Yeast cells engineered to express a surrogate cyclin may be used to identify molecules which inhibit (or enhance) its activity.

### Peptide Libraries

While others have engineered yeast cells to facilitate screening of exogenous drugs as receptor agonists and antagonists, the cells did not themselves produce both the drugs and the receptors. Yeast cells engineered to produce the receptor, but that do not produce the drugs themselves, are inefficient. To utilize them one must bring a sufficient concentration of each drug into contact with a number of cells in order to detect whether or not the drug has an action. Therefore, a microtiter plate well or test tube must be used for each drug. The drug must be synthesized in advance and be sufficiently pure to judge its action on the yeast cells. When the yeast cell produces the drug, the effective concentration is higher.

In the present invention, the yeast cells collectively produce a "peptide library", preferably including at least 10⁷ different peptides, so that diverse peptides may be simultaneously assayed for the ability to interact with the PSP surrogate.

In an especially preferred embodiment, at least some peptides of the peptide library are secreted into the periplasm, where they may interact with the "extracellular" binding site(s) of an exogenous receptor. They thus mimic more closely the clinical interaction of drugs with cellular receptors. This embodiment optionally may be further improved (in assays not requiring pheromone secretion) by preventing pheromone secretion, and thereby avoiding competition between the peptide and the pheromone for signal peptidase and other components of the secretion system.

### Detection of Signal Transduction

Yeast cells may also be engineered so that their pheromone signal transduction pathways provide a more readily detectable evidence of the activity of a suspected drug. In these embodiments, the drug need not be a peptide produced by the same yeast cell, or even a peptide at all.

As previously mentioned, one of the consequences of activation of the pheromone signal pathway in wild-type yeast is growth arrest. If one is testing for an antagonist of a G protein-coupled receptor, or of other pheromone system proteins, this normal response of growth arrest can be used to select cells in which the pheromone response pathway is inhibited. That is, cells exposed to both a known agonist and a peptide of unknown activity will be growth arrested if the peptide is neutral or an agonist, but will grow normally if the peptide is an antagonist. Thus, the growth arrest response can be used to advantage to discover peptides that function as antagonists.

However, when searching for peptides which can function as agonists of G protein-coupled receptors, or other pheromone system proteins, the growth arrest consequent to activation of the pheromone response pathway is an undesirable effect for this reason: cells that bind peptide agonists stop growing while surrounding cells that fail to bind peptides will continue to grow. The cells of interest, then, will be overgrown or their detection obscured by the background cells, confounding identification of the cells of interest. To overcome this problem the present invention teaches engineering the cell such that: 1) growth arrest does not occur as a result of pheromone signal pathway activation (e.g., by inactivating the FAR1 gene); and/or 2) a selective growth advantage is conferred by activating the pathway (e.g., by transforming an auxotrophic mutant with a HIS3 gene under the control of a pheromone-responsive promoter, and applying selective conditions).

It is, of course, desirable that the exogenous receptor (or other PSP surrogate) be exposed on a continuing basis to the peptides. Unfortunately, this is likely to result in desensitization of the pheromone pathway to the stimulus. Desensitization may be avoided by mutating (which may include deleting) the SST2 gene so that it no longer produces a functional protein, or by mutating other genes which may contribute to desensitization, e.g., BAR1 in the case of **a** cells and SVG1 for either **a** or α cells.

If the endogenous pheromone receptor (or other cognate PSP) is produced by the yeast cell, the assay will not be able to distinguish between peptides which interact with the pheromone receptor (or other cognate PSP) and those which interact with the exogenous receptor (or other PSP surrogate). It is therefore desirable that the endogenous gene be deleted or otherwise rendered nonfunctional.

### BRIEF DESCRIPTION OF THE DRAWINGS

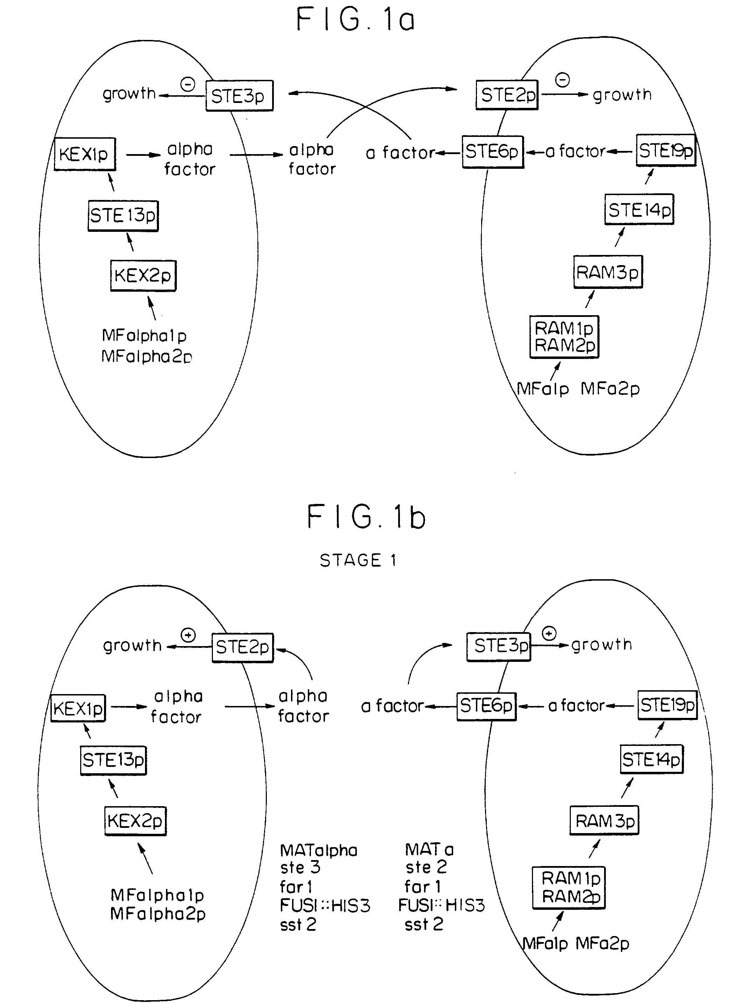

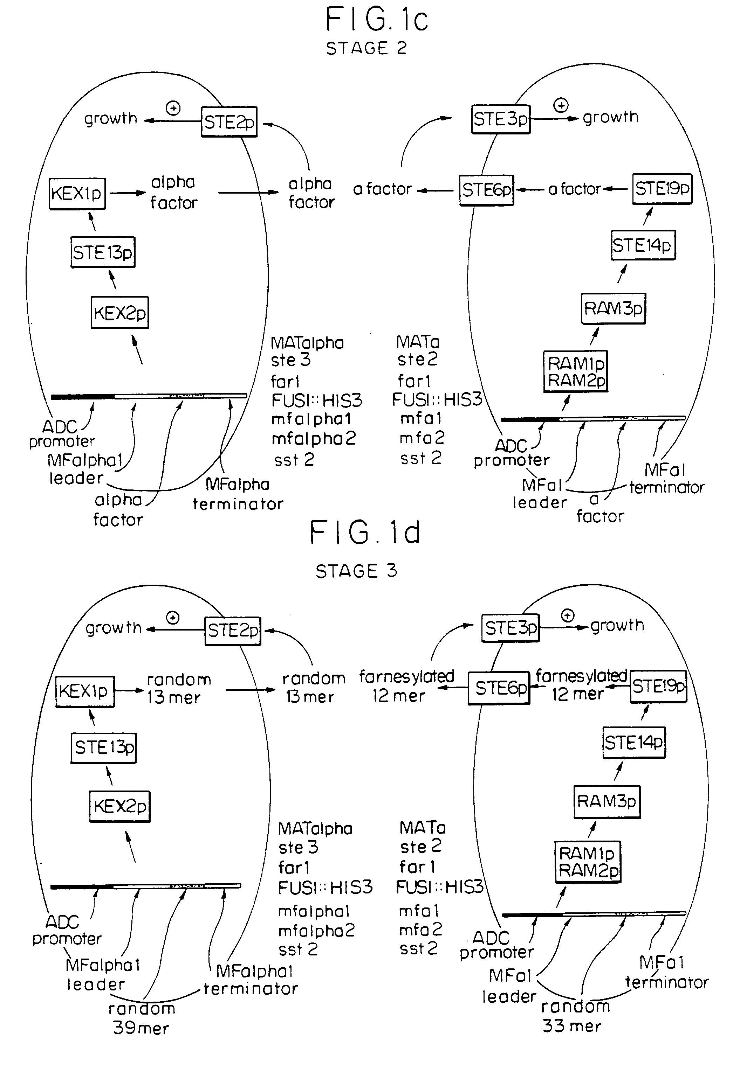

Figure 1. Outline of successive stages in the development of yeast autocrine systems.
An outline of the normal synthesis and release of mating pheromones is diagrammed in the left of Tigla. Two genes, *MFα1* and *MFα2*, encode precursor proteins (MFα1p and MFα2p) containing four and two repeats, respectively, of the tridecapeptide representing mature α-factor. These precursors are processed proteolytically in a series of enzymatic reactions that begin with cleavage of the signal sequence in the endoplasmic reticulum and involve both glycosylation of the leader peptide and cleavage by the proteases KEX2p, STE13p, and KEX1P. The result is the secretion of mature α-factor which, upon binding to STE2p normally expressed on the surface of **a** cells, elicits a number of changes in the **a** cells, including growth arrest. The **a** cells, in turn, express two genes, *MFa1* and *MFa2*, which encode precursors (MFa1p and MFa2p) for **a**-factor as shown in the right of Fig. 1a. These precursors undergo farnesylation by RAM1 and RAM2, proteolytic trimming of the C-terminal three amino acids (by a protein tentatively identified as RAM3p), carboxymethylation of the newly exposed C-terminal cysteine by STE14p, and endoproteolytic removal of the **N**-terminal leader sequence by an activity provisionally identified as STE19p. Upon export of the mature **a**-factor from the cell via STE6p, it binds to STE3p expressed on the surface of α cells and stops their growth.

Stage 1 shown in Fig. 1b, involves the development of yeast strains in which *SST2, FAR1,* and *HIS3* are inactivated and a suitable reporter construct like fus1::HIS3 is integrated into the genomes of both α and **a** cells. α cells are further altered by replacement of the normally expressed STE3p with STE2p, while **a** cells are further modified by replacement of the normally expressed STE2p with STE3p. The resulting strains should show growth on histidine-deficient media in the absence of exogenous pheromone.

Stage 2, shown in Fig. 1c, involves, first, inactivation of *MFα1* and *MFα2* in cells and inactivation of *MF***a**1 and *MF**a**2* in **a** cells developed in Stage 1. These modifications will result in strains which are auxotrophic for histidine. Next, the appropriate expression plasmid will be introduced: the expression plasmid pADC-MF (see Figure 4) containing an oligonucleotide encoding α-factor should confer upon α cells the ability to grow on histidine-deficient media; the expression plasmid pADC-MFa (see Figure 6) containing an oligonucleotide encoding **a**-factor should enable **a** cells to grow on histidine-deficient media.

Stage 3, shown in Fig. 1d, uses the cells developed in Stage 2 for the insertion of expression plasmids. However, instead of using plasmids which contain oligonucleotides that encode genuine pheromone, the yeast will be transformed with expression plasmids that contain random or semi-random oligonucleotides. Transformants which can grow on histidine-deficient media will be expanded and their plasmids isolated for sequencing of the inserted oligonucleotide.

Figure 2. Diagram of the plasmid used for mutagenesis of *MFα1.* A 1.8 kb EcoRI fragment containing *MFα1* is cloned into the EcoRI site of pALTER such that single-stranded DNA containing the *MFα1* minus strand can be synthesized. The diagram illustrates the different regions of *MFα1*, including the promoter, transcription terminator, and different domains of the precursor protein: the signal peptide, the pro peptide, the four repeats of mature α-factor, and the three spacers which separate these repeats. Above the block diagram of the regions of *MFα1* are the amino acid sequences of the signal peptide and the pro peptide; below it are those of the pheromone repeats and the spacers. The sites of proteolytic processing of the precursor protein are indicated by arrows, with each proteolytic activity represented by a different arrow, as indicated in the figure.

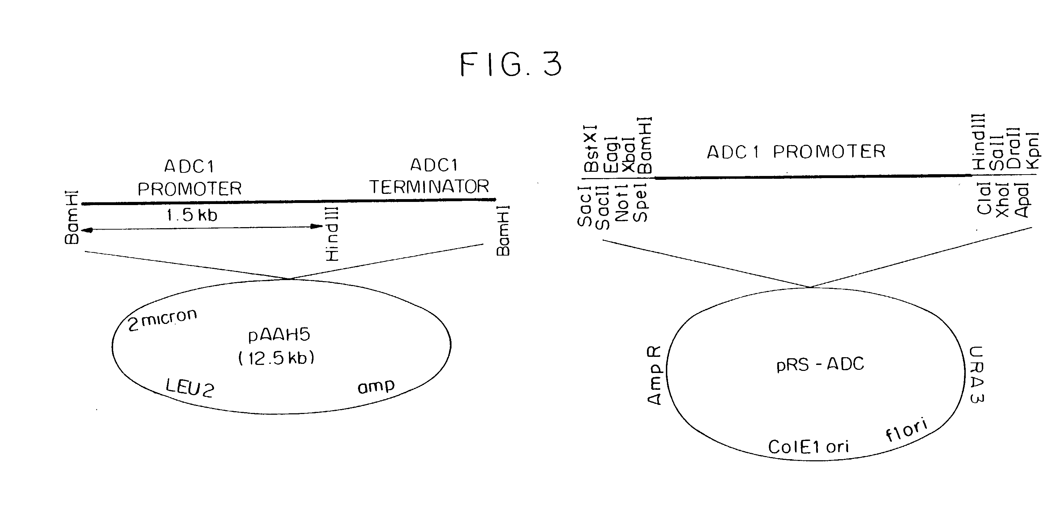

Figure 3. Diagram of the plasmids used in the construction of the MFα expression cassette. pAAE5 contains the ADC1 promoter which will be used to drive expression of synthetic oligonucleotides inserted into the MFα expression cassette. The 1.5 kb BamHI to HindIII fragment containing the ADC1 promoter will be cloned into pRS426, a plasmid which functions as a high-copy episome in yeast, to yield pRS-ADC. pRS-ADC will be the recipient of MFα1 sequences which have been mutated as follows: the region of *MFα1* which encodes mature α-factor will be replaced with restriction sites that can accept oligonucleotides with Afl II and BglII ends. Insertion of oligonucleotides with AflII and BglII ends will yield a plasmid which encodes a protein containing the MFα1 signal and leader sequences upstream of the sequence encoded by the oligonucleotide. The MFα1 signal and leader sequences should direct the processing of this precursor protein through the pathway normally used for the secretion of mature α-factor.

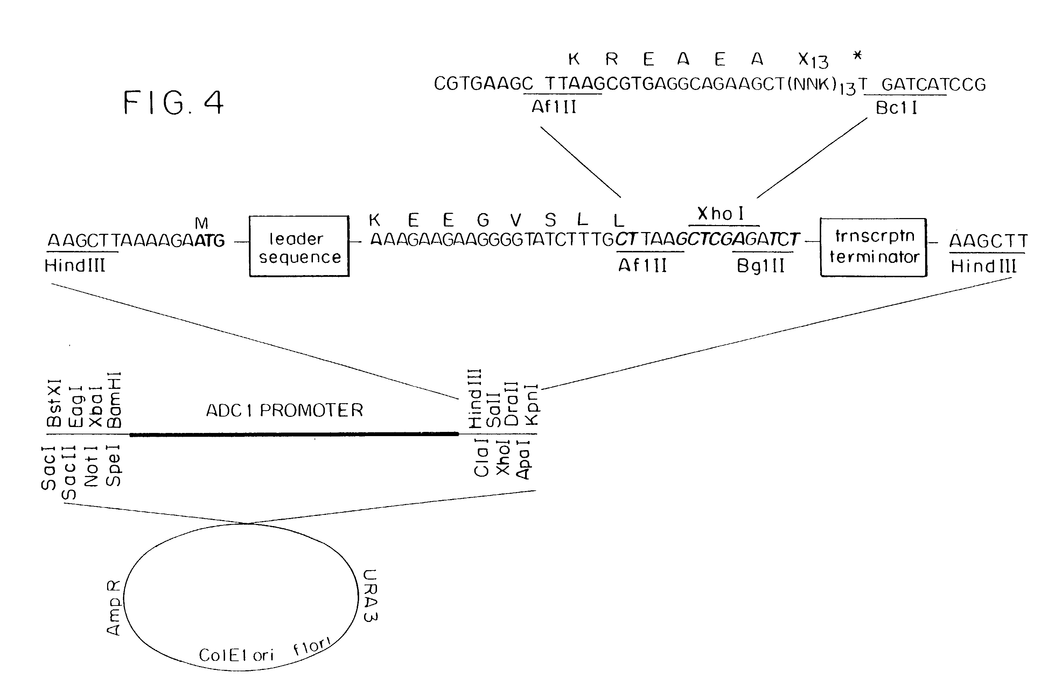

Figure 4. Diagram of constructs used for the expression of random oligonucleotides in the context of MFα1. Oligonucleotides containing a region of 39 random base pairs (shown at the top of the figure) will be cloned into the AflII and BglII sites of the MFα1 expression cassette. These oligonucleotides will encode the six amino acids immediately N-terminal to the first repeat of the α-factor in *MFα1*, followed in succession by a tridecapeptide of random sequence and a stop codon. Yeast transformed with these constructs and selected for an ability to grow on media deficient in uracil will use the ADC1 promoter to express a protein consisting of the MFα1 leader (both pre and pro peptides) followed by 13 random amino acids. Processing of the leader sequences will result in secretion of the tridecapeptide.

Figure 5. Diagram of the plasmid used for mutagenesis of *MF**a**1*. A 1.6 kb BamHI fragment containing *MF**a**1* is cloned into the BamHI site of pALTER such that single-stranded DNA containing the *MF**a**1* minus strand can be synthesized. The diagram illustrates the different regions of *MF**a**1*, including the promoter, transcription terminator, and different domains of the precursor protein: the leader peptide; the dodecapeptide that represents the peptide component of mature **a**-factor and whose C-terminal cysteine becomes farnesylated and carboxymethylated during processing; and the C-terminal three amino acids that are removed during processing of the precursor. Above the block diagram of the regions of *MF**a**1* is the amino acid sequence of the primary translation product.

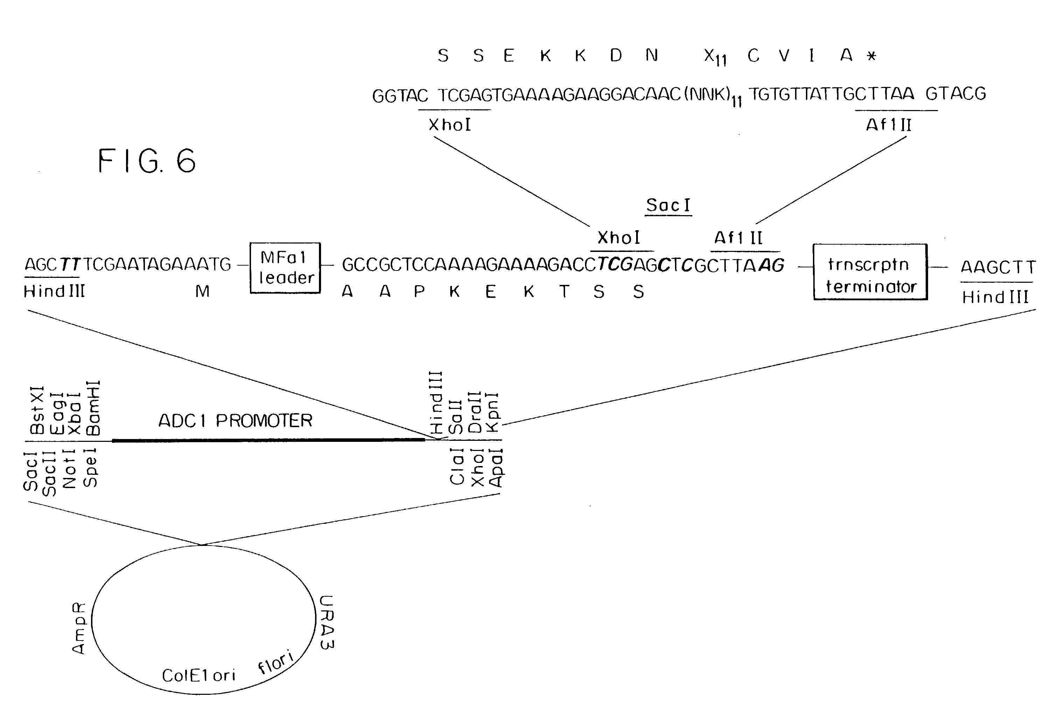

Figure 6. Diagram of constructs used for the expression of random oligonucleotides in the context of MF**a**1. Oligonucleotides containing a region of 33 random base pairs (shown at the top of the figure) will be cloned into the XhoI and AflII sites of the MF**a**1 expression cassette. These oligonucleotides will encode the seven amino acids immediately N-terminal to the first amino acid of mature **a**-factor, followed in succession by a monodecapeptide of random sequence, a cysteine which is farnesylated and carboxymethylated during processing of the precursor, three amino acids (VIA) which are proteolytically removed during processing, and a stop codon. Yeast transformed with these constructs and selected for an ability to grow on media deficient in uracil will use the ADC1 promoter to express a precursor protein consisting cf the MFa1 leader followed by 11 random amino acids and a C-terminal tetrapeptide CVIA. Processing of this precursor will result in secretion of a C-terminally farnesylated, carboxymethylated dodecapeptide which consists of 11 random amino acids and a C-terminal cysteine.

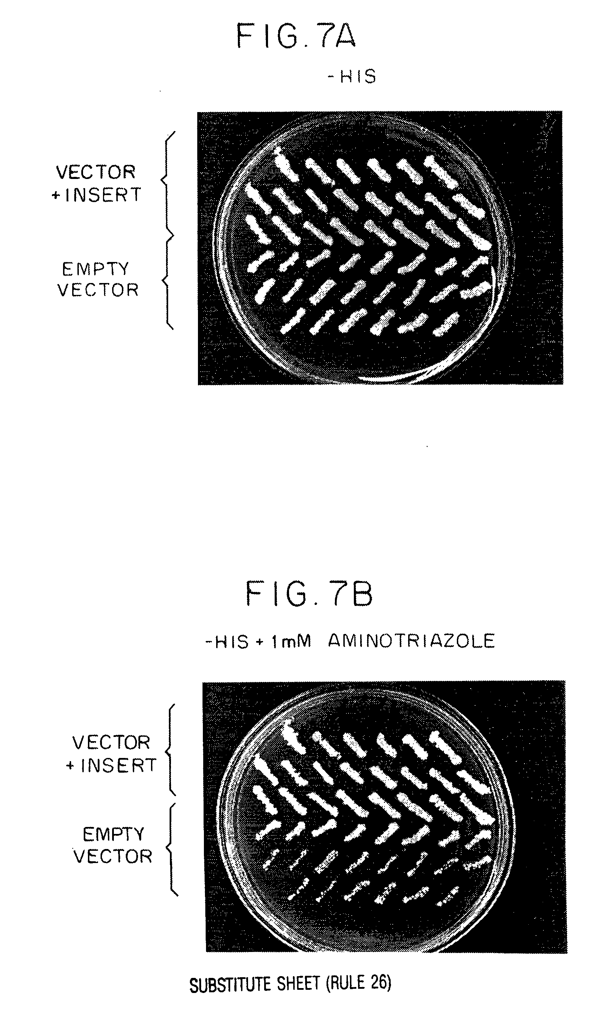

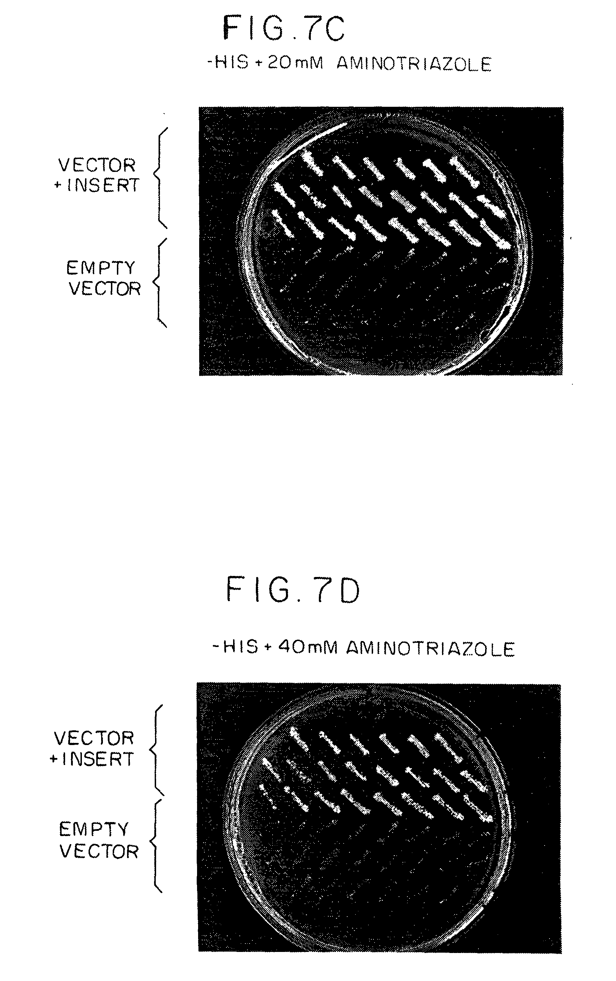

Figure 7. Autocrine Mat**a** strain secretes and responds to signalling by α-factor.
A synthetic oligonucleotide encoding the yeast α-factor pheromone was expressed in Mat**a** cells. These cells normally express the **a**-factor pheromone but were prevented from doing so by deletion cf the endogenous **a**-factor-encoding genes. Expression and release of α-factor by these cells renders them "autocrine" with regard to pheromone signalling. The peptide containing mature α-factor was processed within these cells for transport through the yeast secretory pathway to the extracellular environment. Pheromone signalling was initiated by the binding of α-factor to the Ste2 receptor expressed in Mat**a** cells. Signalling by pheromone in the strain background used in these experiments results in growth of responsive cells on media that is deficient in histidine, shown in Fig. 7A. Background growth of control cells that are not expressing α-factor is prevented by increasing concentrations of the HIS3 inhibitor, aminotriazole, as shown in Figs. 7B, 7C and 7D.

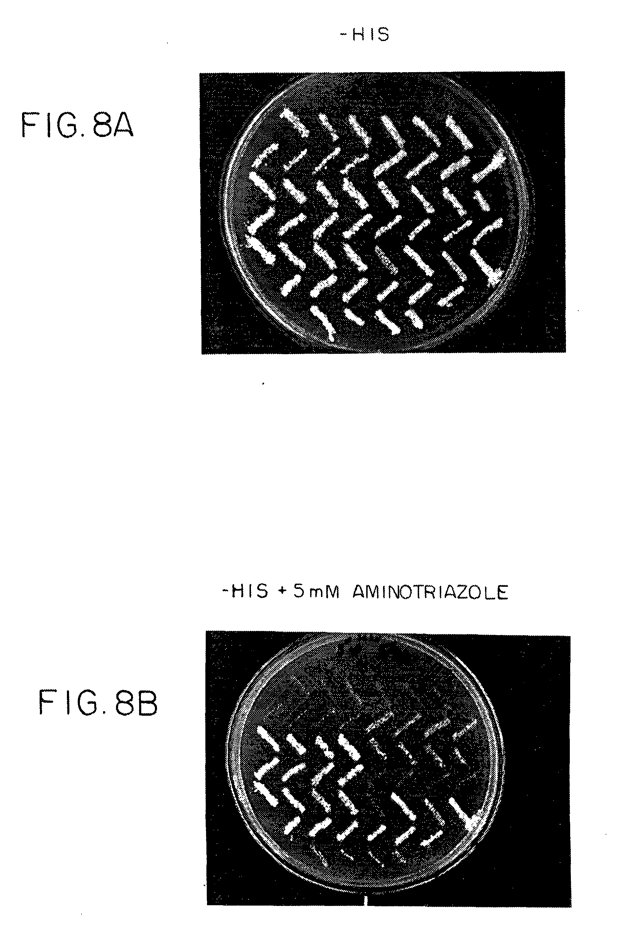

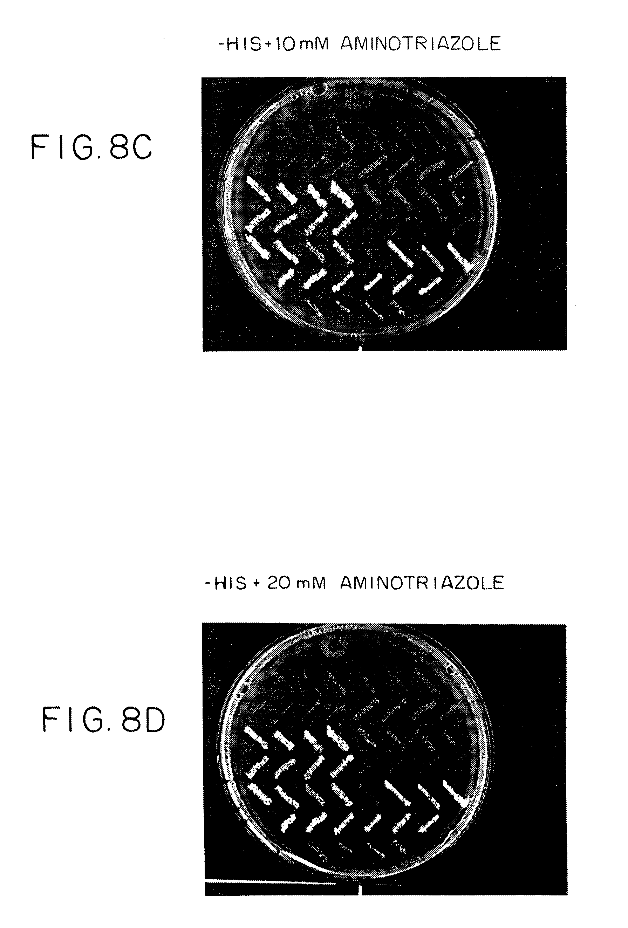

Figure 8. Autocrine MAT**a** strain secretes and responds to signalling by **a**-factor.
Yeast **a**-factor was expressed from a plasmid containing a synthetic pheromone-encoding oligonucleotide in Mat**a** cells. The yeast used in these experiments were made "autocrine" by replacement of the normally expressed Ste2 protein with the receptor for **a**-factor, Ste3. The **a**-factor peptide was processed in these cells and transported to the extracellular environment by the endogenous Ste6 protein, an ATP-dependent transmembrane transporter. Pheromone signalling initiated by the **a**-factor released by these cells when bound to Ste3 is indicated by the growth of the cells on histidine-deficient media, shown in Fig. 8A. Background growth of control cells, which are incapable of expressing **a**-factor (these α cells lack the plasmid which encodes the pheromone) is prevented by increasing concentrations of the HIS3 inhibitor, aminotriazole, shown in Figs. 8B, 8C and 8D.

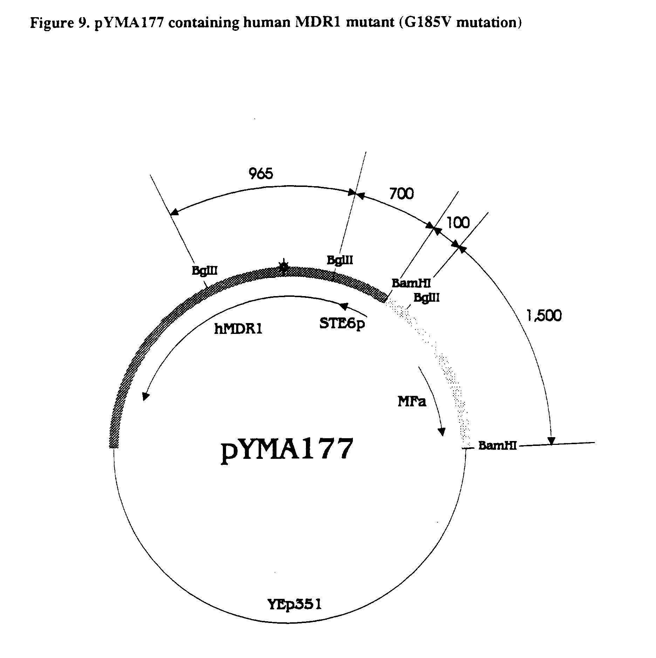

Figure 9. Plasmid pYMA177 containing mutant human MDR1 (G185V mutation).

The plasmid pYMA177 was constructed by Karl Kuchler and permits the simultaneous overexpression of both a mutant human Mdr1 protein and the yeast **a**-factor pheromone precursor (Kuchler & Thorner, Proc. Natl. Acad. Sci. 89, 2302 (1992).

### DETAILED DESCRIPTION OF THE PREFERRED EMBODIMENTS

The present invention contemplates the assaying of peptides, especially when presented in peptide libraries, expressed in genetically engineered yeast cells, for the ability of the peptides to interact with pheromone system proteins and PSP surrogates produced by those yeast cells.

For the purpose of the present invention, an "exogenous" protein is one which sufficiently differs in amino acid sequence from the proteins naturally produced by the yeast cell in question so that its closest cognate is a protein produced by a cell other than a yeast cell. The cell producing this cognate protein may be a microbial cell (other than a yeast cell), a plant cell, or an animal cell. If an animal cell, it may be of invertebrate (e.g., insect or nematode) or of vertebrate (e.g., avian, piscine or mammalian, especially human) origin. A protein is considered to be of, e.g., human origin, regardless of whether it is encoded by the chromosome of a normal human, or by the genome of a virus which infects and replicates in human cells.

An "activator" of a pheromone system protein surrogate is a substance which, in a suitable yeast cell, causes the pheromone system protein surrogate to become more active, and thereby elevates the signal transduced by the native or modified pheromone signal pathway of said cell to a detectable degree. The surrogate may be initially nonfunctional, but rendered functional as a result of the action of the activator, or it may be functional, and the effect of the activator is to heighten the activity of the surrogate. The mode of action of the activator may be direct, e.g., through binding the surrogate, or indirect, e.g., through binding another molecule which otherwise interacts with the surrogate. When the PSP surrogate is a substitute for a pheromone receptor, and the activator takes the place of the pheromone, it is customary to refer to the activator as an agonist of the receptor.

Conversely, an "inhibitor" of a pheromone system protein surrogate is a substance which, in a suitable yeast cell, causes the PSP surrogate to become less active, and thereby reduces the transduced signal to a detectable degree. The reduction may be complete or partial. When the PSP surrogate is a substitute for a pheromone receptor, and the inhibitor competes with the pheromone for binding to the receptor, it is customary to refer to the inhibitor as an "antagonist".

The term "modulator" includes both "activators" and "inhibitors".

A surrogate PSP protein is "functionally homologous" to a yeast protein if, either alone or after being modified by a drug, it is able to perform the function of the yeast PSP, or an analogous function, within the engineered yeast cell. It is not necessary that it be as efficient as the yeast protein, however, it is desirable that it have at least 10% of at least one of the pheromone system-related activities of the yeast protein. Nor is it necessary that it have the same spectrum of action as the yeast protein, e.g., if it is a receptor, it may respond to entirely different ligands than does the endogenous receptor, or to some common ligands and some new ones. The receptors of Table 2 are considered functionally homologous with the yeast pheromone receptors, even though they do not respond to yeast pheromones, and may not couple to the unmodified endogenous G proteins, although they are G protein-coupled receptors. This is considered an "analogous function".

The PSP surrogate may be a protein which must be modified in some way by a drug to be functional. For example, the drug could cause an allosteric change in the PSP surrogate's conformation, or it could cleave off a portion of the surrogate, the balance of the protein then being a functional molecule.

The PSP surrogate may also be one which is functional only if other modifications are made in the yeast cell, e.g., expression of a chimeric G α subunit to interact with an exogenous G protein-coupled receptor.

The term "substantially homologous", when used in connection with amino acid sequences, refers to sequences which are substantially identical to or similar in sequence, giving rise to a homology in conformation and thus to similar biological activity. The term is not intended to imply a common evolution of the sequences.

Typically, "substantially homologous" sequences are at least 50%, more preferably at least 80%, identical in sequence, at least over any regions known to be involved in the desired activity. Most preferably, no more than five residues, other than at the termini, are different. Preferably, the divergence in sequence, at least in the aforementioned regions, is in the form of "conservative modifications".
"Conservative modifications" are defined as
(a) conservative substitutions of amino acids as hereafter defined; and
(b) single or multiple insertions or deletions of amino acids at the termini, at interdomain boundaries, in loops or in other segments of relatively high mobility.

Preferably, except at the termini, no more than about five amino acids are inserted or deleted at a particular locus, and the modifications are outside regions known to contain binding sites important to activity.

Conservative substitutions are herein defined as exchanges within one of the following five groups:
I. Small aliphatic, nonpolar or slightly polar residues:
   Ala, Ser, Thr (Pro, Gly)
II. Polar, negatively charged residues: and their amides
   Asp, Asn, Glu, Gln
III. Polar, positively charged residues:
   His, Arg, Lys
IV. Large, aliphatic, nonpolar residues:
   Met, Leu, Ile, Val (Cys)
V. Large, aromatic residues:
   Phe, Tyr, Trp

Residues Pro, Gly and Cys are parenthesized because they have special conformational roles. Cys participates in formation of disulfide bonds. Gly imparts flexibility to the chain. Pro imparts rigidity to the chain and disrupts α helices. These residues may be essential in certain regions of the polypeptide, but substitutable elsewhere.

Two regulatory DNA sequences (e.g., promoters) are "'substantially homologous" if they have substantially the same regulatory effect as a result of a substantial identity in nucleotide sequence. Typically, "substantially homologous" sequences are at least 50%, more preferably at least 80%, identical, at least in regions known to be involved in the desired regulation. Most preferably, no more than five bases are different.

The term "autocrine cell", as used herein, refers to a cell which produces a substance which can stimulate the pheromone system pathway of that cell. Wild-type α and **a** cells are not autocrine. However a yeast cell which produces both α-factor and α-factor receptor, or both **a**-factor and **a**-factor receptor, in functional form, is autocrine. By extension, yeast cells which produce **a** peptide which is being screened for the ability to activate the pheromone system pathway (e.g., by activating a G protein-coupled receptor) are called "autocrine cells", though it might be more precise to call them "putative autocrine cells". Of course, in a library of such cells, in which a multitude of different peptides are produced, it is likely that one or more of the cells will be "autocrine" in the stricter sense of the term.

### Farnesyltransferases

The activity of yeast **a**-factor requires its farnesylation (mediated by protein farnesyltransferase, comprised of Ram1p and Ram2p), proteolytic cleavage of the C-terminal 3 amino acids of the primary translation product (mediated by an as yet unidentified enzyme), and carboxymethylation of the C-terminal cysteine (mediated by Ste14p). The yeast and mammalian farnesyltransferases are structurally and functionally similar (Gomez R et al., Biochem. J. 289:25-31, 1993; Kohl NE et al., J. Biol. Chem. 266:18884-8, 1991). Sequence homologies exist between the genes encoding the α and β subunits of the yeast farnesyltransferase *(RAM2* and *RAM1*, respectively) and the genes encoding the α and β subunits of the mammalian farnesytransferase (Kohl NE et al., J. Biol. Chem. 266:18884-8, 1991; Chen WJ et al., Cell 66:327-34, 1991). It has been observed that the β subunit of mammalian farnesytransferase and Ram1p are 37% identical in amino acid sequence (Chen WJ et al., Cell 66:327-34, 1991).

The importance of a screen for inhibitors of farnesyltransferase is suggested by the facts that mammalian p21ras, a preeminent regulator of the growth and differentiation of mammalian cells that is involved in a variety of cancers, is a substrate for the farnesyltransferase and that farnesylation of p21ras is required for its activity. In fact, a synthetic organic inhibitor of farnesyl protein transferase has been shown to selectively inhibit *ras*-dependent cell transformation (Kohl et al., Science 260, 1934 (1993). Of the two subunits of farnesyltransferase, the β subunit is a more attractive target for inhibitors, since it is apparently dedicated to farnesylation. The α subunit, in contrast, is shared by geranylgeranyltransferase I, an enzyme involved in the modification of the Gγ subunits of heterotrimeric G proteins and small molecular weight G proteins of the Rho/Rac family. While the β subunit is dedicated to farnesylation, the mammalian farnesyltransferase has a variety of substrates in addition to p21ras. The effect of inhibitors of the β subunit on the farnesylation of these other substrates, e.g., lamin proteins, transducin-γ and rhodopsin kinase, will be considered in the design and use of potential farnesyltransferase inhibitors.

It has not yet been demonstrated that the homologous mammalian gene will functionally substitute for yeast Ram1p, however, this can be formally tested using *ram1* mutants and a vector expressing the mammalian gene encoding the β subunit of the farnesyltransferase. If the mammalian β subunit can function in place of Ram1p, test cells will be both viable (as a result of farnesylation of Ras) and competent for mating (as a result of farnesylation of **a-**factor).

If the mammalian gene encoding the β subunit of farnesyltransferase complements *ram1,* yeast would provide a test system for the discovery of potential inhibitors of mammalian farnesyltransferase. Specifically, MAT**a** yeast tester cells could be exploited that: 1. carry the gene for the β subunit of mammalian farnesyltransferase in lieu of RAM1; 2. carry the *cam* mutation that renders the strains resistant to loss of Ras function in the presence of cAMP; 3. respond to **a**-factor which they export by virtue of heterologous expression of Ste3p; 4. respond to autocrine **a**-factor such that they cannot grow on media containing galactose. The latter characteristic will require expression of GAL1 under the control of a pheromone-responsive promoter and cells engineered to contain mutated GAL7 or GAL10 genes. Expression of GAL1 is toxic in the presence of galactose in strains which contain mutations in either the GAL7 or GAL10 genes. Signaling through the pheromone response pathway would render cells so engineered galactose-sensitive. Exposure of such strains to compounds which inhibit the β subunit of farnesytransferase will confer upon these cells the ability to grow on media containing galactose and cAMP.

If the mammalian gene encoding the β subunit of farnesyltransferase (and all modified versions of the gene) fails to complement *ram1*, we may use the wild-type Ram1p as a surrogate target for potential effectors of mammalian farnesyltransferase. Specifically, we will use as tester cells MATa yeast strains that: 1. carry the *cam* mutation that renders the strains resistent to loss of RAS function in the presence of cAMP; 2. respond to a-factor which they export by virtue of heterologous expression of Ste3p; 3. respond to autocrine a-factor such that they cannot grow on media containing galactose. Exposure of such strains to compounds which inhibit the β subunit of farnesytransferase will confer upon these cells the ability to grow on media containing galactose and cAMP.

In the strategies outlined above, it is desirable to discriminate inhibitors of farnesytransferase from compounds that either directly block the negative response to **a**-factor, e.g. by interfering with the interaction of the Ste4-Ste18 complex with its effector, or by blocking the production of **a-**factor by a mechanism that does not involve farnesyltransferase. Controls would identify such false positives. Candidate agents will be tested on a MATa strain that is engineered to secrete α-factor and to respond to the secreted **a**-factor by failing to grow on galactose-containing media, as in the negative selection scheme outlined above. The strain will express wild type Ramlp. Any agent that enables these cells to grow on media containing galactose and cAMP will not be acting as an inhibitor of farnesyltransferase.

Candidate compounds which pass the foregoing test may act by targeting Ste14p, Ste6p, or other proteins involved in the maturation and export of **a**-factor, rather than farnesyltransferase. (Note, however, that compounds that inhibit processes critical to cell survival will not give rise to false positives. For example, since the protease responsible for the endoproteolytic removal of the C-terminal tripeptide of the **a**-factor precursor likely participates in the processing of Gg and members of the Rho/Rac family of proteins, inhibitors of this enzyme may not permit growth of the tester cells). Of the proteins involved in the production of **a**-factor, only the farnesyltransferase is also a major determinant of RAS function. Due to this effect, *ram1* mutants are defective for growth at 30°C and completely unable to grow at 37 (He B et al., Proc Natl Acad Sci 88:11373-7, 1991). Tester cells (described above) can be grown in the presence of a candidate inhibitor on galactose-containing media ± cAMP. If the test compound inhibits farnesyltransferase, cells will be capable of growth on galactose + CAMP but not on galactose in the absence of cAMP. This difference may be most obvious at 37°. If, on the other hand, the test compound inhibits other proteins involved in **a**-factor production, cells will grow on galactose-containing media regardless of the presence or absence of cAMP.

Compounds which pass the above tests are likely inhibitors of farnesyltransferase. This can be confirmed and their potencies determined with direct *in vitro* enzyme assays. Note that the strategies outlined will identify farnesyltransferase inhibitors which affect Ram1p. Agents which block Ram2p would likely fail to grow under all conditions. Indeed, *ram*2 null mutations are lethal (He B et al., Proc Natl Acad Sci 88:11373-7, 1991), perhaps due to the fact that Ram2p also functions as a component of geranylgeranyltransferase I.

### Carboxymethyltransferases

In yeast, methylation of the C-terminal amino acid of **a**-factor, Ras proteins, and presumably Rho/Rac proteins is catalyzed by Ste14p. Although MATa ste14 mutants are unable to mate, reflecting the requirement of carboxymethylation for the activity of **a**-factor, *ste14* disruptions are not lethal and do not affect the rate of cell proliferation. Carboxymethylation appears to be dispensible for the function of Ras proteins and Ste18p (the yeast homologue of the Gγ subunit). Although Ras function in yeast can apparently tolerate the absence of carboxymethyl modification, it is nonetheless possible that inhibitors of mammalian methyltransferases could alter the activity of mammalian p21ras.

It could be determined if yeast *ste14* mutations can be complemented by the homologous mammalian gene, or a modified version of it. One would use an episomal vector to express the mammalian gene encoding the methyltransferase in yeast that are genotypically *ste14*. The strain would be a modified MATa strain that expresses the **a**-factor receptor in lieu of the normal **a**-factor receptor and that contains an integrated fus1-HIS3 construct, so that the **a**-factor secreted by the cell confers autocrine growth on histidine-deficient media. If the mammalian methyltransferase can function in place of Ste14p, the tester cells will be capable of mating. That is, the mammalian methyltransferase will permit synthesis of active **a**-factor in *ste14* mutants.

If the mammalian gene encoding the methyltransferase will complement *ste14*, tester strains can be constructed to test for potential inhibitors of mammalian methyltransferase. In one embodiment, tester MAT**a** yeast strains will: 1. carry a mammalian carboxymethyltransferase gene in lieu of STE14; 2. respond to a-factor which they export by virtue of heterologous expression of Ste3p; 3. respond to autocrine **a**-factor such that they cannot grow on media containing galactose as in the negative GAL1 selection scheme outlined above. Exposure of such strains to compounds which inhibit the methyltransferase will confer upon these cells the ability to grow on media containing galactose.

It is desirable to discriminate inhibitors of carboxymethyltransferase activity from compounds that either directly block the negative response to **a**-factor, e.g. by interfering with the interaction of the Ste4-Ste18 complex with its effector, or block the production of **a**-factor by a mechanism that does not involve methyltransferase. The following control experiments will identify such false positives. Candidate inhibitors will be tested on a MAT**a** strain that is engineered to secrete **a**-factor and to respond to the secreted **a**-factor by failing to grow on galactose-containing media. Any agent that enables these cells to grow on media containing galactose will be not be acting as an inhibitor of carboxymethyltransferase. Candidate compounds which pass the foregoing test may be targetting the carboxymethyltransferase, farnesyltransferase, Ste6p, or other proteins involved in the maturation and export of **a**-factor. In order to discriminate the target of the compounds, a combination of *in vitro* biochemical and *in vivo* genetic assays can be applied: both the carboxymethyltransferase and the farnesyltransferase can be assayed *in vitro* to test the effect of the candidate agent. Furthermore, if the target is Ste14p its overexpression on high-copy plasmids should confer resistance to the effect of the compound *in vivo.*

### Proteases

Mature yeast α-factor is a thirteen amino acid peptide that is derived from a polyprotein precursor in much the same manner as mature mammalian melanocyte-stimulating hormone (MSH) or calcitonin are derived from precursor polyproteins. Two genes in the yeast genome encode prepro-α-factor, MFα1 and MFα2. MFα1 encodes a precursor polypeptide containing four copies of mature α-factor embedded in a polypeptide of the following structure: hydrophobic pre-sequence / hydrophilic pro-sequence / α-factor / α-factor / α-factor / α-factor. MFα2 encodes a polyprotein precursor of a similar structure containing only two copies of mature α-factor.

Pre-pro-α-factor is synthesized in the cytoplasm and is then transported from the cytoplasm to the endoplasmic reticulum and then to the Golgi along the classical secretory pathway of *S. cerevisiae.* The signal sequence of prepro-α-factor is cleaved during transit into the ER by signal peptidase and asparagine-linked oligosaccharides are added (in the ER) and modified (in the Golgi) on the pro-segment of the precursor as it transits the secretory pathway. Once in the Golgi, three distinct proteolytic processing events occur. First, the Kex2 protease cleaves at dibasic residues (-KR-) near the amino terminus of each α-factor repeat. Kex2 is homologous to the subtilisin-like endoproteases PC2 and PC1/PC3 involved in prohormone processing in mammalian cells (Smeekens and Steiner 1990; Nakayama et al. 1991). Additional mammalian Kex2-like processing endoproteases include PACE, isolated from a human hepatoma, PC4, expressed in testicular germ cells and PC6, a candidate protease for the processing of gastrointestinal peptides (Barr et al. 1991; Nakayama et al. 1992; Nakagawa et al. 1993). It appears that Kex2-like proteins comprise a large family of tissue-specific endoproteases in mammalian cells.

Once Kex2 has released the immature α-factor peptides, two additional proteases act to complete processing. Kex1 is a specific carboxypeptidase that removes the carboxy-terminal-KR remaining after cleavage by Kex2. Like its mammalian counterparts carboxypeptidases B and E, Kex1 is highly specific for peptide substrates with carboxy-terminal basic residues. The final proteolytic processing event that occurs is the removal of the spacer dipeptides at the amino terminus of each pro-α-factor peptide. This is accomplished by the product of the STE13 gene, dipeptidyl aminopeptidase A. This enzyme is a type IV dipeptidyl aminopeptidase: it is capable of cleaving on the carboxyl side of either -x-A- or -x-P- sites in vitro.

Other type IV dipeptidyl aminopeptidases are believed to be active in the processing of a variety of pre-peptides in animal cells (Kreil 1990). In addition, functional similarity has been proved between yeast Kex1 and Kex2 and their mammalian counterparts in that both yeast enzymes will proteolytically cleave endogenous precursors when expressed in mammalian cells deficient in the native enzyme (Thomas et al. 1988, 1990). It appears likely, then, that mammalian homologs of the yeast proteases Kex1, Kex2 and Ste13p, when expressed in yeast, will function to process a synthetic α-factor pheromone precursor bearing appropriate cleavage sites. Human proteases that may so function in yeast include PC2 and PC1/PC3 (or other Kex2 homologs), carboxypeptidases B and E (Kex1 homologs) and type IV dipeptidyl aminopeptidases (Ste13p homologs).

Yeast would provide a facile assay system for the discovery of inhibitors of proteases able to process synthetic α-factor. The yeast could be engineered to express both the potential inhibitor and the exogenous protease, and, preferably, not the latter's yeast cognate.

Furthermore, this means of exploiting yeast pheromone processing to identify protease inhibitors can be expanded to encompass any protease that can be expressed to function in yeast, provided an appropriate cleavage recognition site is included in a synthetic α-factor precursor. In the latter approach, novel proteolytic activities will be added to yeast; these enzymes will substitute for proteases in the α-factor maturation pathway but will not be "catalytic homologues" of Kex1, Kex2 or Ste13p. Production of mature α-factor will become dependent on the activity of the novel protease through removal of the recognition site(s) for a selected yeast enzyme from a synthetic MFα gene and insertion of the recognition sequence for the novel protease(s).

Enzymes for which inhibitors could be identified via this strategy include, by way of example, HIV-1 protease or other virally encoded proteases involved in the maturation of infectious particles; neutrophil elastase believed to be involved in pulmonary disease and inflammatory bowel disease; Factor Xa involved in thrombin processing and clotting disorders; and CD26, a dipeptidyl peptidase IV and putatively the second receptor for HIV-1 on CD4⁺ cells. In addition, metalloproteinases (e.g. collagenase) and serine proteases involved in tissue invasion by tumor cells and in metastasis would be suitable therapeutic targets. In support of this, it has been demonstrated that administration of tissue-derived inhibitors of metalloproteinases results in decreased metastasis in animal models (Schultz et al. 1988). Collagenases have also been implicated in the destruction of connective tissue which accompanies inflammatory processes like rheumatoid arthritis.

The use of the present invention to screen for modulators of prohormone convertase PC1 is described in Example 8.

### Exogenous ABC Transporters

The majority of proteins destined for transport to the extracellular environment proceed through a secretory pathway that includes translation initiation in the cytoplasm, transport to the lumen of the endoplasmic reticulum, passage through the Golgi to secretory vesicles and subsequent exit from cells. Other proteins leave the cell by an alternative mechanism, which involves mediation by an "ABC transporter". The ABC transporters form a family of evolutionarily conserved proteins, share a similar overall structure, and function in the transport of large and small molecules across cellular membranes (Higgins 1992). The characteristic component of members of this protein family is a highly conserved sequence that binds ATP (Higgins et al., 1986; Hyde et al. 1990); these intrinsic membrane proteins are ATPases, deriving energy from the hydrolysis of that nucleotide to effect the transport of molecules. This family includes over 50 prokaryotic and eukaryotic proteins: transporters of amino acids, sugars, oligosaccharides, ions, heavy metals, peptides, or other proteins belong to this superfamily. Representative transmembrane transporters are included in Table 1. Typically, ABC transporters use the energy of ATP hydrolysis to pump substrate across a cell membrane against a concentration gradient. Some import substrate, others export it. See Higgins, *Ann. Rev. Cell, Biol.,* 8:67-113 (1992).

The prototypical structure of an ABC transporter includes four membrane-associated domains: two hydrophobic, putative membrane-spanning sequences, each predicted to traverse the membrane six times, and two nucleotide binding domains that couple ATP hydrolysis to transport. In prokaryotes, the domains of an ABC transporter are often present on separate polypeptides. Various permutations of domain fusions have been described: the *E. coli* iron hydroxamate transporter contains the two membrane-spanning domains in a single polypeptide and the ribose transporter of the same organism bears two nucleotide-binding domains on one molecule. The major histocompatibility complex (MHC) peptide transporter is composed of two polypeptides, Tap1 and Tap2. The N-terminus of each protein contains a hydrophobic membrane-spanning domain while the C-terminus contains an ATP-binding sequence. Together Tap1 and Tap2 form a functional complex. The heavy metal tolerance protein, HMT1, expressed in the fission yeast *Schizosaccharomyces pombe,* consists of a polypeptide containing a single hydrophobic domain and a C-terminal ATP-binding sequence (Ortiz et al. 1992). It may be that the HMT1 transporter functions as a homodimer. The *Saccharomyces cerevisiae* Ste6 **a**-factor transporter is expressed as a single polypeptide containing two membrane-spanning domains and two nucleotide-binding domains. When Ste6 is expressed as two half-molecules, the protein complex which apparently forms retains function at a level greater than 50% that of the wild type, single polypeptide (Berkower and Michaels 1991). In other eukaryotic ABC transporters, including Mdr1, CFTR and MRP, the four domains are also contained within a single polypeptide. Thus, the ABC transporter may be a single multidomain polypeptide, or it may comprise two or more polypeptides, each providing one or more domains.

In general, transporters contain six transmembrane segments per each hydrophobic domain, for a total of twelve segments. The minimum number of transmembrane segments required for formation of a translocation complex appears to be 10. Thus the histidine transporter of *S. typhimurium* lacks an N-terminal transmembrane segment from each of its hydrophophic domains and therefore contains five transmembrane segments per domain (Higgins et al., Nature 298, 723-727 (1982). The MalF protein of the *E. coli* maltose transporter contains an N-terminal extension of hydrophobic sequence which bears two additional transmembrane segments, bringing the total for this hydrophobic domain to 8 (Overduin et al. 1988). The N-terminal extension can be deleted, however, without loss of function of this transporter (Ehrmann et al. 1990). Although the number of segments required for formation of a functional translocator is suggested by these studies, there exists no data on the precise structure of the transmembrane segments themselves. These sequences are assumed to have an α-helical form, but this has not been proven and the structure of the entire translocation complex within the plasma membrane remains to be elucidated.

In order to span the lipid bilayer, a minimum of 20 amino acids is required and sequences believed to form transmembrane segments have been identified using hydrophobicity scales. Hydrophobicity scales assign values to individual amino acid residues indicating the degree of hydrophobicity of each molecule (Kyte and Doolittle 1982; Engleman et al. 1986). These values are based on experimental data (solubility measurements of amino acids in various solvents, analysis of side chains within soluble proteins) and theoretical considerations, and allow prediction of secondary structure in novel sequence with reasonable accuracy. Analysis using hydrophobicity measurements indicates those stretches of a protein sequence which have the hydrophobic properties consistent with a transmembrane helix.

With a few exceptions, there is little or no significant amino acid sequence similarity between the transmembrane domains of two different transporters. This lack of sequence similarity is not inconsistent with the apparent function of these hydrophobic domains. While these residues must be capable of forming the hydrophobic α-helical structures believed to transverse the plasma membrane, many amino acid residues are hydrophobic and can contribute to the formation of an α-helix.

Considerable, if as yet inexplicable, sequence similarity has been detected in comparisons of the transmembrane domains of the yeast STE6, human MDR and *E*. *coli* HlyB hemolysin transporters [Gros et al., Cell 47, 371 (1986); McGrath and Varchavsky, Nature 340, 400 (1989); Kuchler et al., EMBO J. 8, 3973 (1989)]. Other sequence similarities can be explained by gene duplication, as in the case of the transmembrane domains of rodent P-glycoproteins (Endicott et al. 1991). The transmembrane domain of the histidine transporter of S. *typhimurium* bears homology to that of the octopine uptake system of *Agrobacterium tumefaciens,* the latter two transporters translocate chemically similar substrates (Valdiva et al. 1991).

Study of mutant transport proteins has pointed to a role for the transmembrane sequences in the recognition of substrate. Thus maltose transporters in *E. coli* which gain the ability to translocate p-nitrophenyl-α-maltoside bear mutations in the transmembrane domain (Reyes et al. 1986). A mutation in transmembrane segment 11 of MDR has been shown to change the substrate specificity of that transporter (Gros et al. 1991) and mutation of charged residues in the transmembrane domain of CFTR changes its ion selectivity (Anderson et al. 1991).

Some aspects of the involvement of extramembrane loop sequences in transport function are being elucidated. In a number of bacterial transporters a short conserved motif is present on the cytoplasmic loop which connects transmembrane segments 4 and 5 [Dassa and Hofnung (1985)]. It has been hypothesized that this sequence interacts with the ATP-binding domains of these transport proteins; mutation of this conserved sequence will abolish transport function (Dassa 1990). Cytoplasmic loops may also be involved in substrate recognition. Thus the sequences following transmembrane segments 7 and 12 of the yeast **a**-factor transporter resemble sequences in the **a**-factor receptor, Ste3p, and may interact with the pheromone substrate (Kuchler et al. 1989). In fact, mutations in the cytoplasmic loops are known to alter the substrate specificity of a given transporter. The G185V mutation of human MDR, located in the loop between transmembrane segments 2 and 3, alters the interaction of that transporter with vinblastine and colchicine (Choi et al. 1988).

The ATP-binding domains are about 200 amino acids long, and domains from different transporters typically have a sequence identity of 30-50%. The conserved sequences include the "Walker motifs" which are associated with many nucleotide binding proteins. Walker, et al., *EMBO J.* 1:945-951 (1982). Sequence conservation extends over the length of the ATP-binding domain, not being limited to the Walker motifs. Furthermore, the ATP-binding domains of a single transporter exhibit greater sequence identity to one another than to the domains from two different transporters. Not all proteins containing a conserved ATP-binding domain are involved in transport, however. The cytoplasmic enzyme UvrA functions in DNA repair and the EF-3 protein of yeast is an elongation factor. Yet both proteins contain ATP-binding cassettes identifiable by sequence comparison.

ATP-binding domains are highly hydrophilic and, in the case of transporters, appear to reside at the cytoplasmic face of the membrane, anchored there via an association with the membrane-spanning domain of these proteins. The points of interaction between the transmembrane and ATP-binding domains have not been experimentally determined. Models of the structure of the nucleotide binding domain indicate that loop sequences may extend from the core of the structure to interface with the hydrophilic sequences which transverse the membrane (Hyde et al. 1990; Mimura et al. 1991). The two structural models, one based on adenylate cyclase and the other on *ras* p21 structure, predict a core nucleotide binding fold composed of five β-sheets with the Walker A motif (a glycine-rich loop) positioned to interact with ATP during hydrolysis. In addition, loop structures (two loops in one model, one large loop in the other) are predicted to extend from the core to couple the ATP-binding domain to other domains of the transporter. The coupling sequences transmit, most likely through conformational change, the energy of ATP hydrolysis to those portions of the molecule which are involved in transport.

Ste6 function is required for mating but the protein is not necessary for yeast survival (Wilson and Herskowiz 1984; Kuchler et al. 1989; McGrath and Varshavsky 1989). Ste6 is structurally homologous to the mammalian MDRs. Furthermore, it has been demonstrated that two mammalian MDR proteins, murine Mdr3 and human Mdr1, will substitute functionally for the yeast transporter in cells deleted for STE6 (Raymond et al. 1992; Kuchler and Thorner 1992). Yeast strains deleted for STE6 serve as a starting point for the design of screens to discover compounds that modulate the function of exogenous ABC transporters.

Two different yeast screens can be used to identify modulators of ABC transporter function. In the first instance, a mammalian protein that transports **a**-factor will serve as a target for potential inhibitors of transporter function. Thus, a yeast strain will be engineered to express a functional transporter, e.g. mammalian MDR1, which substitutes for the yeast Ste6 protein in the transport of **a-**factor. Furthermore, this strain will be engineered to respond in autocrine fashion to **a**-factor: e.g., so that the cells will be unable to grow on media containing galactose. This negative selection will depend on the expression of the GAL1 gene under the control of a pheromone-responsive promoter in a strain background which includes mutated versions of the GAL7 or GAL10 genes. Expression of GAL1 in the presence of galactose in such a strain background is toxic to cells. In the absence of a-factor transport, signaling down the pheromone response pathway would cease as would the consequent expression of the toxic gene. Cell growth in the presence of a test compound, or upon expression of a specific random peptide, would signal inhibition of transport function and the identification of a potential therapeutic.

In addition to inhibitors of MDR, compounds may be identified which interfere with the interaction of **a**-factor with the **a**-factor receptor. Such compounds can be discriminated by their inhibition of **a**-factor-induced growth arrest in a wild type Matα strain. Compounds may also impact at other points along the pheromone response pathway to inhibit signaling and these compounds will prevent signal transduction in a wild type Matα strain.

In a second screen, a mutant heterologous transporter (e.g., mutant CFTR) that is initially incapable of transporting **a**-factor or an **a**-factor-like peptide can be expressed in autocrine yeast deleted for endogenous Ste6. The cells will be capable of an autocrine response to the **a**-factor which those cells produce. Thus a pheromone-responsive promoter will control expression of a gene that confers an ability to grow in selective media. Such cells will permit identification of compounds which correct defects in the transporter and permit it to function in the export of pheromone analogues to the extracellular space. In this way, therapeutic peptides or other classes of chemical compounds could be identified which stabilize a mutant protein and allow normal processing, transport, localization to the plasma membrane and function. This strategy, if successful, may eliminate the need to "replace" some mutant genes with normal sequence, as envisioned in gene therapies, by recovering the function of mutant proteins through the correction of processing and/or localization defects.

In addition to "activators" of the mutant transporter, compounds may also be identified which are capable of initiating signalling from the **a**-factor receptor in the absence of transport by the endogenously expressed pheromone. These compounds will be distinguished by their ability to cause growth arrest in a wild type Matα strain. Compounds may also impact at other points along the pheromone pathway and can be discerned via an ability to initiate signalling in a wild type Matα strain in the absence of **a**-factor.

In a preferred embodiment, the exogenous protein produced by the yeast cells is one of the exogenous ABC transporters listed in Table 1.

### Exogenous G protein coupled receptors

In some instances, for a drug to cure a disease or alleviate its symptoms, the drug must be delivered to the appropriate cells, and trigger the proper "switches." The cellular switches are known as "receptors." Hormones, growth factors, neurotransmitters and many other biomolecules normally act through interaction with specific cellular receptors. Drugs may activate or block particular receptors to achieve a desired pharmaceutical effect. Cell surface receptors mediate the transduction of an "external" signal (the binding of a ligand to the receptor) into an "internal" signal (the modulation of a cytoplasmic metabolic pathway).

In many cases, transduction is accomplished by the following signaling cascade:
- An agonist (the ligand) binds to a specific protein (the receptor) on the cell surface.
- As a result of the ligand binding, the receptor undergoes an allosteric change which activates a transducing protein in the cell membrane.
- The transducing protein activates, within the cell, production of so-called "second messenger molecules."
- The second messenger molecules activate certain regulatory proteins within the cell that have the potential to "switch on" or "off" specific genes or alter some metabolic process.

This series of events is coupled in a specific fashion for each possible cellular response. The response to a specific ligand may depend upon which receptor a cell expresses. For instance, the response to adrenalin in cells expressing α-adrenergic receptors may be the opposite of the response in cells expressing β-adrenergic receptors.

The above "cascade" is idealized, and variations on this theme occur. For example, a receptor may act as its own transducing protein, or a transducing protein may act directly on an intracellular target without mediation by a "second messenger".

One family of signal transduction cascades found in eukaryotic cells utilizes heterotrimeric "G proteins." Many different G proteins are known to interact with receptors. G protein signaling systems include three components: the receptor itself, a GTP-binding protein (G protein), and an intracellular target protein.

The cell membrane acts as a switchboard. Messages arriving through different receptors can produce a single effect if the receptors act on the same type of G protein. On the other hand, signals activating a single receptor can produce more than one effect if the receptor acts on different kinds of G proteins, or if the G proteins can act on different effectors.

In their resting state, the G proteins, which consist of alpha (α), beta (β) and gamma (γ) subunits, are complexed with the nucleotide guanosine diphosphate (GDP) and are in contact with receptors. When a hormone or other first messenger binds to receptor, the receptor changes conformation and this alters its interaction with the G protein. This spurs the α subunit to release GDP, and the more abundant nucleotide guanosine triphosphate (GTP), replaces it, activating the G protein. The G protein then dissociates to separate the α subunit from the still complexed beta and gamma subunits. Either the Gα subunit, or the Gβγ complex, depending on the pathway, interacts with an effector. The effector (which is often an enzyme) in turn converts an inactive precursor molecule into an active "second messenger," which may diffuse through the cytoplasm, triggering a metabolic cascade. After a few seconds, the Gα converts the GTP to GDP, thereby inactivating itself. The inactivated Gα may then reassociate with the Gβγ complex.

Hundreds, if not thousands, of receptors convey messages through heterotrimeric G proteins, of which at least 17 distinct forms have been isolated. Although the greatest variability has been seen in the α subunit, several different β and γ structures have been reported. There are, additionally, several different G protein-dependent effectors.

Most G protein-coupled receptors are comprised of a single protein chain that is threaded through the plasma membrane seven times. Such receptors are often referred to as seven-transmembrane receptors (STRs). More than a hundred different STRs have been found, including many distinct receptors that bind the same ligand, and there are likely many more STRs awaiting discovery.

In addition, STRs have been identified for which the natural ligands are unknown; these receptors are termed "orphan" G protein-coupled receptors. Examples include receptors cloned by Neote et al. Cell 72, 415 (1993); Kouba et al. FEBS Lett. 321, 173 (1993); Birkenbach et al. J. Virol. 67, 2209 (1993).

The "exogenous G protein-coupled receptors" of the present invention may be any G protein-coupled receptor which is exogenous to the wild-type yeast cell which is to be genetically engineered for the purpose of the present invention. This receptor may be a plant or animal cell receptor. Screening for binding to plant cell receptors may be useful in the development of, e.g., herbicides. In the case of an animal receptor, it may be of invertebrate or vertebrate origin. If an invertebrate receptor, an insect receptor is preferred, and would facilitate development of insecticides. The receptor may also be a vertebrate, more preferably a mammalian, still more preferably a human, receptor. The exogenous receptor is also preferably a seven transmembrane segment receptor.

Suitable receptors include, but are not limited to, dopaminergic, muscarinic cholinergic, α-adrenergic, β-adrenergic, opioid (including delta and mu), cannabinoid, serotoninergic, and GABAergic receptors. Other suitable receptors are listed in Table 2. The term "receptor," as used herein, encompasses both naturally occurring and mutant receptors.

Many of these G protein-coupled receptors, like the yeast **a-** and α-factor receptors, contain seven hydrophobic amino acid-rich regions which are assumed to lie within the plasma membrane. Specific human G protein-coupled STRs for which genes have been isolated and for which expression vectors could be constructed include those listed in Table 2. Thus, the gene would be operably linked to a promoter functional in yeast and to a signal sequence functional in yeast. Suitable promoters include Ste2, Ste3 and gal10. Suitable signal sequences include those of Ste2, Ste3 and of other genes which encode proteins secreted by yeast cells. Preferably, the codons of the gene would be optimized for expression in yeast. See Hoekema et al., Mol. Cell. Biol., 7:2914-24 (1987); Sharp, et al., 14:5125-43 (1986).

The homology of STRs is discussed in Dohlman et al., Ann. Rev. Biochem., 60:653-88 (1991). When STRs are compared, a distinct spatial pattern of homology is discernable. The transmembrane domains are often the most similar, whereas the N-and C-terminal regions, and the cytoplasmic loop connecting transmembrane segments V and VI are more divergent.

The functional significance of different STR regions has been studied by introducing point mutations (both substitutions and deletions) and by constructing chimeras of different but related STRs. Synthetic peptides corresponding to individual segments have also been tested for activity. Affinity labeling has been used to identify ligand binding sites.

It is conceivable that a foreign receptor which is expressed in yeast will functionally integrate into the yeast membrane, and there interact with the endogenous yeast G protein. More likely, either the receptor will need to be modified (e.g., by replacing its V-VI loop with that of the yeast STE2 or STE3 receptor), or a compatible G protein should be provided.

If the wild-type exogenous G protein-coupled receptor cannot be made functional in yeast, it may be mutated for this purpose. A comparison would be made of the amino acid sequences of the exogenous receptor and of the yeast receptors, and regions of high and low homology identified. Trial mutations would then be made to distinguish regions involved in ligand or G protein binding, from those necessary for functional integration in the membrane. The exogenous receptor would then be mutated in the latter region to more closely resemble the yeast receptor, until functional integration was achieved. If this were insufficient to achieve functionality, mutations would next be made in the regions involved in G protein binding. Mutations would be made in regions involved in ligand binding only as a last resort, and then an effort would be made to preserve ligand binding by making conservative substitutions whenever possible.

Preferably, the yeast genome is modified so that it is unable to produce the endogenous **a-** and α-factor receptors in functional form. Otherwise, a positive assay score might reflect the ability of a peptide to activate the endogenous G protein-coupled receptor, and not the receptor of interest.

### G protein

When the PSP surrogate is an exogenous G protein-coupled receptor, the yeast cell must be able to produce a G protein which is activated by the exogenous receptor, and which can in turn activate the yeast effector(s). It is possible that the endogenous yeast G protein will be sufficiently homologous to the "cognate" G protein which is natively associated with the exogenous receptor for coupling to occur. More likely, it will be necessary to genetically engineer the yeast cell to produce a foreign Gα subunit which can properly interact with the exogenous receptor. For example, the Gα subunit of the yeast G protein may be replaced by the Gα subunit natively associated with the exogenous receptor (or by a protein substantially homologous with that cognate Gα subunit).

Dietzel and Kurjan, Cell, 50:1001 (1987) demonstrated that rat Gαs functionally coupled to the yeast Gβγ complex. However, rat Gαi2 complemented only when substantially overexpressed, while Gα0 did not complement at all. Kang, et al., Mol. Cell. Biol., 10:2582 (1990). Consequently, with some foreign Gα subunits, it is not feasible to simply replace the yeast Gα.

Preferably, the yeast Gα subunit is replaced by a chimeric Gα subunit in which a portion, e.g., at least about 20, more preferably at least about 40, amino acids, which is substantially homologous with the corresponding residues of the amino terminus of the yeast Gα, is fused to a sequence substantially homologous with the main body of a mammalian (or other exogenous) Gα. While 40 amino acids is the suggested starting point, shorter or longer portions may be tested to determine the minimum length required for coupling to yeast Gβγ and the maximum length compatible with retention of coupling to the exogenous receptor. It is presently believed that only the final 10 or 20 amino acids at the carboxy terminus of the Gα subunit are required for interaction with the receptor.

This chimeric Gα subunit will interact with the exogenous receptor and the yeast Gβγ complex, thereby permitting signal transduction. While use of the endogenous yeast Gβγ is preferred, if a foreign or chimeric Gβy is capable of transducing the signal to the yeast effector, it may be used instead.

### Protein kinases

Mitogen-activated protein kinase (MAP kinase) and its activator, MAP kinase kinase or MEK, are believed to be key molecules in the transduction of intracellular signals in mammalian cells. The activity of MAPK, a serine/threonine protein kinase, has been shown to depend on its phosphorylation by the dually specific MEK at tyrosine and threonine residues. MEK activity, in turn, depends on its phosphorylation on serine and threonine by a third kinase, MAP kinase kinase kinase, or MEKK, whose function in some systems is fulfilled by the protooncogene product Raf1p.

An essential part of the *S. cerevisiae* pheromone signalling pathway is comprised of a protein kinase cascade composed of the products of the *STE11, STE7,* and *FUS3/KSS1* genes (the latter pair are distinct and functionally redundant). Functional studies have established the dependence of FUS3p activity on tyrosine and threonine phosphorylation by STE7p, whose activity is regulated by its phosphorylation by STE11p. A second protein kinase cascade, responsive to protein kinase C, has been identified in *S. cerevisiae.* When this pathway is disrupted, yeast cells lose their ability to grow in media of low osmotic strength. Although its components have not been characterized to the same extent as that of the mating pathway cascade, sequence analysis identifies BCK1p as a MEKK, MKK1p/MKK2p as MEKs, and MPK1p as a MAPK.

Kinase signalling pathways appear to be conserved among eukaryotes. Thus significant sequence homology is found between *Xenopus* MAP kinase and the products of the following yeast kinase genes: FUS3, KSS1, MPK1 (*S*. *cerevisiae)* and Spk1 *(Schizosaccharomyces pombe*). In addition, mammalian MEK has been found to be homologous to the products of STE7 *(S. cerevisiae)* and Byr1 (*S*. *pombe)* [Crews et al. Science 258, 478 (1992)]. Functional homologies between some kinases has been demonstrated through substitution of heterologous kinase genes in yeast kinase deletion mutants. Thus *Xenopus* MAP kinase will complement an mpk1Δ mutant in *S. cerevisiae* (however, this kinase will not substitute for Fus3p or Ksslp function in the same organism) [Lee et al. Mol. Cell. Biol. 13, 3067 (1993)]. Both mammalian and *Xenopus* MAP kinase will substitute for Spk1 function in *S. pombe* [Neiman et al. Molec. Biol. Cell. 4, 107 (1993); Gotoh et al. Molec. Cell. Biol. 13, 6427 (1993)]. Rabbit MAP kinase kinase will complement a byr1 defect in *S. pombe* but only when co-expressed with Raf1 kinase; the latter thus appears to be a direct activator of MEK (Hughes et al. Nature 364, 349 (1993).

The use of the instant invention to screen for modulators of human MEK is described in Example 9.

### Cyclins

Members of another mammalian protein kinase family, one active in progression through the cell cycle, have been identified by complementation of cell cycle kinase mutants in yeast. The human homologue of p34cdc2 *(S. pombe*) and p34cdc28 (*S. cerevisiae*), proteins which control the progression to DNA synthesis and mitosis in yeast, was identified by complementation of a cdc2 mutation in S. *pombe* [Lee and Nurse, Nature 327, 31-35 (1987)]. CDK2, a second human p34 homologue, was identified by functional complementation of p34cdc28 mutations in S. cerevisiae [Elledge and Spottswood, EMBO J. 10, 2653 (1991)]. Activation of p34 depends on its association with regulatory subunits, termed cyclins. Tight control of cyclin expression as well as the inherent instability of these proteins once expressed contribute to a regulated activation of p34 kinase and progression through the cell cycle.

A number of putative G1 human cyclins have been identified through their ability to substitute for the yeast G1 cyclins, CLN1, CLN2 and CLN3, in the pheromone signalling pathway. Thus human cyclins C, D and E were identified by their ability to rescue cln⁻ yeast from growth arrest [Lew et al., Cell 66, 1197 (1991)]. It has also been demonstrated that other classes of human cyclins can substitute functionally for the CLN proteins in yeast. The human cyclins A, B1 and B2 (cyclins normally associated with governance of mitosis) will also function as G1 cyclins in *S*. *cerevisiae* [Lew et al., Cell 66, 1197 (1991)].

Certain cyclins are periodically accumulating regulators of the p34 kinase (p34cdc2 in humans and p34cdc28 in *S. cerevisiae).* The p34 kinase is functionally conserved from yeast to humans and the activity of this threonine/serine-specific protein kinase is required if cells are to progress through several checkpoints in the cell cycle, including one that is termed START. START occurs late in the G1 phase and is the switch point for cells between the quiescent state and proliferation. The kinase is activated at discrete points in the cell cycle through its association with specific cyclins, regulatory subunits which are also functionally conserved among eukaryotes. Three cyclins appear to operate in progression through START in *S. cerevisiae,* CLN1, CLN2 and CLN3 (Hadwiger et al. 1989; Cross 1988; Nash 1988). The sequences of the CLN proteins bear some homology to those of mammalian A- and B-type cyclins; these proteins are believed to regulate S (DNA synthesis) and M (mitotic) phases of the mammalian cell cycle.

Sequence comparisons among the cyclin proteins identified in different species indicates that a region of high sequence conservation is contained within an approximately 87 residue domain that generally comprises central cyclin sequence but which is located near the amino terminus of the yeast G1 cyclins. This conserved domain is termed the "cyclin box". A second region of homology shared by most of the cyclins is a C-terminal sequence rich in proline, glutamate, serine, threonine and aspartate residues flanked by basic amino acids that is termed a PEST motif (Rogers et al. 1986). PEST motifs are found in unstable proteins and are believed to signal for constitutive ubiquitin-mediated degradation. The degradation of cyclins A and B is signalled via a different sequence, a "mitotic destruction motif" (Glotzer et al. 1991), that is not shared by other mammalian cyclins.

Sequence comparisons made between the yeast CLN proteins and the human A, B, C, D and E cyclins indicate the existence of appreciable homologies (Lew et al. 1991). Across the most conserved regions, including the cyclin box, human cyclin C bears 18% sequence identity both to human cyclins D and E and to the yeast CLN proteins. Human cyclins D and E appear to be more related to human A- and B-type cyclins (33% identical) than to the yeast CLNs (24% identical).

All human cyclins identified to date will substitute functionally for yeast cyclins. In fact, the mammalian cyclins C, D1 and E were identified through their ability to complement defective CLN function in yeast (Lew et al. 1991). Mammalian A-and B-type cyclins also substitute functionally for the CLN proteins in yeast, therefore this ability cannot definitively mark a mammalian cyclin as one that would operate in G1. However, the cyclins C, D1 and E have been shown to be expressed in G1 in mammalian cells and the expression pattern of cyclin E during the cell cycle most closely parallels the expression patterns observed for the yeast G1 cyclins (Lew et al. 1991; Koff et al. 1991).

In mammalian cells, cyclin C mRNA accumulates early in G1 while cyclin E accumulates late in that phase. D cyclin mRNA levels are insufficient to allow tracking of expression patterns in human cells and the role of this cyclin is therefore not clear (Lew et al. 1991). In mouse cells, the D1 gene, CYL1, is expressed in the G1 phase and the D1 gene appears to be regulated by colony-stimulating factor 1 (Matsushime et al. 1991). Expression of D1 cyclin is highly growth factor-dependent and therefore may not be an integral part of the internal cell cycle control mechanism, but may occur only in response to external signalling (Scherr 1993). The PRAD1 gene, found overexpressed in some parathyroid adenomas is identical to the D1 gene (Motokura et al. 1991). D1 has also been found to be overexpressed in a glioblastoma cell line (Xiong et al. 1991) and is subject to deregulation by gene amplification (Lammie et al. 1991; Keyomarsi and Pardee 1993). Deregulation of D1 occurs by unknown mechanisms in some lymphomas, squamous cell tumors and breast carcinomas (Bianchi et al. 1993). This protein is involved in activating the growth of cells and therefore, deregulated expression of this gene appears to be an oncogenic event. Some evidence exists that the E-type cyclin may function in the G1 to S transition in human cells: this cyclin binds to and activates p34cdc2 protein in extracts of human lymphoid cells in G1, the protein is associated with histone H1 kinase activity in HeLa cells (Koff et al. 1991) and cyclin E mRNA is specifically expressed in late G1 in HeLa cells (Lew et al. 1991).

It has been hypothesized that p34 cdc2 acts at discrete transition points in the cell cycle by phosphorylating varying substrates. The phosphorylating activity is manifest upon association of the kinase with cyclins which are differentially expressed throughout the cycle of the cell. These different cyclins may alter the substrate specificity of the kinase or may alter its catalytic efficiency (Pines and Hunter 1990). Obvious potential substrates for the cdc2 kinase are transcription factors that control cell-cycle-stage-specific gene transcription.

Disruption of any one of the three CLN genes in yeast does not appreciably affect cell growth, however, upon disruption of all of the CLN genes, cells arrest in G1. In addition, in response to mating pheromone, the CLN proteins are inhibited and yeast cell growth is arrested. Two genes whose products inhibit cyclin activity have been identified in *S. cerevisiae*. The products of the FAR1 and FUS3 genes inhibit CLN2 and CLN3 function, respectively. With pheromone signalling, the levels of Far1p and Fus3p increase, the G1 cyclins do not accumulate, the CDC28p kinase remains inactive, and cell growth is arrested in G1. These observations suggest that inhibitors of the cyclin proteins, inhibitors of a productive association between the cyclins and the kinase, or inactivators of the kinase can foster cellular growth arrest.

By contrast, cyclins which are uninhibitable appear to function as uncontrolled positive growth regulators. High level expression of the CLN proteins is a lethal condition in yeast cells. Data indicate that the loss of controlled expression of cyclin D1 through chromosomal breakage, chromosomal translocation or gene amplification can promote oncogenicity in mammalian cells (Xiong et al. 1991; Lammie et al. 1991; Bianchi et al. 1993). In addition, it appears that events that disrupt the control of cyclin expression and control of cyclin function can result in bypass of the G1 checkpoint and dysregulated cellular growth. The cyclin proteins which operate in G1 to promote cellular proliferation would be ideal targets for therapeutics aimed at control of cell growth. Candidate surrogate proteins for substitution in the yeast pathway for identification of such therapeutics include human cyclins C, D and E. All three proteins are normally expressed during the G1 phase of the mammalian cell cycle and are thus candidate mediators of the commitment of cells to proliferate.

Examples of compounds which are known to act in G1 to prevent entry into S phase are transforming growth factor β (TGF-β) and rapamycin. Rapamycin, an immunosuppressant, inhibits the activity of cyclin E-bound kinase. This macrolide acts in G1 to prevent the proliferation of IL-2-stimulated T lymphocytes (Scherr 1993). TGF-β has been shown to prevent progression from G1 to S phase in mink lung epithelial cells (Howe et al. 1991). TGF-β appears to interfere with activation of the kinase, perhaps by reducing the stability of the complex which the kinase forms with cyclin E (Koff 1993).

A strain of yeast cells bearing inactive CLN1, CLN2 and CLN3 genes and an integrated chimeric gene encoding a Gall promoter-driven human CLN sequence (see DL1 cells, Lew et al. Cell 66, 1197 (1991) will serve as a tester strain. The Gall promoter permits high level expression when cells are grown in the presence of galactose but this promoter is repressed when cells are grown on glucose. Yeast cells so engineered are nonviable on glucose due to an absence of expression of functional cyclin. These yeast, however, proliferate on galactose-containing medium due to expression of the human cyclin sequence. Exposure of this strain to an inhibitor of cyclin function would render the cells incapable of growth, even on galactose medium, i.e., the cells would growth arrest in the presence or absence of galactose. This phenotype could serve as an indication of the presence of an exogenously applied cyclin inhibitor but would not be useful as a screen for the identification of candidate inhibitors from members of a random peptide library. Growth arrest of a subset of cells in an otherwise growing population is useless as an indicator system. Therefore, in order to identify random peptide inhibitors of mammalian cyclins, a two stage screen is envisioned.
i. A two-hybrid system described by Fields and Song (Nature 340, 245 (1989) permits the detection of protein-protein interactions in yeast. GAL4 protein is a potent activator of transcription in yeast grown on galactose. The ability of GAL4 to activate transcription depends on the presence of an N-terminal sequence capable of binding to a specific DNA sequence (UAS_{G)} and a C-terminal domain containing a transcriptional activator. A sequence encoding a protein, "A", may be fused to that encoding the DNA binding domain of the GAL4 protein. A second hybrid protein may be created by fusing sequence encoding the GAL4 transactivation domain to sequence encoding a protein "B". If protein "A" and protein "B" interact, that interaction serves to bring together the two domains of GAL4 necessary to activate transcription of a UAS_{G}-containing gene. In addition to co-expressing plasmids encoding both hybrid proteins, yeast strains appropriate for the detection of protein-protein interactions using this two-hybrid system would contain a GAL1-lacZ fusion to permit detection of transcription from a UAS_{G} sequence. These strains should also be deleted for endogenous GAL4 and for GAL80, a negative regulator of GAL4.
   In a variation of the two-hybrid system just described, the GAL4 DNA binding domain would be fused to a human cyclin sequence. In addition, oligonucleotides encoding random peptides would be ligated to sequence encoding the GAL4 transactivation domain. Co-transformation of appropriate yeast strains with plasmids encoding these two hybrid proteins and screening for yeast expressing β-galactosidase would permit identification of yeast expressing a random peptide sequence capable of interacting with a human cyclin. Identification of peptides with that capability would be the goal of this first stage of screening.
ii. Once random peptides capable of interacting with a human cyclin of interest had been identified, second stage screening could commence. The second screen would permit the identification of peptides that not only bound to human cyclin but, through that interaction, inhibited cyclin activation of the cell cycle-dependent kinase and, thus, cellular proliferation. Thus, candidate peptides would be expressed, individually, in yeast lacking CLN1, CLN2 and CLN3 but expressing a human CLN sequence, as described above. Those peptides, expression of which does not permit growth of the tester strain on galactose, would be presumed cyclin inhibitors.
   An advantage to this two-stage approach to the identification of potential cyclin inhibitors is the high probability that random peptide sequences selected in stage one interact with human cyclin proteins. A subsequently determined ability of that sequence to cause growth arrest of the tester yeast on galactose would be a strong indication that the growth arrest was due to a direct effect of the peptide on the cyclin and not on another protein, e.g., the cell cycle dependent kinase. Though a strong indication, such a result would not be an absolute indication and verification of the inhibitory effect on cyclin function could be obtained *in vitro* through biochemical assay.

### Screening and Selection

A marker gene is a gene whose expression causes a phenotypic change which is screenable or selectable. If the change is selectable, the phenotypic change creates a difference in the growth or survival rate between cells which express the marker gene and those which do not. If the change is screenable, the phenotype change creates a difference in some detectable characteristic of the cells, by which the cells which express the marker may be distinguished from those which do not. Selection is preferable to screening.

The marker gene may be coupled to the yeast pheromone pathway so that expression of the marker gene is dependent on activation of the G protein. This coupling may be achieved by operably linking the marker gene to a pheromone-responsive promoter. The term "pheromone-responsive promoter" indicates a promoter which is regulated by some product of the yeast pheromone signal transduction pathway, not necessarily pheromone per se. In one embodiment, the promoter is activated by the pheromone pathway, in which case, for selection, the expression of the marker gene should result in a benefit to the cell. A preferred marker gene is the imidazoleglycerol phosphate dehydratase gene (HIS3). If a pheromone responsive promoter is operably linked to a beneficial gene, the cells will be useful in screening or selecting for agonists. If it is linked to a deleterious gene, the cells will be useful in screening or selecting for antagonists.

Alternatively, the promoter may be one which is repressed by the pheromone pathway, thereby preventing expression of a product which is deleterious to the cell. With a pheromone-repressed promoter, one screens for agonists by linking the promoter to a deleterious gene, and for antagonists, by linking it to a beneficial gene.

Repression may be achieved by operably linking a pheromone-induced promoter to a gene encoding mRNA which is antisense to at least a portion of the mRNA encoded by the marker gene (whether in the coding or flanking regions), so as to inhibit translation of that mRNA. Repression may also be obtained by linking a pheromone-induced promoter to a gene encoding a DNA-binding repressor protein, and incorporating a suitable operator site into the promoter or other suitable region of the marker gene.

Suitable positively selectable (beneficial) genes include the following: *URA3, LYS2, HIS3, LEU2, TRP1; ADE1,2,3,4,5,7,8 ; ARG1,3,4,5,6,8; HIS1,4,5; ILV1,2,5; THR1,4; TRP2,3,4,5; LEU1,4; MET2,3,4,8,9,14,16,19; URA1,2,4,5,10; HOM3,6; ASP3; CHO1; ARO 2,7; CYS3; OLE1; INO1,2,4; PRO1,3* Countless other genes are potential selective markers. The above are involved in well-characterized biosynthetic pathways.

The imidazoleglycerol phosphate dehydratase (IGP dehydratase) gene (HIS3) is preferred because it is both quite sensitive and can be selected over a broad range of expression levels. In the simplest case, the cell is auxotrophic for histidine (requires histidine for growth) in the absence of activation. Activation leads to synthesis of the enzyme and the cell becomes prototrophic for histidine (does not require histidine). Thus the selection is for growth in the absence of histidine. Since only a few molecules per cell of IGP dehydratase are required for histidine prototrophy, the assay is very sensitive.

In a more complex version of the assay, cells can be selected for resistance to aminotriazole (AT), a drug that inhibits the activity of IGP dehydratase. Cells with low, fixed level of expression of HIS3 are sensitive to the drug, while cells with higher levels are resistant. The amount of AT can be selected to inhibit cells with a basal level of HIS3 expression (whatever that level is) but allow growth of cells with an induced level of expression. In this case selection is for growth in the absence of histidine and in the presence of a suitable level of AT.

In appropriate assays, so-called counterselectable or negatively selectable genes may be used. Suitable genes include: URA3 (orotidine-5'-phosphate decarboxylase; inhibits growth on 5-fluoroorotic acid), LYS2 (2-aminoadipate reductase; inhibits growth on α-aminoadipate as sole nitrogen source), CYH2 (encodes ribosomal protein L29; cycloheximide-sensitive allele is dominant to resistant allele), CAN1 (encodes arginine permease; null allele confers resistance to the arginine analog canavanine), and other recessive drug-resistant markers.

The natural response to induction of the yeast pheromone response pathway is for cells to undergo growth arrest. This is the preferred way to select for antagonists to a ligand/receptor pair that induces the pathway. An autocrine peptide antagonist would inhibit the activation of the pathway; hence, the cell would be able to grow. Thus, the FAR1 gene may be considered an endogenous counterselectable marker. The FAR1 gene is preferably inactivated when screening for agonist activity.

The marker gene may also be a screenable gene. The screened characteristic may be a change in cell morphology, metabolism or other screenable features. Suitable markers include beta-galactosidase (Xgal, C₁₂FDG, Salmon-gal, Magenta-Gal (latter two from Biosynth Ag)), alkaline phosphatase, horseradish peroxidase, exo-glucanase (product of yeast exb1 gene; nonessential, secreted); luciferase; and chloramphenicol transferase. Some of the above can be engineered so that they are secreted (although not β-galactosidase). The preferred screenable marker gene is beta-galactosidase; yeast cells expressing the enzyme convert the colorless substrate Xgal into a blue pigment. Again, the promoter may be pheromone-induced or pheromone-inhibited.

### Yeast Cells

The yeast may be of any species that possess a G protein-mediated signal transduction pathway and which are cultivatable. Suitable species include Kluyverei lactis, Schizosaccharomyces pombe, and Ustilago maydis; Saccharomyces cerevisiae is preferred. Either Gα or Gβγ may be the activator of the "effecter." (It is suspected that in some species, both Gα-activated and Gβγ-activated effectors exist.) The term "yeast", as used herein, includes not only yeast in a strictly taxonomic sense (i.e., unicellular organisms), but also yeast-like multicellular fungi with pheromone responses mediated by the mating pathway.

The yeast cells of the present invention may be used to test peptides for the ability to interact with an exogenous G protein-coupled receptor or other PSP surrogate. The yeast cells must express both the exogenous G protein-coupled receptor (or other PSP surrogate), and a complementary G protein (or other PSPs necessary for the PSP surrogate to function in the pheromone system, if need be after activation by a drug), and these molecules must be presented in such a manner that a "readout" can be obtained by means of the pheromone response pathway (which may be modified to improve the readout).

For a readout to be possible, a gene encoding a selectable or screenable trait must be coupled to the G protein-mediated signal transduction pathway so that the level of expression of the gene is sensitive to the presence or absence of a signal, i.e., binding to the coupled exogenous receptor. This gene may be an unmodified gene already in the pathway, such as the genes responsible for growth arrest. It may be a yeast gene, not normally a part of the pathway, that has been operably linked to a "pheromone-responsive" promoter. Or it may be a heterologous gene that has been so linked. Suitable genes and promoters were discussed above.

It will be understood that to achieve selection or screening, the yeast must have an appropriate phenotype. For example, introducing a pheromone-responsive chimeric HIS3 gene into a yeast that has a wild-type HIS3 gene would frustrate genetic selection. Thus, to achieve nutritional selection, an auxotrophic strain is wanted.

The yeast cells of the present invention optionally possess one or more of the following characteristics:
(a) the endogenous FAR1 gene has been inactivated;
(b) the endogenous SST2 gene, and/or other genes involved in desensitization, has been inactivated;
(c) the endogenous pheromone **(a**- or α-factor) receptor gene has been inactivated; and
(d) the endogenous pheromone genes have been inactivated.

"Inactivation" means that production of a functional gene product is prevented or inhibited. Inactivation may be achieved by deletion of the gene, mutation of the promoter so that expression does not occur, or mutation of the coding sequence so that the gene product is inactive. Inactivation may be partial or total. Mutants with inactivated supersensitivity-related genes can be identified by conventional genetic screening procedures. The far1 gene was identified as an α-factor resistant mutant that remained blue (with fusl-lacZ) on α-factor/Xgal. far2, as it turns out, is the same as fus3. Supersensitive mutants could be identified as constitutive weak blue colonies expressing fus1-lacZ on Xgal, or as strains that can mate more proficiently with a poor pheromone-secreter.

The DNA sequences of (a) the α- and **a**-factor genes, (b) the α- and **a**-factor receptors, (c) the FAR1 gene, (d) the SST2 gene, and (e) the FUS1 promoter have been reported in the following references:
MF**a**1 and MF**a**2: AJ Brake, C Brenner, R Najarian, P Laybourn, and J Merryweather. Structure of Genes Encoding Precursors of the Yeast Peptide Mating Pheromone a-Factor. In Protein Transport and Secretion. Gething M-J, ed. Cold Spring Harbor Lab, New York, 1985.
MFα1 and MFα2: Singh, A. EY Chen, JM Lugovoy, CN Chang, RA Hitzeman et al. 1983. Saccharomyces cerevisiae contains two discrete genes coding for the α-pheromone. Nucleic Acids Res. 11:4049; J Kurjan and I Herskowitz. 1982. Structure of a yeast pheromone gene (MF): A putative α-factor precursor contains four tandem copies of mature α-factor. Cell 30:933.
STE2 and STE3: AC Burkholder and LH Hartwell. 1985. The yeast α-factor receptor: Structural properties deduced from the sequence of the *STE2* gene. Nucleic Acids Res. 13:8463; N Nakayama, A Miyajima, and K Arai. 1985. Nucleotide sequences of *STE2* and *STE3,* cell type-specific sterile genes from *Saccharomyces cerevisiae.* EMBO J. 4:2643; DC Hagen, G McCaffrey, and GF Sprague, Jr. 1986. Evidence the yeast *STE3* gene encodes a receptor for the peptide pheromone **a**-factor: Gene sequence and implications for the structure of the presumed receptor. Proc Natl Acad Sci 83:1418.
FAR1: F Chang and I Herskowitz. 1990. Identification of a gene necessary for cell cycle arrest by a negative growth factor of yeast: FAR1 is an inhibitor of a G1 cyclin, CLN2. Cell 63:999.
SST2: C Dietzel and J Kurjan. 1987. Pheromonal regulation and sequence of the *Saccharomyces cerevisiae SST2* gene: A model for desensitization to pheromone. Mol Cell Biol 7: 4169.
FUS1: J Trueheart, JD Boeke, and GR Fink. 1987. Two genes required for cell fusion during yeast conjugation: Evidence for a pheromone-induced surface protein. Mol Cell Biol 7:2316.

The various essential and optional features may be imparted to yeast cells by, e.g., one or more of the following means: isolation of spontaneous mutants with one or more of the desired features; mutation of yeast by chemical or radiation treatment, followed by selection; and genetic engineering of yeast cells to introduce, modify or delete genes.

Other explicit characteristics desirable in strains of yeast designed to be used as screening devices for inhibitors/activators of PSP surrogates are discussed in subsections dealing specifically with each molecular target.

### Peptide

The term "peptide" is used herein to refer to a chain of two or more amino acids, with adjacent amino acids joined by peptide (-NHCO-) bonds. Thus, the peptides of the present invention include oligopeptides, polypeptides, and proteins. Preferably, the peptides of the present invention are 2 to 200, more preferably 5 to 50, amino acids in length. The minimum peptide length is chiefly dictated by the need to obtain sufficient potency as an activator or inhibitor. The maximum peptide length is only a function of synthetic convenience once an active peptide is identified.

For initial studies in which the cognate PSP was a yeast pheromone receptor, a 13-amino acid peptide was especially preferred as that is the length of the mature yeast α-factor.

### Peptide Libraries

A "peptide library" is a collection of peptides of many different sequences (typically more than 1000 different sequences), which are prepared essentially simultaneously, in such a way that, if tested simultaneously for some activity, it is possible to characterize the "positive" peptides.

The peptide library of the present invention takes the form of a yeast cell culture, in which essentially each cell expresses one, and usually only one, peptide of the library. While the diversity of the library is maximized if each cell produces a peptide of a different sequence, it is usually prudent to construct the library so there is some redundancy.

In the present invention, the peptides of the library are encoded by a mixture of DNA molecules of different sequence. Each peptide-encoding DNA molecule is ligated with a vector DNA molecule and the resulting recombinant DNA molecule is introduced into a yeast cell. Since it is a matter of chance which peptide-encoding DNA molecule is introduced into a particular cell, it is not predictable which peptide that cell will produce. However, based on a knowledge of the manner in which the mixture was prepared, one may make certain statistical predictions about the mixture of peptides in the peptide library.

It is convenient to speak of the peptides of the library as being composed of constant and variable residues. If the nth residue is the same for all peptides of the library, it is said to be constant. If the nth residue varies, depending on the peptide in question, the residue is a variable one. The peptides of the library will have at least one, and usually more than one, variable residue. A variable residue may vary among any of two to all twenty of the genetically encoded amino acids; the variable residues of the peptide may vary in the same or different manner. Moreover, the frequency of occurrence of the allowed amino acids at a particular residue position may be the same or different. The peptide may also have one or more constant residues.

There are two principal ways in which to prepare the required DNA mixture. In one method, the DNAs are synthesized a base at a time. When variation is desired, at a base position dictated by the Genetic Code, a suitable mixture of nucleotides is reacted with the nascent DNA, rather than the pure nucleotide reagent of conventional polynucleotide synthesis.

The second method provides more exact control over the amino acid variation. First, trinucleotide reagents are prepared, each trinucleotide being a codon of one (and only one) of the amino acids to be featured in the peptide library. When a particular variable residue is to be synthesized, a mixture is made of the appropriate trinucleotides and reacted with the nascent DNA.

Once the necessary "degenerate" DNA is complete, it must be joined with the DNA sequences necessary to assure the expression of the peptide, as discussed in more detail below, and the complete DNA construct must be introduced into the yeast cell.

### Expression

The expression of a peptide-encoding gene in a yeast cell requires a promoter which is functional in yeast. Suitable promoters include the promoters for metallothionein, 3-phosphoglycerate kinase (Hitzeman et al., J. Biol. Chem. 255, 2073 (1980) or other glycolytic enzymes (Hess et al., J. Adv. Enzyme Reg. 7, 149 (1968); and Holland et al. Biochemistry 17, 4900 (1978)), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Suitable vectors and promoters for use in yeast expression are further described in R. Hitzeman et al., EPO Publn. No. 73,657. Other promoters, which have the additional advantage of transcription controlled by growth conditions, are the promoter regions for alcohol dehydrogenase 2, isocytochrome C, acid phosphatase, degradative enzymes associated with nitrogen metabolism, and the aforementioned metallothionein and glyceraldehyde-3-phosphate dehydrogenase, as well as enzymes responsible for maltose and galactose utilization. Finally, promoters that are active in only one of the two haploid mating types may be appropriate in certain circumstances. Among these haploid-specific promoters, the pheromone promoters MF**a**1 and MFα1 are of particular interest.

In screens devised for a subset of PSP surrogates (e.g. kinases, cyclins) random peptide sequences need not be expressed in the context of yeast pheromone and need not be engineered for secretion or transport to the extracellular space. Libraries of random peptides may be expressed in a multiplicity of ways, including as portions of chimeric proteins, as in a two-hybrid protein system designed to signal protein-protein interactions. Random peptides need not necessarily substitute for yeast pheromones but can impinge on the pheromone pathway downstream of the interaction between pheromone and pheromone receptor (as in random peptide inhibitors of the kinases or of the cyclins).

In constructing suitable expression plasmids, the termination sequences associated with these genes, or with other genes which are efficiently expressed in yeast, may also be ligated into the expression vector 3' of the heterologous coding sequences to provide polyadenylation and termination of the mRNA.

### Vectors

The vector must be capable of replication in a yeast cell. It may be a DNA which is integrated into the host genome, and thereafter is replicated as a part of the chromosomal DNA, or it may be DNA which replicates autonomously, as in the case of a plasmid. In the latter case, the vector must include an origin of replication which is functional in the host. In the case of an integrating vector, the vector may include sequences which facilitate integration, e.g., sequences homologous to host sequences, or encoding integrases.

Besides being capable of replication in yeast cells, it is convenient if the vector can also be replicated in bacterial cells, as many genetic manipulations are more conveniently carried out therein. Shuttle vectors capable of replication in both yeast and bacterial cells include YEps, YIps, and the pRS series.

### Periplasmic Secretion

The cytoplasm of the yeast cell is bounded by a lipid bilayer called the plasma membrane. Between this plasma membrane and the cell wall is the periplasmic space. Peptides secreted by yeast cells cross the plasma membrane through a variety of mechanisms and thereby enter the periplasmic space. The secreted peptides are then free to interact with other molecules that are present in the periplasm or displayed on the outer surface of the plasma membrane. The peptides then either undergo re-uptake into the cell, diffuse through the cell wall into the medium, or become degraded within the periplasmic space.

The peptide library may be secreted into the periplasm by one of two distinct mechanisms, depending on the nature of the expression system to which they are linked. In one system, the peptide may be structurally linked to a yeast signal sequence, such as that present in the α-factor precursor, which directs secretion through the endoplasmic reticulum and Golgi apparatus. Since this is the same route that the receptor protein follows in its journey to the plasma membrane, opportunity exists in cells expressing both the receptor and the peptide library for a specific peptide to interact with the receptor during transit through the secretory pathway. This has been postulated to occur in mammalian cells exhibiting autocrine activation. Such interaction would likely yield activation of the linked pheromone response pathway during transit, which would still allow identification of those cells expressing a peptide agonist. For situations in which peptide antagonists to externally applied receptor agonist are sought, this system would still be effective, since both the peptide antagonist and receptor would be delivered to the outside of the cell in concert. Thus, those cells producing an antagonist would be selectable, since the peptide antagonist would be properly and timely situated to prevent the receptor from being stimulated by the externally applied agonist.

An alternative mechanism for delivering peptides to the periplasmic space is to use the ATP-dependent transporters of the STE6/MDR1 class. This transport pathway and the signals that direct a protein or peptide to this pathway are not as well characterized as is the endoplasmic reticulum-based secretory pathway. Nonetheless, these transporters apparently can efficiently export certain peptides directly across the plasma membrane, without the peptides having to transit the ER/Golgi pathway. We anticipate that at least a subset of peptides can be secreted through this pathway by expressing the library in context of the **a-**factor prosequence and terminal tetrapeptide. The possible advantage of this system is that the receptor and peptide do not come into contact until both are delivered to the external surface of the cell. Thus, this system strictly mimics the situation of an agonist or antagonist that is normally delivered from outside the cell. Use of either of the described pathways is within the scope of the invention.

The present invention does not require periplasmic secretion, or, if such secretion is provided, any particular secretion signal or transport pathway.

### Example 1: Development of Autocrine Yeast Strains

In this example, we describe a pilot experiment in which haploid cells were engineered to be responsive to their own pheromones (see Figure 1). (Note that in the examples, functional genes are capitalized and inactivated genes are in lower case.) For this purpose we constructed recombinant DNA molecules designed to:
i. place the coding region of *STE2* under the transcriptional control of elements which normally direct the transcription of *STE3.* This is done in a plasmid that allows the replacement of genomic *STE3* of *S.cerevisiae* with sequences wherein the coding sequence of *STE2* is driven by *STE3* transcriptional control elements.
ii. place the coding region of *STE3* under the transcriptional control of elements which normally direct the transcription of *STE2*. This is done in a plasmid which will allow the replacement of genomic *STE2* of *S.cerevisiae* with sequences wherein the coding sequence of *STE3* is driven by *STE2* transcriptional control elements.
The sequence of the STE2 gene is known see Burkholder A.C. and Hartwell L.H. (1985), "The yeast α-factor receptor: Structural properties deduced from the sequence of the STE2 gene," Nuc. Acids Res. 13, 8463; Nakayama N., Miyajima A., Arai K. (1985) "Nucleotide sequences of STE2 and STE3, cell type-specific sterile genes from *Saccharomyces cerevisiae,"* EMBO J. 4, 2643.

A 4.3 kb BamHI fragment that contains the entire STE2 gene was excised from plasmid YEp24-STE2 (obtained from J. Thorner, Univ. of California) and cloned into pALTER (Protocols and Applications Guide, 1991, Promega Corporation, Madison, WI). An SpeI site was introduced 7 nucleotides (nts) upstream of the ATG of STE2 with the following mutagenic oligonucleotide, using the STE2 minus strand as template (mutated bases are underlined and the start codon is in bold type):

5' GTTAAGAACCATATACTAGTATCAAAAATGTCTG 3'.

A second SpeI site was simultaneously introduced just downstream of the STE2 stop codon with the following mutagenic oligonucleotide (mutated bases are underlined and the stop codon is in bold type):

5' TGATCAAAATTTACTAGTTTGAAAAAGTAATTTCG 3'.

The BamHI fragment of the resulting plasmid (Cadus 1096), containing *STE2* with SpeI sites immediately flanking the coding region, was then subcloned into the yeast integrating vector YIp19 to yield Cadus 1143.

The STE3 sequence is also known. Nakayama N., Miyajima A., Arai K. (1985), "Nucleotide sequences of STE2 and STE3, cell type-specific sterile genes from *Saccharomyces cerevisiae*," EMBO J. 4, 2643; Hagen D.C., McCaffrey G., Sprague G.F. (1986), "Evidence the yeast STE3 gene encodes a receptor for the peptide pheromone **a**-factor: gene sequence and implications for the structure of the presumed receptor," Proc. Natl. Acad. Sci. 83, 1418. STE3 was made available by Dr. J. Broach as a 3.1 kb fragment cloned into pBLUESCRIPT-KS II (Stratagene, 11011 North Torrey Pines Road, La Jolla, CA 92037). STE3 was subcloned as a KpnI-XbaI fragment into both M13mp18 RF (to yield Cadus 1105) and pUC19 (to yield Cadus 1107). The two SpeI sites in Cadus 1107 were removed by digestion with SpeI, fill-in with DNA polymerase I Klenow fragment, and recircularization by blunt-end ligation. Single-stranded DNA containing the minus strand of *STE* 3 was obtained using Cadus 1105 and SpeI sites were introduced 9 nts upstream of the start codon and 3 nts downstream of the stop codon of *STE3* with the following mutagenic oligonucleotides, respectively:

5' GGCAAAATACTAGTAAAATTTTCATGTC 3'
5' GGCCCTTAACACACTAGTGTCGCATTATATTTAC 3'.

The mutagenesis was accomplished using the T7-GEN protocol of United States Biochemical (T7-GEN In *Vitro* Mutagenesis Kit, Descriptions and Protocols, 1991, United States Biochemical, P.O. Box 22400, Cleveland, Ohio 44122). The replicative form of the resulting Cadus 1141 was digested with AflII and KpnI, and the approximately 2 kb fragment containing the entire coding region of *STE3* flanked by the two newly introduced Spe I sites was isolated and ligated with the approximately 3.7 kb vector fragment of AflII- and KpnI-digested Cadus 1107, to yield Cadus 1138. Cadus 1138 was then digested with XbaI and KpnI, and the *STE3*-containing 2.8 kb fragment was ligated into the XbaI- and KpnI-digested yeast integrating plasmid pRS406 (Sikorski, R.S. and Hieter, P. (1989) "A System of Shuttle Vectors and Yeast Host Strains Designed for Efficient Manipulation of DNA in *Saccharomyces cerevisiae*", Genetics 122:19-27 to yield Cadus 1145.

The SpeI fragment of Cadus 1143 was replaced with the SpeI fragment of Cadus 1145 to yield Cadus 1147, in which the coding sequences of *STE3* are under the control of *STE2* expression elements. Similarly, the SpeI fragment of Cadus 1145 was replaced with the SpeI fragment of Cadus 1143 to yield Cadus 1148, in which the coding sequences of *STE2* are under the control of *STE3* expression elements. Using the method of pop-in/pop-out replacement (Rothstein, R. (1991) "[19] Targeting, Disruption, Replacement, and Allele Rescue: Integrative DNA Transformation in Yeast", Methods in Enzymology, 194:281-301), Cadus 1147 was used to replace genomic *STE2* with the *ste2-STE3* hybrid in a MATa cell and Cadus 1148 was used to replace genomic *STE3* with the *ste3-STE2* hybrid in a MATα cell. Cadus 1147 and 1148 contain the selectable marker URA3.

Haploid yeast of mating type **a** which had been engineered to express HIS3 under the control of the pheromone-inducible FUS1 promoter were transformed with CADUS 1147, and transformants expressing URA3 were selected. These transformants, which express both Ste2p and Ste3p, were plated on 5-fluoroorotic acid to allow the selection of clones which had lost the endogenous *STE2,* leaving in its place the heterologous, integrated *STE3.* Such cells exhibited the ability to grow on media deficient in histidine, indicating autocrine stimulation of the pheromone response pathway.

Similarly, haploids of mating type α that can express HIS3 under the control of the pheromone-inducible FUS1 promoter were transformed with CADUS 1148 and selected for replacement of their endogenous STE3 with the integrated STE2. Such cells showed, by their ability to grow on histidine-deficient media, autocrine stimulation of the pheromone response pathway.

### Example 2: Strain Development

In this example, yeast strains are constructed which will facilitate selection of clones which exhibit autocrine activation of the pheromone response pathway. To construct appropriate yeast strains, we will use: the YIp-STE3 and pRS-STE2 knockout plasmids described above, plasmids available for the knockout of FAR1, SST2, and HIS3, and mutant strains that are commonly available in the research community. The following haploid strains will be constructed, using one-step or two-step knockout protocols described in Meth. Enzymol 194:281-301, 1991:
1. *MATα ste3::STE2::ste3 far1 sst2 FUS1::HIS3*
2. *MATa ste2::STE3::ste2 far1 sst2 FUS1::HIS3*
3*. MATα ste3::STE2::ste3 far1 sst2 mfα1 mfα2 FUS1::HIS3*
4*. MATa ste2::STE3::ste2 far1 sst2 mfa1 mfa2 FUS1::HIS3*
5*. MATa bar1 far1-1 fus1-HIS3 ste14::TRP1 ura3 trp1 leu2 his3*
6. *MATa mfa1 mfa2 far1-1 his3::fus1-HIS3 ste2-STE3 ura3 met1 ade1 leu2*

Strains 1 and 2 will be tested for their ability to grow on histidine-deficient media as a result of autocrine stimulation of their pheromone response pathways by the pheromones which they secrete. If these tests prove successful, strain 1 will be modified to inactivate endogenous *MFα1* and *MFα2.* The resulting strain 3, *MATα far1 sst2 ste3::STE2::ste3 FUS1::HIS3 mfa1 mfa2,* should no longer display the selectable phenotype (i.e., the strain should be auxotrophic for histidine). Similarly, strain 2 will be modified to inactivate endogenous *MFa1* and *MFa2.* The resulting strain 4, *MATa far1 sst2 ste2::STE3::ste2 FUS1::HIS3 mfa1 mfa2*, should be auxotrophic for histidine. The uses of strains 5 and 6 are outlined in Examples 3 and 4 below.

### Example 3: Peptide Library

In this example, a synthetic oligonucleotide encoding a peptide is expressed so that the peptide is secreted or transported into the periplasm.
i. The region of *MFα1* which encodes mature α-factor has been replaced via single-stranded mutagenesis with restriction sites that can accept oligonucleotides with AflII and BglII ends. Insertion of oligonucleotides with AflII and BglII ends will yield plasmids which encode proteins containing the MFα1 signal and leader sequences upstream of the sequence encoded by the oligonucleotides. The MFα1 signal and leader sequences should direct the processing of these precursor proteins through the pathway normally used for the transport of mature α-factor.
   The MFα1 gene, obtained as a 1.8 kb EcoRI fragment from pDA6300 (J. Thorner, Univ. of California) was cloned into pALTER (see Figure 2) in preparation for oligonucleotide-directed mutagenesis to remove the coding region of mature α-factor while constructing sites for acceptance of oligonucleotides with AflII and BclI ends. The mutagenesis was accomplished using the minus strand as template and the following mutagenic oligonucleotide: 
5'CTAAAGAAGAAGGGGTATCTTTGCTTAAGCTCGAGATCTCGACTGAT AACAACAGTGTAG
3'
   A HindIII site was simultaneously introduced 7 nts upstream of the MFα1 start codon with the oligonucleotide: 5'CATACACAATATAAAGCTTTAAAAGAATGAG 3'.
   The resulting plasmid, Cadus 1214, contains a HindIII site 7 nts upstream of the MFα1 initiation codon, an AflII site at the positions which encode the KEX2 processing site in the MFα1 leader peptide, and XhoI and BglII sites in place of all sequences from the leader-encoding sequences up to and including the normal stop codon. The 1.5 kb HindIII fragment of Cadus 1214 therefore provides a cloning site for oligonucleotides to be expressed in yeast and secreted through the pathway normally travelled by endogenous α-factor.
   A sequence comprising the *ADH1* promoter and 5' flanking sequence was obtained as a 1.5 kb BamHI-HindIII fragment from pAAH5 (Ammerer, G. (1983) "[11] Expression of Genes in Yeast Using the ADCI Promoter", Academic Press, Inc., Meth. Enzymol. 101, 192-201 and ligated into the high copy yeast plasmid pRS426 (Christianson, T.W et al. (1992) "Multifunctional yeast high-copy-number shuttle vectors", Gene 110:119-122) (see Figure 3). The unique XhoI site in the resulting plasmid was eliminated to yield Cadus 1186. The 1.5 Kb HindIII fragment of Cadus 1214 was inserted into HindIII-digested Cadus 1186; expression of sequences cloned into this cassette initiates from the *ADH1* promoter. The resulting plasmid, designated Cadus 1215, can be prepared to accept oligonucleotides with AflII and BclI ends by digestion with those restriction endonucleases. The oligonucleotides will be expressed in the context of MFα1 signal and leader peptides (Figure 4).
   Two single-stranded oligonucleotide sequences (see below) are synthesized, annealed, and repetitively filled in, denatured, and reannealed to form double-stranded oligonucleotides that, when digested with AflII and BclI, can be ligated into the polylinker of the expression vector, Cadus 1215. The two single-stranded oligonucleotides have the following sequences: 5' G CTA CTT AAG CGT GAG GCA GAA GCT 3' and 5' C GGA TGA TCA (NNN)n AGC TTC TGC CTC ACG CTT AAG TAG C 3' where N is any chosen nucleotide and n is any chosen integer. Yeast transformed with the resulting plasmids will secrete -- through the α-factor secretory pathway -- peptides whose amino acid sequence is determined by the particular choice of N and n (Figure 4).
ii. The region of *MF**a**1* which encodes mature **a**-factor will be replaced via single stranded mutagenesis with restriction sites that can accept oligonucleotides with XhoI and AflII ends. Insertion of oligonucleotides with XhoI and AflII ends will yield plasmids which encode proteins containing the MF**a**1 leader sequences upstream of the sequence encoded by the oligonucleotides. The MF**a**1 leader sequences should direct the processing of these precursor proteins through the pathway normally used for the transport of mature **a**-factor.

*MFA1*, obtained as a BamHI fragment from pKK1 (provided by J. Thorner and K. Kuchler), was ligated into the BamHI site of pALTER (Promega) (Figure 5). Using the minus strand of *MFA1* as template, a HindIII site was inserted by oligonucleotide-directed mutagenesis just 5' to the *MFA1* start codon using the following oligonucleotide:

5' CCAAAATAAGTACAAAGCTTTCGAATAGAAATGCAACCATC.

A second oligonucleotide was used simultaneously to introduce a short polylinker for later cloning of synthetic oligonucleotides in place of MFA1 sequences. These MFA1 sequences encode the C-terminal 5 amino acids of the 21 amino acid leader peptide through to the stop codon:
5'GCCGCTCCAAAAGAAAAGACCTCGAGCTCGCTTAAGTTCTGCGTACA AAAACGTTGTTC 3'. The 1.6 kb HindIII fragment of the resulting plasmid, Cadus 1172, contains sequences encoding the MFA1 start codon and the N-terminal 16 amino acids of the leader peptide, followed by a short polylinker containing XhoI, SacI, and AflII sites for insertion of oligonucleotides. The 1.6 kb HindIII fragment of Cadus 1172 was ligated into HindIII-digested Cadus 1186 (see above) to place expression of sequences cloned into this cassette under the control of the *ADH1* promoter. The SacI site in the polylinker was made unique by eliminating a second SacI site present in the vector. The resulting plasmid, designated Cadus 1239, can be prepared to accept oligonucleotides with XhoI and AflII ends by digestion with those restriction endonucleases for expression in the context of MF**a**1 leader peptides (Figure 6).

Two single-stranded oligonucleotide sequences (see below) are synthesized, annealed, and repetitively filled in, denatured, and reannealed to form double-stranded oligonucleotides that, when digested with AflII and BglII, can be cloned into the polylinker of the expression vector, Cadus 1239. The two single-stranded oligonucleotides used for the cloning have the following sequences: 5' GG TAC TCG AGT GAA AAG AAG GAC AAC 3' 
5' CG TAC TTA AGC AAT AAC ACA (NNN)n GTT GTC CTT CTT TTC ACT CGA
GTA CC 3'
 where N is any chosen nucleotide and n is any chosen integer. Yeast transformed with the resulting plasmids will transport -- through the pathway normally used for the export of **a**-factor -- farnesylated, carboxymethylated peptides whose amino acid sequence is determined by the particular choice of N and n (Figure 6).

### Example 4. Peptide Secretion/Transport.

This example demonstrates the ability to engineer yeast such that they secrete or transport oligonucleotide-encoded peptides (in this case their pheromones) through the pathways normally used for the secretion or transport of endogenous pheromones.

### Autocrine MATa strain CY588:

A MATa strain designed for the expression of peptides in the context of MFα1 (i.e., using the MFα1 expression vector, Cadus 1215) has been constructed. The genotype of this strain, which we designate CY588, is *MATa bar1 far1-1 fus1-HIS3 ste14::TRP1 ura3 trp1 leu2 his3*. The *bar1* mutation eliminates the strain's ability to produce a protease that degrades α-factor and that may degrade some peptides encoded by the cloned oligonucleotides; the *far1* mutation abrogates the arrest of growth which normally follows stimulation of the pheromone response pathway; an integrated FUS1-HIS3 hybrid gene provides a selectable signal of activation of the pheromone response pathway; and, finally, the *ste14* mutation lowers background of the FUS1-HIS3 readout. The enzymes responsible for processing of the MFα1 precursor in MATα cells are also expressed in MAT**a** cells (Sprague and Thorner, in The Molecular and Cellular Biology of the Yeast *Saccharomyces:* Gene Expression, 1992, Cold Spring Harbor Press), therefore, CY588 cells should be able to secrete peptides encoded by oligonucleotides expressed from plasmid Cadus 1215.

### Secretion of peptides in the context of yeast α-factor:

Experiments were performed to test: 1. the ability of Cadus 1215 to function as a vector for the expression of peptides encoded by synthetic oligonucleotides; 2. the suitability of the oligonucleotides, as designed, to direct the secretion of peptides through the α-factor secretory pathway; 3. the capacity of CY588 to secrete those peptides; and 4. the ability of CY588 to respond to those peptides that stimulate the pheromone response pathway by growing on selective media. These experiments were performed using an oligonucleotide which encodes the 13 amino acid α-factor; i.e., the degenerate sequence (NNN)ₙ in the oligonucleotide cloned into Cadus 1215 (see above) was specified (n=13) to encode this pheromone. CY588 was transformed with the resulting plasmid (Cadus 1219), and transformants selected on uracil-deficient medium were transferred to histidine-deficient medium supplemented with a range of concentrations of aminotriazole (an inhibitor of the *HIS3* gene product that serves to reduce background growth). The results, shown in Figure 7, demonstrate that the synthetic oligonucleotide, expressed in the context of MFα1 by Cadus 1215, conferred upon CY588 an ability to grow on histidine-deficient media supplemented with aminotriazole. In summation, these data indicate that: 1. CY588 is competent for the secretion of a peptide encoded by the (NNN)n sequence of the synthetic oligonucleotide cloned into and expressed from Cadus 1215; and 2. CY588 can, in an autocrine fashion, respond to a secreted peptide which stimulates its pheromone response pathway, in this case by α-factor binding to STE2.

### Autocrine Mata strain CY599:

A MATa strain designed for the expression of peptides in the context of MFA1 (i.e., using the MFA1 expression vector, Cadus 1239) has been constructed. The genotype of this strain, designated CY599, is *MATa mfa1 mfa2 far1-1 his3::fus1-HIS3 ste2-STE3 ura3 met1 ade1 leu2.* In this strain, Cadus 1147 (see above) was used to replace STE2 with a hybrid gene in which the STE3 coding region is under the control of expression elements which normally drive the expression of STE2. As a result, the **a**-factor receptor replaces the α-factor receptor. The genes which encode **a**-factor are deleted from this strain; the *far1* mutation abrogates the arrest of growth which normally follows stimulation of the pheromone response pathway; and the FUS1-HIS3 hybrid gene (integrated at the HIS3 locus) provides a selectable signal of activation of the pheromone response pathway. CY599 cells were expected to be capable of the transport of **a**-factor or **a**-factor-like peptides encoded by oligonucleotides expressed from Cadus 1239 by virtue of expression of the endogenous yeast transporter, Ste6.

### Transport of peptides by the yeast a-factor pathway:

Experiments were performed to test: 1. the ability of Cadus 1239 to function as a vector for the expression of peptides encoded by synthetic oligonucleotides; 2. the suitability of the oligonucleotides, as designed, to direct the export of farnesylated, carboxymethylated peptides through the pathway normally used by **a**-factor; 3. the capacity of CY599 to export these peptides; and 4. the ability of CY599 to respond to those peptides that stimulate the pheromone response pathway by growing on selective media. These tests were performed using an oligonucleotide which encodes the 12 amino acid **a**-factor; specifically, the degenerate sequence (NNN)ₙ in the oligonucleotide cloned into Cadus 1239 (see above) (with n=12) encodes the peptide component of **a**-factor pheromone. CY599 was transformed with the resulting plasmid (Cadus 1220), and transformants selected on uracil-deficient medium were transferred to histidine-deficient medium supplemented with a range of concentrations of aminotriazole. The results, shown in Figure 8, demonstrate that the synthetic oligonucleotide, expressed in the context of MFA1 by Cadus 1220, conferred upon CY599 enhanced aminotriazole-resistant growth on histidine-deficient media. In summation, these data indicate: 1. Cadus 1220 and the designed oligonucleotide are competent to direct the expression and export of a farnesylated, carboxymethylated peptide encoded by the (NNN)ₙ sequence of the synthetic oligonucleotide; and 2. CY599 can, in an autocrine fashion, respond to a farnesylated, carboxymethylated peptide that stimulates its pheromone response pathway, in this case signaling initiates as **a**-factor binds to STE3.

### Example 5. Proof of Concept

This example will demonstrate the utility of the autocrine system for the discovery of peptides which behave as functional pheromone analogues. By analogy, this system can be used to discover peptides that productively interact with any pheromone receptor surrogates.

CY588 (see strain 5, Example 2 above) will be transformed with CADUS 1215 containing oligonucleotides encoding random tridecapeptides for the isolation of functional α-factor analogues (Figure 4). CY599 (see strain 6, Example 2 above) will be transformed with CADUS 1239 containing oligos of random sequence for the isolation of functional **a**-factor analogues (Figure 6). Colonies of either strain which can grow on histidine-deficient media following transformation will be expanded for the preparation of plasmid DNA, and the oligonucleotide cloned into the expression plasmid will be sequenced to determine the amino acid sequence of the peptide which presumably activates the pheromone receptor. This plasmid will then be transfected into an isogenic strain to confirm its ability to encode a peptide which activates the pheromone receptor. Successful completion of these experiments will demonstrate the potential of the system for the discovery of peptides which can activate membrane receptors coupled to the pheromone response pathway.

Random oligonucleotides to be expressed by the expression plasmid CADUS 1215 will encode tridecapeptides constructed as 5' CGTGAAGCTTAAGCGTGAGGCAGAAGCT (NNK)BTGATCATCCG, where N is any nucleotide, K is either T or G at a ratio of 40:60 (see Proc Natl Acad Sci 87:6378, 1990; *ibid* 89:5393, 1992), and the AflII and BclI sites are underlined. This oligonucleotide is designed such that: the AflII and BclI sites permit inserting the oligos into the AflII and BglII site of CADUS 1215 (see Figure 4); the HindIII site just 5' to the AflII site in the 5' end of the oligo allows future flexibility with cloning of the oligos; the virtual repeat of GAGGCT and the GAGA repeats which are present in the wild-type sequence and which can form triple helixes are changed without altering the encoded amino acids. The random oligonucleotides described above will actually be constructed from the following two oligos: 5' CGTGAAGCTTAAGCGTGAGGCAGAAGCT and 5' CGGATGATCA(MNN)13AGCTTCTG, where M is either A or C at a ratio of 40:60. The oligos will be annealed with one another and repetitively filled in, denatured, and reannealed (Kay et al, Gene, 1993). The double-stranded product will be cut with AflII and BclI and ligated into the AflII-and BglII-digested CADUS 1215. The BglII/BclI joint will create a TGA stop codon for termination of translation of the randomers (Figure 4). Because of the TA content of the Afl overhang, the oligos will be ligated to the AflII-and BglII-digested pADC-MFα at 4°C.

Random oligonucleotides to be expressed by the expression plasmid CADUS 1239 will encode monodecapeptides constructed as 5' GGTACTCGAGTGAAAAGAAGGACAAC (NNK)11TGTGTTATTGCTTAAGTACG, where N is any nucleotide, K is either T or G at a ratio of 40:60 (see Proc Natl Acad set 87:6378, 1990; *ibid* 89:5393, 1992), and the XhoI and AflII sites are underlined. When cloned into the XhoI and AflII sites of CADUS 1239 the propeptides expressed under the control of the ADH1 promoter will contain the entire leader peptide of MFa1, followed by 11 random amino acids, followed by triplets encoding CVIA (the C-terminal tetrapeptide of wild-type **a**-factor). Processing of the propeptide should result in the secretion of dodecapeptides which contain 11 random amino acids followed by a C-terminal, farnesylated, carboxymethylated cysteine.

Using the procedure described above, the oligonucleotides for expression in CADUS 1239 will actually be constructed from the following two oligos: 5' GGTACTCGAGTGAAAAGAAGGACAAC and 5' CGTACTTAAGCAATAACAca(MNN)11GTTGTCC, where M is either A or C at a ratio of 40:60, and the XhoI and AflII sites are underlined.

### Example 6

### Drug screens designed to permit discovery of molecules which modulate the function of ATP-dependent transmembrane transporters:

The availability of cloned DNA encoding the related proteins human Mdr1, human CFTR and human MRP, will allow the construction of yeast strains expressing these molecules. The resultant strains will be essential to the design of microbiological assays which can be used to probe the function of these proteins and to discover molecules capable of inhibiting or enhancing their function in cellular resistance to chemotherapeutics or in ion transport. The present assay makes use of the transport of the yeast mating pheromone **a**-factor from autocrine yeast expressing a human protein capable of substituting for yeast Ste6.
A. MFa1- and Mdr1-containing plasmids obtained from Karl Kuchler (University of Vienna) for use in these experiments:
   (1) pYMA177 (denoted Cadus 1067), see Figure 9.
   (2) pKK1 includes sequence encoding MF**a**1 in YEp351; **a**-factor is overexpressed from this plasmid due to increased plasmid copy number
   (3) pHaMDR1(wt) provides wild type Mdr1 cDNA in a retroviral vector (initially obtained from Michael Gottesman, NIH).
B. Plasmids constructed at Cadus for use in these experiments:
A 1.5 kb BamHI-BglII fragment which includes MFa1 sequence was derived from pYMA177 and ligated to BamHI-digested pYMA177 to yield Cadus 1079. Cadus 1079 was digested with BglII and recircularized to delete a 950 bp fragment containing sequence encoding the G185V mutant of human Mdr1; the resultant plasmid is Cadus 1093. pHaMDR1 was digested with BglII to allow isolation of a 965 bp fragment containing wild type human Mdr1 (G185) sequence. The 965 bp BglII fragment was inserted into BglII-digested Cadus 1093 to yield Cadus 1097. The Cadus 1097 construct was verified by sequencing using dideoxy nucleotides. To yield Cadus 1164, pYMA177 was digested with BamHI and recircularized to eliminate the sequence encoding MFa1. Cadus 1165 was constructed by ligating a 700 bp BglII-BamHI fragment from pYMA177 to the large BamHI to BglII fragment of pYMA177.

This results in the removal of both the 1.6 kb MFa BamHI fragment and the 965 bp BglII fragment encoding human Mdr1 (G185V); the resulting plasmid is Cadus 1165.

Cadus 1176 resulted from the ligation of a 965 bp BglII fragment from pHaMDR1, containing sequence encoding wild type human Mdr1, to BglII-digested Cadus 1165.
pRS426 (Cadus 1019) served as a URA3 control plasmid.

The final plasmid array used in these experiments is as follows:

| | no Mdr1 | mutant Mdr1 (G185V) | wt Mdr1 (G185) |
|---|---|---|---|
| **a**-factor overexpression | 1065 | 1079 | 1097 |
| no **a**-factor overexpression | 1019 | 1164 | 1176 |

### Evidence for the expression of human Mdr1 constructs in yeast.

CADUS plasmids 1065, 1079 and 1097 as well as a URA3 control plasmid (pRS426 = 1019) were transformed into a *ura3*^{*-*}, *ste6*^{*-*} strain of yeast (WKK6 = CY20 = MAT**a** *ura3-1 leu2-3, 112 his3-11,15 trp1-1 ade2-1 can1-100 ste6::HIS3,* obtained from Karl Kuchler). Individual transformants were grown overnight in SD-URA media and lawns containing approximately 5x10⁶ cells were poured onto YPD plates in 3 ml of YPD top agar. Sterile filter disks were placed on the plates after the top agar had solidified, and 5 ml of DMSO or 5mM valinomycin in DMSO were spotted onto the filter disks. By this assay, expression of mutant Mdr1 from plasmid 1079 in WKK6 cells conferred weak resistance to valinomycin while expression of wild type Mdr1 from plasmid 1097 conferred complete resistance.

### Attempts to assay Mdr1 activity by a-factor transport in a two cell system.

Several attempts to demonstrate mating of WKK6 transformed with the various human Mdr1 constructs failed. This is in contrast to published experiments utilizing the mouse *mdr3* gene, in which a partial complementation of mating deficiency was seen (Raymond et al., 1992).

A "halo" assay was performed by patching WKK6 cells containing the various Mdr1 constructs (1019, 1065, 1079 & 1097) onto a lawn of Matα cells which are supersensitive to **a**-factor (CY32 = MATα *leu2-3,112 trp1-289 ura3-52 his3*1 sst2*2 GAL+*)*.* Although all halos were much smaller than those produced by *STE6*^{*+*} cells, a small difference in halo size was observed with the relative order 1097 > 1079 ~ 1065 > 1019. This indicates that **a**-factor can be transported by the wild-type human Mdr1 protein expressed in yeast deleted for *STE6,* however, the halo assay does not appear to be amenable to rapid drug screening.

The halo assay detects **a**-factor secreted from *ste6* Mdr1 strains by growth arrest of cells present in the indicator lawn. An alternative, and potentially more sensitive, method makes use of the transcriptional response to pheromone signaling. A strain with an **a**-factor responsive *HIS3* gene (CY104 = MATα *ura3 leu2 trp1 his3 fus1-HIS3 can1 ste14::TRP1)* was cross-streaked with *STE6*^{*+*} (CY19 = W303-1a = MATa *ura3-1 leu2-3,112 his3-11,15 trp1-1 ade2-1 can1-100)* or *ste6* (WKK6 = CY20 = MATa *ura3-1 leu2-3,112 his3-11,15 trp1-1 ade2-1 can1-100 ste6::HIS3)* strains containing various plasmids. The cross-streaking was performed on a plate lacking histidine and tryptophan so that only the CY104 indicator strain would grow if stimulated by **a**-factor. The order of **a**-factor secretion seen by this method was CY58>CY61≈CY62≈ CY63>CY60 where: CY58 = CY19 *(STE6*^{*+*}, 1019), CY60 = CY20(*ste6,* 1019), CY61 = CY20 (*ste6*⁻, 1065), CY62 = CY20 (*ste6*⁻, 1079) and CY63 = CY20 (*ste6*⁻, 1097). Thus, the difference in **a**-factor production between *STE6*^{*+*} and *ste6*⁻ and between **a**-factor overproduction or not is detectable in this system, whereas the activity of Mdr1 in secreting **a**-factor is not. It was not clear from these experiments whether the signal generated from the *ste6*⁻, **a**-factor overproducing strains was due to an alternate pathway for **a**-factor secretion or the release of **a**-factor from lysed cells.

### Assay of Mdr1 activity by a-factor transport in an autocrine system.

A single strain system for the detection of Mdr1-mediated **a**-factor transport was constructed in order to improve sensitivity and reproducibility and to circumvent the potential false signal of **a**-factor release from lysed cells (a cell concentration-dependent phenomenon). A strain was constructed (CY293 = MAT**a** *ura3 leu2 trp1 his3 fus1-HIS3 can1 ade2-1 ste2-STE3 ste6::TRP1)* which could respond to **a**-factor by growing on media lacking histidine and in which the only impediment to the secretion of **a**-factor is the lack of a functional *STE6* gene. Indeed, the immediate precursor to this strain, which did contain a functional *STE6* gene, was able to grow vigorously on -HIS media containing 3 mM aminotriazole, whereas CY293 did not grow at all. The addition of aminotriazole, a competitive inhibitor of the HIS3 enzyme, is necessary to reduce the background growth of these strains.

The Mdr1-containing plasmids were introduced into CY293, and the transformants were streaked onto -HIS plates containing either 0.1 or 0.33 mM aminotriazole. The growth pattern thus generated was 1097 > 1067 >1065≈1176>1164≈1019. The *STE6*^{*+*} parent of CY293 exhibited a much more vigorous growth than any of these transformants. However, human Mdr1-mediated **a**-factor secretion is clearly detectable in this autocrine system. CY293 transformed with 1097 can be used to screen for drugs which enhance the activity of the Mdr1 protein (increased growth on -HIS +aminotriazole) as well as drugs which inhibit Mdr1 activity (decreased growth on -HIS +aminotriazole). In the latter case, controls must be designed to identify compounds which inhibit yeast growth in an Mdr1-independent fashion.

### Improvements on the autocrine yeast expressing Mdr1.

The results described above indicate that the human Mdr1 protein transports **a**-factor less efficiently than does the yeast STE6 protein. Attempts will be made to isolate mutant **a**-factor molecules which are transported more efficiently by human Mdr1 and yet which retain agonist activity on the **a**-factor receptor (STE3 protein). To do this, **a**-factor coding sequences will be chemically synthesized using "dirty" nucleotides and inserted into an **a**-factor expression cassette. An example of "dirty" synthesis would be to incorporate nucleotide from a mixture of 70% G and 10% each of A, T and C at a position in the **a**-factor sequence where G would normally appear. Using oligonucleotides generated in this manner, a diverse library of peptides can be expressed in yeast and screened to identify those peptides which retain the ability to signal to the STE3 protein but which are also a favorable substrate for transport by human Mdr1.

A second improvement to the system will be the addition of a pheromone-inducible "negative selection" marker. For instance, the FUS1 promoter can be connected to GAL1 coding sequences. Expression of GAL1 is toxic in the presence of galactose in strains which contain mutations in either the GAL7 or GAL10 genes. In the context of an autocrine Mdr1 strain, this selection system should render cells galactose-sensitive. Addition of a compound which inhibits the ability of the Mdr1 protein to secrete **a**-factor would allow this strain to grow on galactose-containing media. This selection system would also eliminate false positives due to lethality. Controls must still be designed to identify compounds which interfere at other points in the pheromone response pathway.

The third improvement in the system involves inactivation of yeast genes which function equivalently to mammalian MDR genes.

A network of genes involved in pleiotropic drug resistance (PDR) have been identified in yeast. This modification will be useful in any yeast screen designed to assay the interaction of compounds with intracellular targets.

The improved autocrine yeast strain, expressing human Mdr1, will be used to screen compound libraries for molecules which inhibit the transport function of this protein. In addition, Mfα expression cassettes containing oligonucleotides encoding random peptides will be expressed in the autocrine Mdr1 strain to identify peptides capable of inhibiting the transport of **a**-factor, or an **a**-factor analogue by Mdr1.

### Example 7.

### Identification of an analogue of yeast a-factor that is transported by wild type human CFTR.

This example describes the use of autocrine yeast strains to identify molecules capable of enhancing transport by dysfunctional ATP-dependent transmembrane transporters, e.g. mutant human CFTR proteins. The wild type human CFTR protein will not substitute for Ste6 function in yeast by transporting native **a**-factor pheromone (John Teem, unpublished observations) .

In order to maximally exploit autocrine yeast strains for the discovery of molecules which enhance mutant CFTR function, an **a**-factor-like peptide which can serve as a substrate for transport by CFTR will be identified. An **a**-factor analogue must also bind functionally to the pheromone receptor, Ste3, in order to initiate pheromone signalling. The CFTR transport substrate will be identified by expressing a randomly mutated MF**a** expression cassette in autocrine yeast deleted for Ste6 expression, but expressing the wild type human CFTR protein.

### Expression of mutant human CFTR proteins in autocrine yeast.

Transport of an **a**-factor-like peptide pheromone by CFTR will serve as the basis for the design of screens to be used in the identification of compounds which augment the transport function of CFTR proteins containing cystic fibrosis (CF) mutations. Based on studies done by Teem et al. (1993) using Ste6/CFTR chimeras, mutant CFTR is not expected to efficiently transport an **a**-factor analogue. Teem et al. (1993) have made chimeric proteins by substituting varying portions of the sequences encoding the first nucleotide binding domain of human CFTR for analogous sequence in yeast *STE6*. The chimeric proteins will transport native yeast **a**-factor when expressed in yeast. However, introduction of a CF mutation (ΔF508) reduces the ability of these proteins to transport the yeast pheromone. Teem et al. have also identified revertants in yeast, i.e., proteins containing second site mutations which restore the ability of chimeras bearing the CF mutation to transport **a**-factor. Introduction of the revertant mutations into defective CFTR protein expressed in mammalian cells decreased in part the processing and channel defects of the ΔF508 protein.

With the identification of an analogue of yeast **a**-factor that will serve as a substrate for transport by CFTR, mutations can be introduced directly into the human CFTR protein expressed in yeast, eliminating the necessity of creating chimeric proteins containing Ste6 sequence. This will permit the targeting of potential CF therapeutics to the entire human CFTR molecule. Mutations of interest include G551D, N1303K and ΔI507 in addition to ΔF508; these mutations are naturally occurring, give rise to cystic fibrosis in affected individuals, and appear to affect either the transport and processing of CFTR or the regulation of function of that protein (Welsh and Smith 1993). These mutations also rank among the most common alterations of CFTR thus far identified in CF patients.
If a peptide which will serve as a substrate for transport by CFTR cannot be identified, chimeric Ste6/CFTR proteins and unaltered **a**-factor can be utilized in screens based on autocrine strains of yeast. These strains offer distinct advantages in screening applications: easy adaptability to large-scale automation, simplicity and increased sensitivity when compared to traditional yeast mating cell assays of pheromone signaling, and the potential to employ the instant technology to identify active peptide structures.

### Identification of compounds that enhance transport of a-factor-like peptide by mutant CFTR.

Autocrine strains expressing mutant human CFTR protein will be capable of growth on rich media but, due to inadequate transport of and signaling by an **a**-factor analogue, will not grow efficiently on selective (histidine-deficient) media. Pheromone signaling in these strains initiates expression from a *FUS1* promoter sequence controlling transcription of the His3 enzyme; expression of His3 is required for growth on media lacking histidine. These strains will be used to screen compound libraries to identify molecules which reverse the inability of the mutant CFTR to transport **a**-factor analogue and permit growth of the yeast on histidine-deficient medium. Alternatively, active compounds would be capable of signaling to the **a**-factor receptor, Ste3, directly, or may interact with the pheromone response pathway elsewhere. Suitable controls would differentiate among these possibilities.

### Identification of random peptides that enhance transport by mutant CFTR.

Plasmids containing oligonucleotides which encode random peptides will be expressed in autocrine yeast bearing a mutant human CFTR. These peptides, expressed using α-factor-based expression cassettes, will be transported to the extracellular environment via the yeast secretory pathway. Peptides of interest will be identified by virtue of their ability to permit the growth of a mutant CFTR-containing strain on histidine-deficient medium.

Active peptides would permit transport of **a**-factor analogue by the mutant human CFTR. Alternatively, a peptide may interact at other points along the pheromone response pathway. Suitable controls would differentiate between these possible outcomes.

### Example 8.

### Prophetic Example of Substitution of Prohormone Convertase PC1 for Yeast KEX2.

The mammalian prohormone convertases PC1/PC3 and PC2 are involved in the proteolytic processing of proopiomelanocortin (POMC), with PC1 preferentially releasing adrenocorticotropic hormone (ACTH) and β-lipotropin and PC2 preferentially releasing β-endorphin, N-terminally extended ACTH containing the joining peptide (JP), and either α-melanocyte-stimulating hormone (α-MSH) or des-acetyl α-MSH (Benjannet, S., et al. (1991) Proc. Natl. Acad. Sci. USA 88:3564-3568; Seideh N.G. et al. (1992) FEBS Lett. 310, 235-239). By way of example, a yeast strain is described in which mammalian PC1 processes a chimeric pre-pro-POMC/α-factor peptide, permitting the secretion of mature α-factor and stimulation of the screening strain in an autocrine fashion to histidine protrotrophy. Autocrine strains will be constructed in which: 1. yeast KEX2 is disrupted; 2. yeast KEX2 activity is substituted with that of mammalian PC1; 3. a novel MFα construct containing the dibasic cleavage site recognized by PC1 (in place of the KEX2 recognition sequence) will be expressed; 4. production of mature α-factor will require PC1 activity; and 5. growth of the strain will be stimulated by the production of mature α-factor.

The genotype of the parental strain for this example is MAT**a** bar1::hisG far1-1 fus1-HIS3 ste14::TRP1 ura3 trp1 leu2 his3. Initially, the KEX2 allele of this strain will be disrupted using an integrating plasmid (p*kex2*Δ) encoding the flanking regions of the KEX2 locus with the entire coding regions from the initiator codon to the terminator codon deleted. Cleavage of p*kex2*Δ_with Bsu36I followed by transfection into the parental strain will result in integration of this plasmid into the KEX2 locus. Transformation can be scored as conversion to uracil prototrophy. Subsequent transfer of URA+ transformants to plates containing 5-fluoroorotic acid results in the growth of colonies with the *kex1*Δ_allele. Integration can be confirmed by Southern blot analysis and by colony PCR using oligonucleotide primers flanking the KEX2 locus. This *kex1*Δ_strain will be able to grow on histidine-deficient media in the presence of exogenenously added α-factor, but will not be able to grow in an autocrine fashion on histidine-deficient media once transfected with Cadus 1219 (URA3 2mu-ori REP3 AmpR f1-ori α-factor) since processing of the pre-pro-POMC/α-factor chimera expressed from Cadus 1219 requires KEX2 activity.

In the screening strain, PC1 activity will substitute for the deleted KEX2 activity in the maturation of α-factor peptide. PC1 cDNA (accession # M69196) obtained from mouse (Korner et al (1991) Proc. Natl. Acad. Sci USA 88:6834-6838) was found to encode a protein of 753 amino acids. Sequences encoding PC1 will be cloned into a high copy replicating plasmid (Cadus 1289). Yeast cells transformed with this plasmid will acquire the ability to grow on leucine-deficient media and will express high levels of PC1 protein due to the presence of the PGK promotor.

A hybrid gene encoding the prepro-region of human POMC (accession # K02406; Takahashi, H., et al (1983) Nucleic Acids Research 11:6847-6858) and the coding region of a single repeat of mature α-factor will be constructed in the following fashion. The prepro-region of human POMC will be amplified with an HindIII site at the 5' end and a BbsI site at the 3' end using VENT polymerase and the following primers: 5' GGGAAGCTT ***ATG***CCGAGATCGTGCTGCCAGCCGC 3' (HindIII site is underlined and initiation codon is italic bold) and antisense 5' GGGGAAGACTTCTGCCCTGCGCCGCTGCTGCC 3' (BbsI recognition is underlined), leaving the amino acid sequence -SSGAGQ**KR**- at the 3' end with a Bbs1 site leaving an overhang at the -KR- dibasic cleavage sequence. The coding region of α-factor will be amplified from Cadus 1219 with a Bbs1 site at the 5' end and a BglII site at the 3' end using the primers 5' GGGGAAGACCCGCAGGAGGCAGAAGCTT GGTTGCAG 3' (BbsI site is underlined) and 5' GGGAGATCT**TCA**GTACATTGGTTGGCC 3' (BglII site is underlined, termination codon is bold). The PCR fragment encoding the pre-pro segment of PCMC is restricted with HindIII and BbsI and gel purifed, the PCR fragment encoding_α-factor is cut with BbsI and BglII and gel purified, and Cadus 1215 is cut with BglII and partially with HindIII and the HindIII-BglII restricted vector containing the pAlter polylinker sequences is gel purified. Three-part ligation of the two PCR products with HindIII and BglII digested Cadus 1215 will yield a hybrid POMC/α-factor gene in which the first 104 amino acids residues are from POMC and the remaining 17 are from α-factor. The structure of this hybrid gene around the PC1 cleavage site is: -- RNSSSSGSSGAGO**KR**EAEA*WHWLQLKPGQPMY** where residues donated by POMC are underlined, the dibasic cleavage site is underlined bold, and the sequence of mature α-factor is in italics. The tetrapeptide -EAEA- juxtaposed between the dibasic cleavage site and the amino-terminal tryptophan of mature α-factor should be removed by the dipeptidyl aminopeptidase activity of ste13p.

Introduction of the PC1-encoding plasmid and the POMC/α-factor plasmid into a kex2Δ_strain with the genotype MATa bar1::hisG far1-1 fus1-HIS3 ste14::TRP1 ura3 trp1 leu2 his3 should result in autocrine growth that is dependent on PC1-mediated cleavage of the dibasic motif at the amino-terminal side of the single copy of α-factor encoded by the POMC/α-factor plasmid. That is, the autocrine behaviour of this strain should be due to expression of PC1. A suitable negative control is provided by expression of mammalian PC2 in place of PC1; the cleavage site of POMC that is inserted upstream of the α-factor gene is not recognized by PC2. Therefore, it should be possible to construct strains specific for PC1- versus PC2-dependent processing by judicious choice of cleavage sites from POMC to append to the 5' end of the α-factor gene. Compounds or random peptides that disrupt the autocrine growth of this strain but which do not have growth inhibitory effects when this strain is grown in histidine-containing media are potential inhibitors of PC1 activity.

### Example 9.

### Prophetic Example of Substitution of Human MEK (MAP Kinase Kinase) for Yeast STE7.

In order to develop a screen for compounds which act as inhibitors of a mammalian kinase, one could construct a yeast Strain that is deficient in STE7 activity, and which contains human MEK in a yeast expression vector. In addition, the strain would be equipped with reporter capacities, as described below.

To disrupt the yeast *STE7* gene, the following approach could be taken: pBluescriptKS+, a multicopy *E. coli* vector, is engineered to contain *STE7* sequences deleted for 5' noncoding and promoter sequence and for a sizeable portion of the coding region. The |ste7 sequence is then subcloned into pRS406¦ClaI, a Bluescript-based plasmid containing the yeast URA3 gene and the resulting plasmid, pRS406 |ClaI|ste7, is used to disrupt the wild type *STE7* gene as follows. pRS406|ste7 is digested with ClaI, and used to transform yeast strain CY252 (genotype *MAT***a** *ste14::TRP1 fus1-HIS3 far1-1 ura3 leu2 trp1 his3 ade2-1 met1*) to uracil prototrophy. Subsequent transfer of Ura+ transformants to media containing 5-fluoroorotic acid results in colonies containing the *ste7*| allele, which is confirmed by Southern analysis and by the inability of the strain to grow in the absence of histidine when stimulated with α pheromone.

To construct a plasmid capable of expressing human MEK in yeast cells, the following oligonucleotides are constructed: 5'-CCG**CGTCTC**ACATGCCCAAGAAGAAGCCG-3' (forward) and 5'-CCG**TCTAGA**TGCTGGCAGCGTGGG-3' (reverse). When used in a polymerase chain reaction (PCR) with human cDNA as template, these primers will direct the amplification of DNA encoding human MEK. To insert the human MEK gene into a yeast expression vector, the PCR product is digested with Esp3I and Xbal (bold sequences above) and ligated to the yeast-*E*. *coli* shuttle plasmid Cadus1289, previously digested with NcoI and Xbal. The resulting plasmid will replicate autonomously in yeast cells, confer leucine prototrophy to a yeast leu2 mutant, and direct the expression of MEK from the constitutive *PGK1* promoter.

When the above-described PGK1-MEK plasmid is introduced into the CY252-*ste*7| cells, it should restore their ability to grow in the absence of histidine when incubated with α pheromone, due to the ability of MEK to functionally replace STE7 in the pheromone response pathway and thereby effect the stimulation of the *fus1-HIS3* fusion situated in the chromosome. One could then screen for compounds which are able to reverse α pheromone-dependent growth in the absence of histidine, but which have no nonspecific toxic effect in the presence of histidine.

In an autocrine embodiment, the yeast cells made *ste7*¦ are of the genotype *MAT***a** *bar1::hisG far1-1 fus1-HIS3 ste14::trp1::LYS2 ura3 trp1 leu2 his3 lys2* (CY588trp). The procedure followed is exactly as above for CY252. After construction of CY588trp*ste7*|, the cells created are transformed with the plasmid expressing MEK, as well as with a plasmid capable of expressing secreted α pheromone. This transformed strain (CY588trp*ste7*| [MEK/MFα]) should be able to grow on media lacking histidine and containing high (20 22222222222222mM) amounts of 3-aminotriazole. The growth of this strain on this media should be strictly dependent on the presence of both plasmids (each necessary, neither sufficient). Compounds that interfere with this growth could be tested as above, with the exception that exogenously added α pheromone is unnecessary.

In addition, CY588trp*ste*7| [MEK/MFα] could be transformed with a plasmid library expressing cytoplasmically targeted random peptides. Those that interfere with the function of MEK would be identified by replica plating to media deficient in histidine (and potentially containing 3-aminotriazole); cells expressing such inhibitory peptides would be His. Such a screen could be streamlined with the addition of reporter constructs with the potential of negative selection, such as *fus1-URA3* or *fus1-GAL1*. In this case inhibitory peptides would confer on the target strain the ability to grow on 5-FOA- (for cells with diminished *fus1-URA3*) or galactose- (for cells with diminished *fus1-GAL1* in a *gal10*^{*-*} background) containing media.

Confirmatory tests would include biochemical assay of the activity of MEK (either *in vitro* or *in vivo*) in the presence of random peptides or other molecules identified as potential MEK inhibitors.

**TABLE 1:**

| ABC TRANSPORTERS* | | |
|---|---|---|
| Species | System | Substrate |
| Bacteria | | |
| *Salmonella typhimurium* | OppABCDF | Oligopeptides |
| *Streptococcus pneumoniae* | AmiABCDEF | Oligopeptides |
| *Bacillus subtilis* | Opp (SpoOK) | Oligopeptides |
| *E. coli* | Dpp | Dipeptides |
| *Bacilus subtilis* | DciA | Dipeptides |
| *S. typhimurium* | HisJQMP | Histidine |
| *E. coli* | HisJQMP | Histidine |
| *E. coli* | MalEFGK | Maltose |
| *S. typhimurium* | MalEFGK | Maltose |
| *Enterobacter aerogenes* | MalEFGK | Maltose |
| E. *coli* | UgpABCE | sn-Glycerol-3-phosphate |
| *E. coli* | AraFGH | Arabinose |
| *E. coli* | RbsACD | Ribose |
| *E. coli* | GlnHPQ | Glutamine |
| *S. typhimurium* | ProU (VWX) | Glycine-betaine |
| *E. coli* | ProU (VWX) | Glycine-betaine |
| *E. coli* | LivHMGF (JK) | Leucine-isoleucine-valine |
| *E. coli* | PstABC | Phosphate |
| *Pseudomonas stutzeri* | NosDYF | Copper |
| *E. coli* | ChlJD | Molybdenum |
| *E. coli* | CysPTWAM | Sulphate-Thiosulfate |
| *E*. *coli* | BtuCDE | Vitamin B12 |
| *E. coli* | FhuBCD | Fe³⁺ - ferrichrome |
| *E. coli* | FecBCDE | Fe³⁺-dicitrate |
| *S. marcescens* | SfuABC | Fe³⁺ |
| *Mycoplasma* | p37,29,69 | ? |
| *E. coli* | Phn/Psi | Alkyl-phosphonates (?) |
| *Streptomyces peucetius* | DrrAB | Daunomycin/Doxorub icin |
| *Streptomyces fradiae* | TlrC | Tylosin |
| *Staphylococcus* | MsrA | Erythromycin resistance |
| *Agrobacterium tumefaciens* | OccJQMP | Octopine |
| *E. coli* | HlyB | Haemolysin |
| Pasturella | LtkB | leukotoxin |
| *E. coli* | CvaB | Colicin V |
| *Erwinia chrysanthemi* | PrtD | Proteases |
| *Bordetella pertussis* | CyaB | Cyclolysin |
| *Streptococcus pneumoniae* | ComA | Competence factor |
| *Rhizobium meliloti* | NdvA | β-1,2-glucan |
| *Agrobacterium tumefaciens* | ChvA | β-1,2-glucan |
| *Haemophilus influenzae* | BexAB | Capsule polysaccharide |
| *E. coli* | KpsMT | Capsule polysaccharide |
| *Niesseria* | CrtCD | Capsule polysaccharide |
| *E. coli* | FtsE | Cell division |
| *E. coli* | UvrA | DNA repair |
| *Rhizobium leguminosarum* | NodI | Nodulation |
| *Rhizobium meliloti* | OFR1 | ? |

| Cyanobacteria | | |
|---|---|---|
| *Anabaena* | HetA | Differentiation |
| *Synchococcus* | CysA | Sulphate |

| Yeast | | |
|---|---|---|
| *S. cerevisiae* | STE6 | a-mating peptide |
| S. cerevisiae | ADP1 | ? |
| *S*. *cerevisiae* | EF-3 | Translation |

| Protozoa | | |
|---|---|---|
| *Plasmodium* | pfMDR | Chloroquine |
| *Lieshmania* | ltpgpA | Methotrexate/heavy metals |

| Insect | | |
|---|---|---|
| *Drosophila* | *white-brown* | Eye pigments |
| *Drosophila* | Mdr49 | Hydrophobic drugs? |
| | Mdr65 | ? |

| Plants | | |
|---|---|---|
| Liverwort chloroplast | MbpX | ? |

| Animals | | |
|---|---|---|
| Man | CFTR | Chloride |
| Mouse | CFTR | Chloride |
| *Xenopus* | CFTR | Chloride |
| Cow | CFTR | Chloride |
| Dogfish | CFTR | Chloride |
| Man | MDR1 | Hydrophobic drugs |
| | MDR3 | ? |
| Mouse | MDR1 | Hydrophobic drugs |
| | MDR2 | ? |
| | MDR3 | Hydrophobic drugs |
| | MDR3 | Hydrophobic drugs |
| Hamster | Pgp1 | Hydrophobic drugs |
| | Pgp2 | Hydrophobic drugs |
| | Pgp3 | ? |
| Man | PMP70 | Polypeptides? |
| Man | RING4-11 | Peptides |
| | PSF1-PSF2 | Peptides |
| Mouse | HAM1-HAM2 | Peptides |
| Rat | Mtp1 | Peptides |

| | | |
|---|---|---|
| * Adapted from Higgins, C.F. 1992. ABC Transporters: From Microorganizmz to Man. Annu. Rev. Cell Biol. 8, 67-113. | | |

**TABLE 2:**

| **HUMAN G PROTEIN-COUPLED SEVEN TRANSMEMBRANE RECEPTORS: REFERENCES FOR CLONING** | |
|---|---|
| Receptor | Reference |
| α_{1A}-adrenergic receptor | Bruno et al. (1991) |
| α_{1B}-adrenergic receptor | Ramarao et al. (1992) |
| α₂-adrenergic receptor | Lomasney et al. (1990) |
| α_{2B}-adrenergic receptor | Weinshank et al. (1990) |
| β₁-adrenergic receptor | Frielle et al. (1987) |
| β₂-adrenergic receptor | Kobilka et al. (1987) |
| β₃-adrenergic receptor | Regan et al. (1988) |
| m₁AChR, m2 AChR, m₃ AChR, m₄ AChR | Bonner et al. (1987) Peralta et al. (1987) |
| m5 AChR | Bonner et al. (1988) |
| D₁ dopamine | Dearry et al. (1990) Zhou et al. (1990) Sunahara et al. (1990) Weinshank et al. (1991) |
| D₂ dopamine | Grandy et al. (1989) |
| D₃ dopamine | Sokoloff et al. (1990) |
| D₄ dopamine | Van Tol et al. (1991) |
| D₅ dopamine | M. Caron (unpub.) Weinshank et al. (1991) |
| A1 adenosine | Libert et al. (1992) |
| adenosine A2b | Pierce et al. (1992) |
| 5-HT1a | Kobilka et al. (1987) Fargin et al. (1988) |
| 5-HT1b | Hamblin et al. (1992) Mochizuki et al. (1992) |
| 5HT1-like | Levy et al. (1992a) |
| 5-HT1d | Levy et al. (1992b) |
| 5HT1d-like | Hamblin and Metcalf (1991) |
| 5HT1d beta | Demchyshyn et al. (1992) |
| substance K (neurokinin A) | Gerard et al. (1990) |
| substance P (NK1) | Gerard, et al. (1991); Takeda et al. (1991) |
| f-Met-Leu-Phe | Boulay et al. (1990) Murphy & McDermott (1991) DeNardin et al. (1992) |
| angiotensin II type 1 | Furuta et al. (1992) |
| *mas* proto-oncogene | Young et al. (1986) |
| endothelin ETA | Hayzer et al. (1992) Hosoda et al. (1991) |
| endothelin ETB | Nakamuta et al. (1991) Ogawa et al. (1991) |
| thrombin | Vu et al. (1991) |
| growth hormone-releasing hormone (GHRH) | Mayo (1992) |
| vasoactive intestinal peptide (VIP) | Sreedharan et al. (1991) |
| oxytocin | Kimura et al., (1992) |
| somatostatin SSTR1 and SSTR2 | Yamada et al. (1992a) |
| SSTR3 | Yamada et al. (1992b) |
| cannabinoid | Gerard et al. (1991) |
| follicle stimulating hormone (FSH) | Minegish et al. (1991) |
| LH/CG | Minegish et al. (1990) |
| thyroid stimulating hormone (TSH) | Nagayama et al. (1989) Libert et al. (1989) Misrahi et al. (1990) |
| thromboxane A2 | Hirata et al. (1991) |
| platelet-activating factor (PAF) | Kunz et al. (1992) |
| C5a anaphylatoxin | Boulay et al. (1991) Gerard and Gerard (1991) |
| Interleukin 8 (IL-8) IL-8RA | Holmes et al. (1991) |
| IL-8RB | Murphy and Tiffany (1991) |
| Delta Opioid | Evans et al. (1992) |
| Kappa Opioid | Xie et al. (1992) |
| mip-1/RANTES | Neote et al. (1993) Murphy et al., in press |
| Rhodopsin | Nathans and Hogness (1984) |
| Red opsin, Green opsin, Blue opsin | Nathans, et al. (1986) |
| metabotropic glutamate mGluR1-6 | Tanabe et al. (1992) |
| histamine H2 | Gantz et al. (1991) |
| ATP | Julius, David (unpub.) |
| neuropeptide Y | Herzog et al. (1992) Larhammar et al. (1992) |
| amyloid protein precursor | Kang et al. (1987) Mita, et al. (1988) Lemaire et al. (1989) |
| insulin-like growth factor II | Kiess et al. (1988) |
| bradykinin | Hess et al. (1992) |
| gonadotropin-releasing hormone | Chi et al. (1993) |
| cholecystokinin | Pisegna et al. (1992) |
| melanocyte stimulating hormone receptor | Chhajlane et al. (1992) Mountjoy et al. (1992) |
| antidiuretic hormone receptor | Birnbaumer et al. (1992) |
| glucagon receptor | Sprecher et al. (1993) |
| adrenocorticotropic hormone II | Mountjoy et al. (1992) |

### References to Table 2:

Bonner, T.I., Buckley, N.J., Young, A. C., and Brann, M. R. (1987). Identification of a family of muscarinic acetylcholine receptor genes. Science 237, 527-532.

Bonner, T. I., Young, A. C., Brann, M. R., and Buckley, N. J. (1988). Cloning and expression of the human and rat m5 muscarinic acetylcholine receptor genes. Neuron 1, 403-410.

Boulay, F., Mery, L., Tardif, M., Brouchon, L., and Vignais, P. (1991) Expression cloning of a receptor for C5a anaphylatoxin on differentiated HL-60 cells. Biochemistry 30, 2993-2999.

Boulay, F., Tardif, M., Brouchon, L., Vignais, P. (1990) The human N-formylpeptide receptor. Characterization of two cDNA isolates and evidence for a new subfamily of G protein-coupled receptors. Biochemistry 29, 11123-11133.

Bruno, J. F., Whittaker, J., Song, J. F., Berelowitz, M. (1991) Molecular cloning and sequencing of a cDNA encoding a human 1A adrenergic receptor. Biochem. Biophys. Res. Commun. 179, 1485-1490.

De-Nardin-E. Radel-S-J. Lewis-N. Genco-R-J. Hammarskjold-M. (1992) Identification of a gene encoding for the human formyl peptide receptor. Biochem-Int. 26, 381-387.

Dearry, A., Gingrich, J. A., Falardeau, P., Fremeau, R. T., Bates. M. D., Caron, M. G. (1990) Molecular cloning and expression of the gene for a human D₁ dopamine receptor. Nature 347, 72-76.

Demchyshyn-L. Sunahara-R-K. Miller-K. Teitler-M. Hoffman-B-J. Kennedy-J-L. Seeman-P. Van-Tol-H-H. Niznik-H-B. A human serotonin 1D receptor variant (5HT1D beta) encoded by an intronless gene on chromosome 6. (1992) Proc-Natl-Acad-Sci-U-S-A. 89, 5522-5526.

Evans, C. J., Keith, D. E. Jr., Morrison, H., Magendzo, K., and Edwards, R. H. (1992) Cloning of a delta opioid receptor by functional expression. Science 258, 1952-1955.

Fargin, A., Raymond, J. R., Lohse, M. J., Kobilka, B. K., Caron, M. G., and Lefkowitz, R. J. (1988). The genomic clone G-21 which resembles a β-adrenergic receptor sequence encodes the 5-HT₁ₐ receptor. Nature 335, 358-360.

Frielle, T., Collins, S., Daniel, K. W., Caron, M. G., Lefkowitz, R. J., and Kobilka, B. K. (1987) Cloning of the cDNA for the human 1-adrenergic receptor. Proc. Natl. Acad. Sci. U. S. A. 84, 7920-7924.

Furuta-H. Guo-D-F. Inagami-T. (1992) Molecular cloning and sequencing of the gene encoding human angiotensin II type 1 receptor. Biochem-Biophys-Res-Commun. 183, 8-13.

Gantz, I., Munzert, G., Tashiro, T., Schaffer, M., Wang, L., DelValle, J., Yamada, T. (1991b) Molecular cloning of the human histamine H2 receptor. Biochem. Biophys. Res. Commun. 178, 1386-1392.

Gerard, N. P., Eddy, R. L. Jr., Shows, T. B., and Gerard, C. (1990) The human neurokinin A (Substance K) receptor. Molecular cloning of the gene, chromosomal localization, and isolation of cDNA from tracheal and gastric tissues. J. Biol. Chem. 265, 20455-20462.

Gerard, N. P. and Gerard, C. (1991) The chemotactic receptor for C5a anaphylatoxin. Nature 349, 614-617.

Gerard, N. P., Garraway, L. A., Eddy, R. L. Jr., Shows, T. B., Iijima H., Paquet, J.-L.and Gerard, C. (1990) Human substance P receptor (NK-1) : Organization of the gene, chromosome localization, functional expression of cDNA clones. Biochem. 30, 10640-10646.

Gerard, C. M., Mollereau, C., Vassart, G., and Parmentier, M. (1991) Molecular cloning of a human cannabinoid receptor which is also expressed in testis. Biochem. J. 279,129-134.

Grandy, D. K., Marchionni, M. A., Makam, H., Stofko, R. E., Alfanzo, M., Frothingham, L., Fischer, J. B., Burke-Howie, K. J., Bunzow, J. R., Server, A. C. and Civelli, O. (1989) Cloning of the cDNA and gene for a human D₂ dopamine receptor. Proc. Natl. Acad. Sci. U. S. A. 86, 9762-9766.

Hamblin, M. W., and Metcalf, M. A. (1991) Primary structure and functional characterization of a human 5-HT1D-type serotonin receptor. Mol. Pharmacol. 40, 143-148.

Hamblin-M-W. Metcalf-M-A. McGuffin-R-W. Karpells-S. (1992) Molecular cloning and functional characterization of a human 5-HT1B serotonin receptor: a homologue of the rat 5-HT1B receptor with 5-HT1D-like pharmacological specificity. Biochem-Biophys-Res-Commun. 184, 752-759.

Hayzer-D-J. Rose-P-M. Lynch-J-S. Webb-M-L. Kienzle-B-K. Liu-E-C. Bogosian-E-A. Brinson-E. Runge-M-S. (1992) Cloning and expression of a human endothelin receptor: subtype A. Am-J-Med-Sci. Oct. 304, 231-238.

Herzog, H., Hort, Y. J., Ball, H. J., Hayes, G., Shine, J., and Selbie, L. A. (1992) Cloned human neuropeptide Y receptor couples to two different second messenger systems. Proc. Natl. Acad. Sci. U. S. A. 89, 5794-5798.

Hirata, M., Hayashi, Y., Ushikubi, F., Yokota, Y., Kageyama, R., Nakanishi, S and Narumiya, S. (1991) Cloning and expression of cDNA for a human thromboxane A₂ receptor. Nature 349, 617-620.

Holmes, W. E., Lee, J., Kuang, W.-J., Rice, G. C., and Wood, W. I. (1991) Structure and functional expression of a human interleukin-8 receptor. Science 253, 1278-1280

Hosoda-K. Nakao-K. Hiroshi-Arai. Suga-S. Ogawa-Y. Mukoyama-M. Shirakami-G. Saito-Y. Nakanishi-S. Imura-H. (1991) Cloning and expression of human endothelin-1 receptor cDNA. FEBS-Lett. 287, 23-26.

Kang, J. Lemaire, H.-G., Unterbeck, A., Salbaum, J. M., Masters,, C. L., Grzeschik, K.-H., Multhaaup, G., Beyreuther, K., Muller-Hill, B. (1987) The precursor of Alzheimer's disease amyloid protein resembles a cell-surface receptor. Nature 325, 733-736.

Kiess, W., Blickenstaff, G. D., Sklar, M. M., Thomas, C. L., Nissley, S. P. and Sahagian G. G. (1988) Biochemical evidence that the type II insulin-like growth factor receptor is identical to the cation-independent mannose 6-phosphate receptor. J. Biol. Chem. 263, 9339-9344.

Kimura, T., Tanizawa, O., Mori, K., Brownstein, M. J., and Okayama, H. (1992) Structure and expression of a human oxytocin receptor. Nature 356, 526-529.

Kobilka, B. K., Dixon, R. A. F., Frielle, T., Dohlman, H. G., Bolanowski, M. A., Sigal, I. S., Yang-Feng, T. L., Francke, U., Caron, M.G., and Lefkowitz, R. J. (1987) cDNA for the human β₂-adrenergic receptor: A protein with multiple membrane spanning domains and a chromosomal location shared with the PDGF receptor gene. Proc. Natl. Acad. Sci. U.S.A. 84, 46-50.

Kobilka, B. K., Frielle, T., Collins, S., Yang-Feng, T., Kobilka, T. S., Francke, U., Lefkowitz, R. J. and Caron, M. G. (1987). An intronless gene encoding a potential member of the family of receptors coupled to guanine nucleotide regulatory proteins. Nature 329, 75-79

Kunz, D., Gerard, N. P., and Gerard, C. (1992) The human leukocyte platelet-activating factor receptor. cDNA cloning, cell surface expression and construction of a novel epitope-bearing analog. J. Biol. Chem. 267, 9101-9106.

Larhammar, D., Blomqvist, A. G., Yee, F., Jazin, E., Yoo, H., Wahlested, C. (1992) Cloning and functional characterization of a human neuropeptide Y/peptide YY receptor of the Y1 type. J. Biol. Chem. 267, 10935-10938.

Lemaire, H. G., Salbaum, J. M., Multhaup, Kang, J., Bayney, R. M., Unterbeck, A., Beyreuther, K., Muller-Hill, B. (1989) The PreA4₆₉₅ precursor protein of Alzheimer's disease A4 amyloid is encoded by 16 exons. Nuc. Acids. Res. 17, 517-522.

Levy, F. O., Gudermann, T., Perez-Reyes, E., Birnbaumer, M., Kaumann, A. J., and Birnbaumer, L. (1992b) Molecular cloning of a human serotonin receptor (S12) with a pharmacological profile resembling that of the 5-HT1d subtype. J. Biol. Chem. 267, 7553-7562.

Levy, F. O., Gudermann, T., Birnbaumer, M., Kaumann, A. J., and Birnbaumer, L. (1992a) Molecular cloning of a human gene (S31) encoding a novel serotonin receptor mediating inhibition of adenylyl cyclase. FEBS. Lett., 296, 201-206.

Libert, F., Lefort, A., Gerard, C., Parmentier, M., Perret, J., Ludgate, M., Dumont, J.,Vassart, G. (1989) Cloning, sequence and expression of the human thyrotropin (TSH) receptor: Evidence for the binding of autoantibodies. Biochem. Biophys. Res. Commun. 165, 150-155.

Libert, F., Van Sande, J., Lefort, A., Czernilofsky, A., Dumont, J. E., Vassart, G., Ensinger, H. A., and Mendla, K. D. (1992) Cloning and functional characterization of a human A1 adenosine receptor. Biochem. Biophys. Res. Commun. 187, 919-926.

Lomasney, J. W., Lorenz, W., Allen, L. F., King, K., Regan, J. W., Yang-Feng, T. L., Caron, M. G., and Lefkowitz, R. J. (1990) Expansion of the α₂-adrenergic receptor family: cloning and characterization of a human α₂-adrenergic receptor subtype, the gene for which is located on chromosome 2. Proc. Natl. Acad. Sci. U. S. A. 87, 5094-5098.

Mayo, K. E. (1992) Molecular cloning and expression of a pituitary-specific receptor for growth hormone-releasing hormone. Mol. Endocrin. 6, 1734-1744.

Minegish, T., Nakamura, K., Takakura, Y., Ibuki, Y., and Igarashi, M. (1991) Cloning and sequencing of human FSH receptor cDNA. Biochem. Biophys. Res. Commun. 175, 1125-1130.

Minegish, T., Nakamura, K., Takakura, Y., Miyamoto, K., Hasegawa, Y., Ibuki, Y., and Igarashi, M. (1990) Cloning and sequencing of human LH/hCG receptor cDNA. Biochem. Biophys. Res. Commun. 172, 1049-1054.

Misrahi, M., Loosfelt, H., Atger, M., Sar, S., Guiochen-Mantel, A., Milgrom, E. (1990) Cloning sequencing and expression of human TSH receptor. Biochem. Biophys. Res. Commun. 166, 394-403. Mita, S., Sadlock, J., Herbert, J., Schon, E. A. (1988) A cDNA specifying the human amyloid precursor protein (ABPP) encodes a 95-kDa polypeptide. Nuc. Acids Res. 16, 9351.

Mochizuki-D. Yuyama-Y. Tsujita-R. Komaki-H. Sagai-H. (1992) Cloning and expression of the human 5-HT1B-type receptor gene. Biochem-Biophys-Res-Commun. 185, 517-523.

Murphy, P. M. and McDermott, D. (1991) Functional expression of the human formyl peptide receptor in *Xenopus* oocytes requires a complementary human factor. J. Biol. Chem. 266, 12560-12567.

Murphy, P. M. and Tiffany, H. L. (1991) Cloning of complementary DNA encoding a functional human interleukin-8 receptor. Science 253, 1280-1283.

Nagayama, Y., Kaufman, K. D., Seto, P., and Rapoport, B. (1989) Molecular cloning, sequence and functional expression of the cDNA for the human thyrotropin receptor. Biochem. Biophys. Res. Commun. 165, 1184-1190.

Nakamuta-M. Takayanagi-R. Sakai-Y. Sakamoto-S. Hagiwara-H. Mizuno. Saito-Y. Hirose-S. Yamamoto-M. Nawata-H. (1991) Cloning and sequence analysis of a cDNA encoding human non-selective type of endothelin receptor. Biochem-Biophys-Res-Commun. 177, 34-39.

Nathans, J., and Hogness, D. S. (1984) Isolation and nucleotide sequence of the gene encoding human rhodopsin. Proc. Natl. Acad. Sci. U.S.A 81, 4851-4855.

Nathans, J., Thomas, D., and Hogness, D. S. (1986) Molecular genetics of human color vision: The genes encoding blue, green, and red pigments. Science 232, 193-202.

Neote, K. DiGregorio, D., Mak, J. Y., Horuk, R., and Schall, T. J. (1993) Molecular cloning, functional expression, and signaling characteristics of a C-C chemokine receptor. Cell 72, 415-425.

Ogawa-Y. Nakao-K. Arai-H. Nakagawa-O. Hosoda-K. Suga-S. Nakanishi-S. Imura-H. (1991) Molecular cloning of a non-isopeptide-selective human endothelin receptor. Biochem-Biophys-Res-Commun. 178, 248-255.

Peralta, E.G., Ashkenazi, A., Winslow, J. W., Smith, D. H., Ramachandran, J., and Capon, D. J. (1987b). Distinct primary structures, ligand-binding properties, and tissue-specific expression of four human muscarinic acetylcholine receptors. EMBO J. 6, 3923-3929.

Pierce, K. D., Furlong, T. J., Selbie, L. A., and Shine, J. (1992) Molecular cloning and expression of an adenosine A2b receptor from human brain. Biochem. Biophys. Res. Commun. 187, 86-93.

Ramarao-C-S. Denker-J-M. Perez-D-M. Gaivin-R-J. Riek-R-P. Graham-R-M. (1992) Genomic organization and expression of the human α 1B-adrenergic receptor. J-Biol-Chem. 267, 21936-21945.

Regan, J. W., Kobilka, T. S., Yang-Feng, T. L., Caron, M. G., and Lefkowitz, R. J. (1988) Cloning and expression of a human kidney cDNA for a novel β₂-adrenergic receptor. Proc. Natl. Acad. Sci. U.S.A. 85, 6301-6305.

Sokoloff, P., Giros, B., Martres, M.-P., Bouthenet, M. L., Schwartz, J.-C. (1990) Molecular cloning and characterization of a novel dopamine receptor (D₃) as a target for neuroleptics. Nature 347, 146-151.

Sreedharan, S. P., Robichon, A., Peterson, K. E., and Goetzl, E. J. (1991) Cloning and expression of the human vasoactive intestinal peptide receptor. Proc. Natl. Acad. Sci. U.S.A. 88, 4986-4990.

Sunahara, R. K., Niznik, H. B., Weiner, D. M., Stormann, T. M., Brann, M. R., Kennedy, J. L., Gelernter, J. E., Rozmahel, R., Yang, Y., Israel, Y., Seeman, P., O'Dowd, B. F. (1990) Human dopamine D₁ receptor encoded by an intronless gene on chromosome 5. Nature 347, 80-83.

Takeda, Y., Chou, K. B., Takeda, J., Sachais, B. S., and Krause, J. E. (1991) Molecular cloning, structural characterization and functional expression of the human substance P receptor. Biochem. Biophys. Res. Commun. 179, 1232-1240.

Tanabe, Y., Hasu, H., Shigemoto, R., Nakanishi, S. (1992) A family of metabotropic glutamate receptors. Neuron 8, 169-179. Van Tol, H. H., Bunzow, J. R., Guan, H. C., Sunahara, R. K., Seeman, P., Niznik, H. B., Civelli, O. (1991) Cloning of the gene for a human dopamine D4 receptor with high affinity for the antipsychotic clozapine. Nature 350, 610-614.

Vu, T.-K. H., Hung, D. T., Wheaton, V. I., and Coughlin, S. R. (1991) Molecular cloning of a functional thrombin receptor reveals a novel proteolytic mechanism of receptor activation. Cell 64, 1057-1068.

Weinshank, R. L., Zgombick, J. M., Macchi, M., Adham, N., Lichtblau, H., Branchek, T. A., and Hartig, P. R. (1990) Cloning, expression and pharmacological characterization of a human α_{2B}-adrenergic receptor. Mol. Pharmacol. 38, 681-688.

Weinshank-R-L. Adham-N. Macchi-M. Olsen-M-A. Branchek-T-A. Hartig-P-R. (1991) Molecular cloning and characterization of a high affinity dopamine receptor (D1 beta) and its pseudogene. J-Biol-Chem. 266, 22427-22435.

Xie, G.-X., Miyajima, A., and Goldstein, A. (1992) Expression cloning of cDNA encoding a seven-helix receptor from human placenta with affinity for opioid ligands. Proc. Natl. Acad. Sci. U. S. A. 89, 4124-4128.

Yamada, Y., Post, S. R., Wang, K., Tager, H. S., Bell, G. I., Seino, S., (1992a) Cloning and functional characterization of a family of human and mouse somatostatin receptors expressed in brain, gastrointestinal tract and kidney. Proc. Natl. Acad. Sci. U.S.A. 89, 251-255.

Yamada, Y., Reisine, T., Law, S. F., Ihara, Y., Kubota, A., Kagimoto, S., Seino, M., Seino, Y., Bell, G. I., Seino, S., (1992b) Somatostatin receptors, an expanding gene family: Cloning and functional characterization of human SSTR3, a protein coupled to adenylyl cyclase. Mol. Endocrin. 6, 2136-2142.

Young, D., Waitches, G., Birchmeier, C., Fasano, O., and Wigler, M. (1986) Isolation and characterization of a new cellular oncogene encoding a protein with multiple transmembrane domains. Cell 45, 711-719.

Zhou, Q.-Y., Grandy, D. K., Thambi, L., Kushner, J. A., Van Tol, H. H. M., Cone, R., Pribnow, D., Salon, J., Bunzow, J. R., Civelli, O. (1990) Cloning and expression of human and rat D₁ dopamine receptors. Nature 347, 76-80.

Birnbaumer, M., Seibold, A., Gilbert, S., Ishido, M., Barberis, C., Antaramian, A., Brabet, P., Rosenthal, W., (1992) Molecular cloning of the receptor for human antidiuretic hormone. Nature 357,333-5.

Chhajlan, V., Wikberg, J.-E., (1992) Molecular cloning and expression of melanocycte stimulating hormone receptor cDNA. FEBS Lett. 309, 417-420.

Chi, L., Zhou, W., Prikhosan, A., Flanagan, C., Davidson, J.S., Golembo, M., Illing, N., Millar, R.P., Sealfon, S.C. (1993) Cloning and characterization of human gonadotropin-releasing hormone receptor. Mol. Cell Endrocrinol, 91,R1-R6.

Hess, J.-F., Borkowski, J.-A., Young, G.-S., Strader, C.-D., Ransom, R.-W., (1992) Cloning and pharmacological characterization of a human bradykinin (BK-2) receptor. Biochem-Biophys-Res-Commun. 184, 260-8.

Mountjoy, K.G., Robbins, L.S., Mortrud, M.T., Cone R.D., (1992) The cloning of a family of genes that encode the melanocortin receptors. Science 257, 1248-1251.

Pisegna, J.R., de-Weerth, A., Huppi, K., Wank, S.A., (1992) Molecular cloning of the human brain and gastric cholecystokinin receptor: structure, functional expression and chromosomal localization. Biochem-Biophys-Res-Commun. 189,296-303.

Zhou, Q.Y., Grandy, D.K., Thambi, L., Kushner, J.A., Van Tol, H.H.M., Cone, R., Pribnow, D., Salon, J., Bunzow, J.R., Civelli, O., (1990) Cloning and expression of human and rat D₁ dopamine receptors. Nature 347,76-80.

### General References

Anderson M.P., Gregory R.J., Thompson S., Souza D.W., Paul,S. et al. (1991) Demonstration that CFTR is a chloride channel by alteration of its anion selectivity. Science 253, 202-205.

Barr P.J., Mason O.B., Landsberg K.E., Wong P.A. et al. (1991) cDNA and gene structure for a human subtilisin-like protease with cleavage specificity for paired basic amino acid residues. DNA Cell Biol. 10, 319-328.

Benjannet, S., Rondeau N., Day R., Chretien M., Seidah N.G. (1991) PC1 and PC2 are proprotein convertases capable of cleaving proopiomelanacortin at distinct pairs of basic residues. Proc. Natl. Acad. Sci. USA 88:3564-3568.

Bianchi A.B., Fischer S.M., Robles A.I., Rinchik E.M., Conti C.J. (1993) Overexpression of cyclin D1 in mouse skin carcinogenesis. Oncogene 8, in press.

Birkenbach M., Josefsen K., Yalamanchili R., Lenoir G., Kieff E. (1993) Epstein-Barr Virus-induced genes: First lymphocyte-specific G protein-coupled peptide receptors J. Virol. 67, 2209-2220.

Buratowski S., Hahn S., Sharp P.A., Guarente L. (1988) Function of a yeast TATA element-binding protein in a mammalian transcription system. Nature 334, 37.

Burkholder A.C. and Hartwell L.H. (1985) The yeast α-factor receptor: Structural properties deduced from the sequence of the STE2 gene. Nuc. Acids Res. 13, 8463.

Cavallini B., Huet J., Plassat, J.-L., Sentenac A., Egly J.-M., Chambon P. (1988) A yeast activity can substitute for the HeLa cell TATA box factor. Nature 334, 77.

Chen WJ, Andres D.A., Goldstein J.L., Brown M.S. (1991) Cloning and expression of a cDNA encoding the subunit of rat p21^{ras} protein farnesyltransferase. Cell 66:327-334.

Choi K., Chen C.-J., Kriegler M., Roninson I.B. (1988) An altered pattern of cross-resistance in multidrug resistant human cells results from spontaneous mutation in the *mdr1* (P-glycoprotein) gene. Cell 53, 519-529.

Crews C.M., Allessandrini A., Erikson R.L. (1992) The primary structure of MEK, a protein kinase that phophorylates the ERK gene product. Science 258, 478-480.

Cross F. (1988) DAF1, a mutant gene affecting size control, pheromone arrest, and cell cycle kinetics of Saccharomyces cerevisiae. Mol. Cell. Biol. 8, 4675-4684.

Dassa E. (1990) Cellular localization of the *MALg* protein from the maltose transport system in *Escherichia coli* K12. Mol. Gen. Genet. 222:33-36.

Dassa E. and Hofnung M. (1985) Sequence of gene *malG* in E. *coli* K12 : homologies between integral membrane components from binding protein-dependent transport systems. EMBO J. 4:2287-2293.

Dmochowska A., Dignard D., Henning D., Thomas D.Y., Bussey H. (1987) Yeast KEX1 gene encodes a putative protease with a carboxypeptidase B-like function involved in killer toxin and α-factor precursor processing. Cell 50, 573.

Ehrmann M., Boyd D., Beckwith J. (1990) Genetic analysis of membrane protein topology by a sandwich gene fusion approach. Proc. Natl. Acad. Sci. 87, 7574-7578.

Elledge S.J. and Spottswood M.R. (1991) A new human p34 protein kinase, CDK2, identified by complementation of a *cdc28* mutation in *Saccharomyces cerevisiae,* is a homolog of *Xenopus* Eg1. EMBO J. 10, 2653-2659.

Emter O., Mechler B., Achstetter T., Muller H., Wolf D.H. (1983) Yeast pheromone α-factor is synthesized as a high molecular weight precursor. Biochem. Biophys. Res. Commun. 116, 822-829.

Endicott J.A., Sarangi F., Ling V. (1991) Complete cDNA sequences encoding the Chinese hamster P-glycoprotein gene family. DNA Sequence 2, 89-101.

Engleman D.A., Steitz T.A., Goldman A. (1986) Identifying nonpolar transbilayer helices in amino acid sequences of membrane proteins. Ann. Rev. Biophys. Chem. 15, 321-353.

Etienne G., Armau E., Tiraby G. (1990) A screening method for antifungal substances using Saccharomyces cerevisiae strains resistant to polyene macrolides. J. Antibiot. 43, 199-206.

Fikes J.D., Becker D.M., Winston F., Guarente L. (1990) Striking conservation of TFIID in *Schizosaccaharomyces pombe* and *Saccharomyces cerevisiae.* Nature 346, 291.

Gasch A.A., Hoffman M., Horikoshi M., Roeder R., Chua N. (1990) *Arabodopsis thaliana* contains two genes for TFIID. Nature 346, 390.

Glotzer M., Murray A.W., Kirschner M.W. (1991) Cyclin is degraded by the ubiquitin pathway. Nature 349, 132-138.

Gomez R., Goodman LE; Tripathy SK; O'Rourke E; et al. Purified yeast protein farnesyltransferase is structurally and functionally similar to its mammalian counterpart. (1993) Biochem. J. 289, 25-31.

Gotoh Y., Nishida E.,Shimanuki M., Toda T., Imai Y., Yamamoto M. (1993) *Schizosaccharomyces pombe* SPK1 is a tyrosine-phosphorylated protein functionally related to *Xenopus* mitogen-activated protein kinase. Mol. Cell. Biol. 13, 6427-6434.

Gros P., Dhir R., Croop J., Talbot F. (1991) A single amino acid substitution strongly modulates the activity and substrate specificity of the mouse mdr1 and mdr3 drug efflux pumps. Proc. Natl. Acad. Sci. 88, 7289-7293.

Guarente L. (1988) UASs and enhancers: Common mechanism of transcriptional activation in yeast and mammals. Cell 52, 303.

Guarente L. in The molecular and cellular biology of the yeast *Saccharomyces:* Gene Expression, Jones E.W., Pringle J.R., Broach J.R., eds., Cold Spring Harbor Laboratory Press, New York, 1992, p49-98.

Hadwiger J.A., Wittenberg C., Richardson H.E., de Barros Lopes M., Reed S.I. (1989) A family of cyclin homologs that control the G1 phase in yeast. Proc. Natl. Acad. Sci. 86, 6255-6259.

Hagen D.C., McCaffrey G., Sprague G.F. (1986) Evidence the yeast STE3 gene encodes a receptor for the peptide pheromone **a**-factor: gene sequence and implications for the structure of the presumed receptor. Proc. Natl. Acad. Sci. 83, 1418.

Hara M., Akasaka K., Akinaga S., Okabe M., Nakano H., Gomez R., Wood D., Uh M., Tamanoi F. (1993) Identification of Ras farnesyltransferase inhibitors by microbial screening. Proc. Natl. Acad. Sci. 90, 2281-2285.

Harshman K.D., Moye-Rowley W.S., Parker C.S. (1988) Transcriptional activation by the SV40 AP-1 recognition element in yeast is mediated by a factor similar to AP-1 that is distinct from GCN4. Cell 53, 321.

He B., Chen P., Chen S.-Y., Vancura K.L., Michaelis S., Powers S. (1991) *RAM2,* an essential gene of yeast, and *RAM1* encode the two polypeptide components of the farnesyltransferase that prenylates **a**-factor and Ras proteins. Proc Natl Acad Sci 88:11373-11377.

Higgins C.F., Haag P.D., Nikaido K., Aedeshir F., Garcia G. et al. (1982) Complete nucleotide sequence and identification of membrane components of the histidine transport operon of *S. typhimurium.* Nature 298, 723-727.

Hoey T., Dynlacht B.D., Peterson M.G., Pugh B.F., Tjian R. (1990) Isolation and characterization of the *Drosophila* gene encoding the TATA box binding protein, TFIID. Cell 61, 1179.

Hoffman A., Sinn E., Yamamoto T., Wang J., Roy A., Horikoshi M., Roeder R.G. (1990) Highly conserved core domain and unique N terminus with presumptive regulatory motifs in a human TATA factor (TFIID). Nature 346, 387.

Howe OP.H., Draetta G., Leof E.B. (1991) Transforming growth factor 1 inhibition of p34cdc2 phosphorylation and histone H1 kinase activity is associated with G1/S-phase growth arrest. Mol. Cell. Biol. 11, 1185-1194.

Hrycyna C.A., Sapperstein S.K., Clarke S., Michaelis S. (1991) The *Saccharomyces cerevisiae STE14* gene encodes a methyltransferase that mediates C-terminal methylation of **a**-factor and RAS proteins. EMBO J. 10, 1699.

Hughes D.A., Ashworth A., Marshall C.J. (1993) Complementation of *byr1* in fission yeast by mammalian MAP kinase kinase requires coexpression of Raf kinase. Nature 364, 349-352.

Hyde S.C., Emsley P., Hartshorn M., Mimmack M.M., Gileadi U. et al. (1990) Structural model of ATP-binding proteins associated with cystic fibrosis, multidrug resistance and bacterial transport. Nature 346, 362-365.

Jabbar, M. A., Sivasubramanian, N., Nayak, D. P. (1985) Influenza viral (A/WSN/33) hemagglutinin is expressed and glycosylated in the yeast *Saccharomyces cerevisiae.* Proc. Natl. Acad. Med. U.S.A. 82, 2019-2023.

Julius D., Brake A., Blair L., Kunisawa R., Thorner J. (1984) Isolation of the putative structural gene for the lysine-arginine-cleaving endopeptidase required for processing of yeast prepro-α-factor. Cell 37, 1075-1089.

Julius D., Schekman, Thorner J. (1984) Glycosylation and processing of prepro-α-factor through the yeast secretory pathway. Cell 36, 309-318.

Julius D., Blair L., Brake A., Sprague G., Thorner J. (1983. Yeast α-factor is processed from a larger precursor polypeptide: the essential role of a membrane-bound dipeptidyl aminopeptidase. Cell 32, 839.

Kakidani H. and Ptashne M. (1988) GAL4 activates gene expression in mammalian cells. Cell 52, 161.

Kao C.C., Lieberman P.M., Schmidt M.C., Zhou Q., Pei R., Berk A.J. (1990) Cloning of a transcriptionally active human TATA binding factor. Science 248, 1646.

Kay B.K., Adey N.B., He Y.-S., Manfredi J.P., Mataragnon A.H., Fowlkes D.F. (1993) An M13 phage library displaying random 38-amino-acid peptides as a source of novel sequences with affinity to selected targets. Gene 128, 59-65.

Keyomarsi K. and Pardee A.B. (1993) Redundant cyclin overexpression and gene amplification in breast cancer cells. Proc. Natl. Acad. Sci. 90, 1112-1116.

Khavari P.A., Peterson C.L., Tamkun J.W., Mendel D.B., Crabtree G.R. (1993) BRG1 contains a conserved domain of the SWI2/SNF2 family necessary for normal mitotic growth and transcription. Nature 366, 170-174.

Kingsman S.M., Kingsman A.J., Mellor J. (1987) The production of mammalian proteins in *Saccharomyces cerevisiae.* TIBTECH 5, 53-57.

Koff A., Cross F., Fisher A., Schumacher J., Leguellec K., Phillipe M., Roberts J.M. (1991) Human cyclin E, a new cyclin that interacts with two members of the CDC2 gene family. Cell 66, 1217-1228.

Koff A., Ohtsuli M., Polyak K., Roberts J.M. Massagué J. (1993) Negative regulation of G1 in mammalian cells: inhibition of cyclin E-dependent kinase by TGF-β. Science 260, 536-539.

Kohl N.E., Mosser S., deSolms S.J., Giuliani E.A., Pompliano D.L., Graham S.L., Smith R.L., Scolnick E.M., Oliff A., Gibbs J.B. (1993) Selective inhibition of *ras*-dependent transformation by a farnesyltransferase inhibitor. Science 260, 1934-1937.

Kohl NE, Diehl R.E., Schaber M.D., Rands E., Soderman D.D., He B., Moores S.L., Pompliano D.L., Ferro-Novick S., Powers S. et al. (1991) Structural homology among mammalian and Saccharomyces cerevisiae isoprenyl-protein transferases. J. Biol. Chem. 266, 18884-18888.

Korner, J., Chun J., Harter D., Axel R. (1991) Isolation and functional expression of a mammalian prohormone processing enzyme, murine prohormone convertase 1. Proc. Natl. Acad. Sci USA 88:6834-6838.

Kouba M; Vanetti M; Wang X; Schafer M; Hollt V (1993) Cloning of a novel putative G-protein-coupled receptor (NLR) which is expressed in neuronal and lymphatic tissue. FEBS Lett 321, 173-178.

Kramer R.A., Schaber M.D., Skalka A.M., Ganguly K., Wong-Staal F., Reddy E.P. (1086) HTLV-III *gag* protein is processed in yeast cells by the virus *pol*-protease.

Kreil G. (1990) Processing of precursors by dipeptidylaminopeptidases: a case of molecular ticketing. Trends Biochem. Sci. 15, 23.

Kuchler K., Sterne R.E., Thorner J. (1989) *Saccharomyces cerevisiae STE6* gene product: a novel pathway for protein export in eukaryotic cells. EMBO J. 8, 3973.

Kurjan, J., Herskowitz, I. (1982) Structure of a yeast pheromone gene (MF): a putative α-factor precursor contains four tandem copies of mature α-factor. Cell 30, 933-943.

Kurjan J. (1985) α-factor structural gene mutations in yeast: effects on α-factor production and mating. Mol. Cell. Biol. 5, 787-796.

Kyte and Doolittle (1982) A simple method for displaying the hydropathic character of a protein. J. Molec. Biol. 157, 105-132.

Lammie G.A., Fant1 V., Smith R., Schuuring E., Brookes S., Michalides R., Dickson C., Arnold A. Peters G. (1991) D11S287, a putative oncogene on chromosome 11q13, is amplified and expressed in squamous cell and mammary carcinomas and linked to BCL-1. Oncogene 6, 439-444.

Lee K.S., Irie K., Gotoh Y., Watanabe Y., Arakai H., Nishida E., Matsumoto K., Levin D.E. (1993) A yeast mitogen-activated protein kinase homolog (Mpklp) mediates signalling by protein kinase C. Mol. Cell. Biol. 13, 3067-3075.

Lee M.G. and Nurse P. (1987) Complementation used to clone a human homologue of the fission yeast cell cycle control gene *cdc2.* Nature 327, 31-35.

Lew D.J., Dulic V., Reed S.I. (1991) Isolation of three novel human cyclins by rescue of G1 cyclin (Cln) function in yeast. Cell 66, 1197-1206.

Ma J., Przibilla J., Bogorad L., Ptashne M. (1988) Yeast activators stimulate plant gene expression. Nature 334, 631.

Manney T.R., Duntze W., Betz R. (1981) The isolation, characterization, and physiological effects of the S. *cerevisiae* sex pheromones. In *Sexual interactions in eukaryotic microbes* (ed. D.H. O'Day et al.), p 21. Academic Press, New York.

Matsushime H., Roussel M.F., Ashmun R.A., Scherr C.J. (1991) Colony-stimulating factor 1 regulates novel cyclins during the G1 phase of the cell cycle. Cell 65, 701-713.

McDonnell D.P., Nawaz Z., Densmore C., Weigel N.L. et al. (1991) High level expression of biologically active estrogen receptor in *Saccharomyces cerevisiae.* J. Steroid Biochem. Mol. Biol. 39, 291-297.

Metzger, D., Losson R., Bornert J.M., Lemoine Y., Chambon P. (1992) Promoter specificity of the two transcriptional activation functions of the human oestrogen receptor in yeast. Nuc. Acids Res. 20, 2813-2817.

Metzger D., White J.H., Chambon P. (1988) The human oestrogen receptor functions in yeast. Nature 334, 31.

Mimura C.S., Holbrook S.R., Ames G.F.-L. (1991) Structural model of the nucleotide binding conserved component of periplasmic permeases. Proc. Natl. Acad. Sci. 88, 84-88.

Moir DT; Davidow LS. (1991) Production of proteins by secretion from yeast. Methods Enzymol 194, 491-507.

Motokura T., Bloom T., Kim H.G., Juppner H., Ruderman J.V., Kronenberg H.M., Arnold A. (1991) A novel cyclin encoded by a bcl1-linked candidate oncogene. Nature 350, 512-515.

Moye-Rowley W.S., Harshman K.D., Parker C.S. (1989) Yeast YAP1 encodes a novel form of the jun family of transcriptional activator proteins. Genes Dev. 3, 283.

Nakagawa T., Hosaka M., Torii S., Watanabe T. et al. (1993) Identification and functional expression of a new member of the mammalian Kex-2-like processing endoprotease family: its striking structural similarity to PACE4. J. Biochem. (Tokyo) 113, 132-135.

Nakayama K., Hosaka M., Hatsuzawa K., Murakami K. (1991) Cloning and functional expression of a novel endoprotease involved in prohormone processing at dibasic sites. J. Biochem. 109, 803-806.

Nakayama K., Kim W.S., Torii S., Hosaka M. et al. (1992) Identification of a fourth member of the mammalian endoprotease family homologous to the yeast Kex2 protease. Its testis specific expression. J. Biol. Chem. 267, 5897-5900.

Nakayama N., Miyajima A., Arai K. (1985) Nucleotide sequences of STE2 and STE3, cell type-specific sterile genes from *Saccharomyces cerevisiae.* EMBO J. 4, 2643.

Nash R., Tokiwa G, Awand S., Erickson K., Futcher A.B. (1988) WHI1+ gene of Saccharomyces cerevisiae tethers division to cell size and is a cyclin homolog. EMBO J. 7, 4335-4346.

Neiman A.M., Stevenson B.J., Xu H.P., Sprague G.F. et al. (1993) Functional homology of protein kinases required for sexual differentiation in *Schizosaccharomyces pombe* and *Saccharomyces cerevisiae* suggests a conserved signal transduction module in eukaryotic organisms. Mol. Biol. Cell. 4, 107-120.

Neote, K. DiGregorio, D., Mak, J. Y., Horuk, R., and Schall, T. J. (1993) Molecular cloning, functional expression, and signaling characteristics of a C-C chemokine receptor. Cell 72, 415-425.

Norman C., Runswick M., Pollock R., Treisman R. (1988) Isolation and properties of cDNA clones encoding SRF, a transcription factor that binds to the *c-fos* serum response element. Cell 55, 989.

Oeda, K., Sakaki, T., Ohkawa, H. (1985) Expression of rat liver cytochrome P-450MC cDNA in *Saccharomyces cerevisiae.* DNA 3, 203-210.

Ogden, J.E., Stanway, C., Kuim, S.,Mellor, J., Kingsman, A.J., and Kingsman, S.M. (1986) Efficient expression of the *Saccharomyces cerevisiae* PGK gene depends on an upstream activation sequence but does not require TATA sequences. Mol. Cell. Biol. 6,4335.

Overduin P., Boos W., Tomassen J. (1988) Nucleotide sequence of the *ugp* genes of *Escherichia coli* K-12: homology to the maltose system. Mol. Microbiol. 2, 767-775.

Peterson M.G., Tanese N., Pugh B.F., Tjian R. (1990) Functional domains and upstream activation properties of cloned human TATA binding protein. Science 248, 1625.

Pines J. and Hunter T. (1990) Human cyclin A is adenovirus E1A-associated protein p60 and behaves differently from cyclin B. Nature 346, 760-763.

Reyes M., Treptow M.A., Schuman H.A. (1986) Transport of p-nitrophenyl--maltoside by the maltose transport system of *Escherichia coli* and its subsequent hydrolysis by a cytoplasmicα -maltosidase. J. Bacteriol. 165, 918-922.

Rogers S., Wells R., Rechsteiner M. (1986) Amino acid sequences common to rapidly degraded proteins: the PEST hypothesis. Science 234, 364-368.

Scharer, E. and R. Iggo (1992). "Mammalian p53 can function as a transcription factor in yeast." Nuc. Acids Res. 20 (7): 1539-1545.

Schafer W.R., Kim R., Sterne R., Thorner J., Kim S.-H., Rine J. (1989) Genetic and pharmacological suppression of oncogenic mutations in RAS genes of yeast and humans. Science 245, 379.

Schafer W.R., Trueblood C.E., Yang C.-C., Maayer M.P., Rosenberg S., Poulter C.D., Kim S.-H., Rine J. (1990) Enzymatic coupling of cholesterol intermediates to a mating pheromone precursor and to the Ras protein. Science 249, 1133.

Schena M. and Yamamoto K.R. (1988) Mammalian glucocorticoid receptor derivatives enhance transcription in yeast. Science 241, 965.

Scherr C.J. (1993) Mammalian G1 cyclins. Cell 73, 1059-1065.

Schultz R.M., Silberman S., Persky B. et al. (1988) Inhibition by human recombinant tissue inhibitor of metalloproteinases of human amnion invasion and lung colonization by murine B16-F10 melanoma cells. Cancer Res. 48, 5539.

Seideh N.G., Fournier H., Boileau G., Benjannet S. et al. (1992) The cDNA structure of the procine pro-hormone convertase PC2 and the comparative processing by PC1 and PC2 of the N-terminal glycopeptide segment of porcine POMC. FEBS Lett., 310, 235-239.

Singh A., Chen E.Y., Lugovoy J.M., Chang C.N., Hitzman R.A., Seeburg P.H. (1983) *Saccharomyces cerevisiae* contains two discrete genes coding for the α-factor pheromone. Nuc. Acids Res. 11, 4049-4063.

Smeekens S.P. and Steiner D.F. (1990) Identification of a human insulinoma cDNA encoding a novel mammalian protein structurally related to the yeast dibasic processing protease Kex2. J. Biol. Chem. 265, 2997-3000.

Smith R.A., Sisk R., Lockhart P., Mathewes S. et al. (1993) Isolation of glucagon antagonists by random molecular mutagenesis and screening. Mol. Pharmacol. 43, 741-748.

Steube K; Chaudhuri B; Marki W; Merryweather JP; Heim J. α-factor-leader-directed secretion of recombinant humaninsulin-like growth factor I from Saccharomyces cerevisiae. Precursor formation and processing in the yeast secretory pathway. (1991) Eur J Biochem 198, 651-657.

Strubin M. and Struhl K. (1992) Yeast and human TFIID with altered DNA-binding specificity for TATA elements. Cell 68, 721-730.

Struhl, K. (1986) Constitutive and inducible *Saccharomyces cerevisiae* promoters: Evidence for two distinctive molecular mechanisms. Mol. Cell. Biol. 6, 3847.

Struhl K. (1989) Molecular mechanisms of transcriptional regulation in yeast. Annu. Rev. Biochem. 58, 1051.

Takahashi, H., Hakamata Y., Watanabe Y., Kikuno R. et al. (1983) Complete nucleotide sequence of the human corticotropin-betalipotropin precursor gene. Nucleic Acids Research 11:6847-6858.

Thomas G., Thorne B.A., Thomas L., Allen R.G., Hruby D.E., Fuller R., Thorner J. (1988) Yeast KEX2 endopeptidase correctly cleaves a neuroendocrine prohormone in mammalian cells. Science 241, 226.

Thomas L., Cooper A., Bussey H., Thomas G. (1990) Yeast KEX1 protease cleaves a prohormone processing intermediate in mammalian cells. J. Biol. Chem. 265, 10821.

Valdiva R.H., Wang L., Winans S.C. (1991) Characterization of a putative periplasmic transport system for octopine accumulation encoded by Agrobacterium tumefaciens T: plasmid pTi46. J. Bacteriol. 173, 6398-6405.

Vogt P.K., Bos T.J., Doolittle R.F. (1987) Homology between the DNA-binding domain of the GCN4 regulatory protein of yeast and the carboxy-terminal region of a protein coded for by the oncogene jun. Proc. Natl. Acad. Sci. 84, 3316.

Waters M.G., Evans E.A., Blobel G. (1988) Prepro-α-factor has a cleavable signal sequence. J. Biol. Chem. 263, 6209.

Webster N., Jin J.R., Green S., Hollis M., Chambon P. (1988) The yeast UAS_{G} is a transcriptional enhancer in human HeLa cells in the presence of the GAL4 transactivator. Cell 52, 169.

Wood C.R., Boss M.A., Kenten J.H., Calvert J.E., Roberts N.A., Emtage J.S. (1985) The synthesis and *in vivo* asembly of functional antibodies in yeast. Nature 314, 446-449.

Xiong Y., Connolly T., Futcher B., Beach D. (1991) Human D-type cyclin. Cell 65, 691-699.

## Claims

1. A yeast cell culture comprising a plurality of yeast cells, each cell of which comprises:
(a) a first heterologous gene encoding a heterologous surrogate of a yeast pheromone system protein, said surrogate capable of performing in the pheromone system of the yeast cell a function naturally performed by the corresponding yeast pheromone system protein, and
(b) a second heterologous gene encoding a heterologous peptide,
said yeast culture collectively expressing a library of heterologous peptides, wherein interaction of a heterologous peptide within the library with said surrogate modulates interaction of said surrogate with said pheromone system and this modulation is a selectable or screenable event.

2. A process for producing a yeast cell having a pheromone system, which cell comprises:
(a) a first heterologous gene encoding a heterologous surrogate of a yeast pheromone system protein, said surrogate performing in the pheromone system of the yeast cell a function naturally performed by the corresponding yeast pheromone system protein, and
(b) a second heterologous gene encoding a heterologous peptide, wherein said heterologous peptide modulates the interaction of said surrogate with said pheromone system in the yeast cell and said modulation is a selectable or screenable event,
the process comprising the step of screening or selecting said cell from the yeast culture of claim 1.

3. The yeast cell culture of claim 1 or the process of claim 2 wherein the endogenous pheromone system protein is not produced in functional form.

4. The yeast cell culture of claim 1 or the process of claim 2, wherein the heterologous peptide is secreted by the cell into the periplasmic space, from which it interacts with said surrogate.

5. The yeast cell culture or process of claim 4 wherein the heterologous peptide is expressed in the form of a precursor peptide comprising a yeast cleavable leader peptide and a mature peptide, which leader peptide directs secretion of said heterologous peptide.

6. The yeast cell culture or process of claim 5 wherein the leader peptide corresponds to a leader peptide of the *Saccharomyces cerevisiae* α-factor or a-factor.

7. The yeast cell culture or process of claim 5 in which a wild-type pheromone of the yeast pheromone system is not secreted.

8. The yeast cell culture or process of claim 4 wherein the heterologous peptide is also expressed in a nonsecretory form.

9. The yeast cell culture of claim 1 or process of claim 2 wherein the cell is a mutant strain less susceptible than the wild-type strain to desensitization of its pheromone signal pathway by repeated or prolonged stimulation thereof.

10. The yeast cell culture or process of claim 9 in which the SST2 gene is not functionally expressed.

11. The yeast cell culture of claim 1 or process of claim 2, in which the endogenous FAR1 gene is not functionally expressed.

12. The yeast cell culture of claim 1 or process of claim 2, further comprising a selectable marker that is activated by the pheromone signal pathway.

13. The yeast cell culture or process of claim 12, said selectable marker comprising reporter gene including a pheromone-responsive promoter operably linked to a foreign selectable gene.

14. The yeast cell culture or process of claim 13 wherein the selectable gene is an IGP dehydratase gene.

15. The yeast cell culture or process of claim 13, wherein the homologous wild-type promoter is the FUS1 promoter.

16. The yeast cell cullture of claim 1 or process of claim 2 wherein the cell(s) belong to the species Saccharomyces cerevisiae.

17. The yeast cell cullture of claim 1 or process of claim 2, in which the pheromone system protein is a farnesyltransferase.

18. The yeast cell culture of claim 1 or process of claim 2, in which the pheromone system protein is a carboxymethyltransferase.

19. The yeast cell culture of claim 1 or process of claim 2, in which the pheromone system protein is a kinase.

20. The yeast cell culture of claim 1 or process of claim 2, wherein the yeast pheromone system protein is a protease involved in the production of the mature form of the yeast pheromone, through the cleavage of a precursor protein, and the surrogate is also a protease.

21. The yeast cell culture or process of claim 20, wherein the precursor protein produced in the cell(s) is itself a surrogate of the yeast pheromone precursor protein, and said surrogate precursor protein has an amino acid sequence comprising a recognition site recognized by the surrogate protease, said recognition site differing from that recognized by the yeast pheromone system protease, but said surrogate precursor protein is cleaved by the surrogate protease to produce the mature form of the yeast pheromone.

22. The yeast cell culture or process of claim 21, wherein the wild-type yeast pheromone precursor protein is not produced in said cell(s).

23. The yeast cell culture of claim 1 or process of claim 2, wherein the yeast pheromone system protein is an ABC transporter involved in the membrane transport of the yeast pheromone, and the surrogate is also an ABC transporter.

24. The yeast cell culture or process of claim 23, wherein the surrogate transports the yeast pheromone unless the peptide interferes with such transport.

25. The yeast cell culture or process of claim 23, wherein the surrogate transports the yeast pheromone only with the aid of said peptide.

26. The yeast cell culture of claim 1 or process of claim 2, wherein the yeast pheromone system protein is the yeast pheromone receptor.

27. The yeast cell culture or process of claim 26 in which in at least one cell the peptide is an agonist for the surrogate receptor.

28. The yeast cell culture or process of claim 26 in which in at least one cell the peptide is an antagonist for the surrogate receptor.

29. The yeast cell culture or process of claim 26, wherein the yeast cell(s) further comprise a chimeric Gα subunit of a G protein.

30. The yeast cell culture or process of claim 29, wherein the amino terminal portion of the chimeric Gα subunit is homologous with the Gα subunit of a yeast G protein and the remainder is homologous with the corresponding portion a Gα subunit of a heterologous G protein.

31. The yeast cell culture of claim 1 or process of claim 2, in which the pheromone system protein is a cyclin.

32. The yeast cell culture or process of claim 31, where said yeast cell further expresses a non- pheromone-responsive screenable marker different from said peptide.

33. A method of assaying a peptide for modulation of the activity of a non-yeast surrogate for a pheromone system protein which comprises providing a culture of yeast cells according to any of claims 1 and 3 to 32 which cells functionally express said surrogate and said heterologous peptide, and determining by detecting a change in said selectable or screenable event whether the pheromone signal pathway is activated or inhibited by interaction of said surrogate and said peptide.

34. The method of claim 33 in which the cell s comprise a pheromone-responsive selectable marker, and cell s are selected for expression of a different peptide having the desired activating or inhibiting effect.

35. The method of claim 33 in which the cell s comprise a pheromone-responsive screenable marker, and cell s are screened for expression of a peptide having the desired activating or inhibiting effect.

36. A method of assaying a peptide library for activity of a non-yeast pheromone system protein surrogate which comprises providing a yeast culture according to claim 1 or any one of claims 3-32 whose cells functionally express said surrogate and a heterologous peptide of said library, said culture collectively expressing said peptide library, and determining by detecting a change in said selectable or screenable event whether the pheromone signal pathway is activated or inhibited by interaction of said surrogate and said peptides in each of the cells of said culture.

37. The method of claim 34, in which the surrogate is human Mdr1, the cells grow on histidine-free media only if the surrogate transports α-factor, the cells are galactose-sensitive only if the surrogate transports α-factor, and endogenous pleiotropic drug resistance genes have been inactivated.

38. The cell culture or process of any of claims 1-32 wherein the heterologous peptide is 5 to 50 amino acids in length.

## Patentansprüche

1. Hefezell-Kultur umfassend eine Vielzahl von Hefezellen, wobei jede Zelle von dieser folgendes umfaßt:
(a) ein erstes heterologes Gen, das ein heterologes Surrogat eines Proteins des Hefe-Pheromon-Systems kodiert, wobei das Surrogat dazu in der Lage ist, im Pheromon-System der Hefezelle eine Funktion durchzuführen, die natürlicherweise durch das entsprechende Protein des Hefe-Pheromon-Systems durchgeführt wird, und
(b) ein zweites heterologes Gen, das ein heterologes Peptid kodiert,
wobei die Hefe-Kultur eine Bibliothek heterologer Peptide exprimiert, wobei eine Interaktion eines heterologen Peptids innerhalb der Bibliothek mit dem Surrogat die Interaktion des Surrogats mit dem Pheromon-System moduliert und diese Modulierung ein selektierbares oder screenbares Ereignis ist.

2. Verfahren zur Herstellung einer Hefezelle, die ein Pheromon-System aufweist, wobei die Zelle folgendes umfaßt:
(a) ein erstes heterologes Gen, das ein heterologes Surrogat eines Proteins des Hefe-Pheromon-Systems kodiert, wobei das Surrogat im Pheromon-System der Hefezelle eine Funktion durchführt, die natürlicherweise durch das entsprechende Hefe-Pheromon-System-Protein durchgeführt wird, und
(b) ein zweites heterologes Gen, das ein heterologes Peptid kodiert, wobei das heterologe Peptid die Interaktion des Surrogats mit dem Pheromon-System in der Hefezelle moduliert und die Modulierung ein selektierbares oder screenbares Ereignis ist, wobei das Verfahren den Schritt umfaßt, die Zelle aus der Hefe-Kultur nach Anspruch 1 zu screenen oder zu selektieren.

3. Hefezell-Kultur nach Anspruch 1 oder Verfahren nach Anspruch 2,
bei dem das endogene Pheromon-System-Protein nicht in funktioneller Form erzeugt wird.

4. Hefezell-Kultur nach Anspruch 1 oder Verfahren nach Anspruch 2,
bei dem das heterologe Peptid durch die Zelle in den periplasmatischen Raum sezerniert wird, von dem aus es mit dem Surrogat interagiert.

5. Hefezell-Kultur oder Verfahren nach Anspruch 4,
bei dem das heterologe Peptid in Form eines Vorläufer-Peptids exprimiert wird, das ein spaltbares Hefeleit-Peptid und ein reifes Peptid umfaßt, wobei das Leit-Peptid die Sekretion des heterologen Peptids lenkt.

6. Hefezell-Kultur oder Verfahren nach Anspruch 5,
bei dem das Leit-Peptid einem Leit-Peptid des Saccharomyces cerevisiae α-Faktor oder a-Faktor entspricht.

7. Hefezell-Kultur oder Verfahren nach Anspruch 5,
bei dem ein Wild-Typ-Pheromon des Hefe-Pheromon-Systems nicht sezerniert wird.

8. Hefezell-Kultur oder Verfahren nach Anspruch 4,
bei dem das heterologe Peptid ebenfalls in einer nicht-sekretorischen Form exprimiert wird.

9. Hefezell-Kultur nach Anspruch 1 oder Verfahren nach Anspruch 2,
bei dem die Zelle ein mutantierter Stamm ist, der weniger als der Wild-Typ-Stamm gegen eine Desensibilisierung seines Pheromon-Signalweges durch wiederholte oder verlängerte Stimulation hiervon empfindlich ist.

10. Hefezell-Kultur oder Verfahren nach Anspruch 9,
bei dem das SST2-Gen nicht funktionell exprimiert wird.

11. Hefezell-Kultur nach Anspruch 1 oder Verfahren nach Anspruch 2,
bei dem das endogene FAR1-Gen nicht funktionell exprimiert wird.

12. Hefezell-Kultur nach Anspruch 1 oder Verfahren nach Anspruch 2,
weiterhin umfassend einen selektierbaren Marker, der durch den Pheromon-Signalweg aktiviert wird.

13. Hefezell-Kultur oder Verfahren nach Anspruch 12,
wobei der selektierbare Marker ein Reportergen umfaßt, das einen Pheromon-responsiven Promotor einschließt, der funktionell an ein fremdes selektierbares Gen gebunden ist.

14. Hefezell-Kultur oder Verfahren nach Anspruch 13,
bei dem das selektierbare Gen ein IGP-Dehydratase-Gen ist.

15. Hefezell-Kultur oder Verfahren nach Anspruch 13,
bei dem der homologe Wild-Typ-Promotor der FUS1-Promotor ist.

16. Hefezell-Kultur nach Anspruch 1 oder Verfahren nach Anspruch 2,
bei dem die Zelle(n) zur Spezies Saccharomyces cerevisiae gehören.

17. Hefezell-Kultur nach Anspruch 1 oder Verfahren nach Anspruch 2,
bei dem das Protein des Pheromon-Systems eine Farnesyltransferase ist.

18. Hefezell-Kultur nach Anspruch 1 oder Verfahren nach Anspruch 2,
wobei das Protein des Pheromon-Systems eine Carboxymethyltransferase ist.

19. Hefezell-Kultur nach Anspruch 1 oder Verfahren nach Anspruch 2,
wobei das Protein des Pheromon-Systems eine Kinase ist.

20. Hefezell-Kultur nach Anspruch 1 oder Verfahren nach Anspruch 2,
wobei das Hefe-Pheromon-System-Protein eine Protease ist, die in die Erzeugung der reifen Form des Hefe-Pheromons durch die Spaltung eines Vorläufer-Proteins involviert ist, und das Surrogat ebenfalls eine Protease ist.

21. Hefezell-Kultur oder Verfahren nach Anspruch 20,
wobei das Vorläufer-Protein, das in der Zelle(n) erzeugt wird, selbst ein Surrogat des Hefe-Pheromon-Vorläufer-Proteins ist, und wobei das Surrogat-Vorläufer-Protein eine Aminosäuresequenz aufweist, die eine Erkennungsstelle umfaßt, die durch die Surrogat-Protease erkannt wird, wobei die Erkennungsstelle sich von derjenigen, die durch die Hefe-Pheromon-System-Protease erkannt wird, unterscheidet, wobei jedoch das Surrogat-Vorläufer-Protein durch die Surrogat-Protease zur Erzeugung der reifen Form des Hefe-Pheromons gespalten wird.

22. Hefezell-Kultur oder Verfahren nach Anspruch 21,
wobei das Pheromon-Vorläufer-Protein der Wild-Typ-Hefe nicht in der (den) Zelle(n) erzeugt wird.

23. Hefezell-Kultur nach Anspruch 1 oder Verfahren nach Anspruch 2,
wobei das Protein des Hefe-Pheromon-Systems ein ABC-Transporter ist, der in den Membrantransport des Hefe-Pheromons involviert ist, und das Surrogat ebenfalls ein ABC-Transporter ist.

24. Hefezell-Kultur oder Verfahren nach Anspruch 23,
wobei das Surrogat das Hefe-Pheromon transportiert, solange bis das Peptid einen derartigen Transport stört.

25. Hefezell-Kultur oder Verfahren nach Anspruch 23,
wobei das Surrogat das Hefe-Pheromon nur mit Hilfe des Peptids transportiert.

26. Hefezell-Kultur nach Anspruch 1 oder Verfahren nach Anspruch 2,
wobei das Protein des Hefe-Pheromon-Systems der Hefe-Pheromon-Rezeptor ist.

27. Hefezell-Kultur oder Verfahren nach Anspruch 26,
wobei in zumindest einer Zelle das Peptid ein Agonist für den Surrogat-Rezeptor ist.

28. Hefezell-Kultur oder Verfahren nach Anspruch 26,
bei der zumindest in einer Zelle das Peptid ein Antagonist des Surrogat-Rezeptors ist.

29. Hefezell-Kultur oder Verfahren nach Anspruch 26,
bei dem die Hefe-Zelle(n) weiterhin eine chimäre Gα-Untereinheit eines G-Proteins umfaßt.

30. Hefezell-Kultur oder Verfahren nach Anspruch 29,
bei der der amino-terminale Anteil der chimären Gα-Untereinheit mit der Gα-Untereinheit eines Hefe-G-Proteins homolog ist und der Rest mit dem entsprechenden Anteil einer Gα-Untereinheit eines heterologen G-Proteins homolog ist.

31. Hefezell-Kultur nach Anspruch 1 oder Verfahren nach Anspruch 2,
bei dem das Protein des Pheromon-System ein Cyclin ist.

32. Hefezell-Kultur oder Verfahren nach Anspruch 31,
wobei die Hefe-Zelle weiterhin einen nicht-pheromon-responsiven screenbaren Marker exprimiert, der von dem Peptid verschieden ist.

33. Verfahren der Untersuchung eines Peptides zur Modulierung bezüglich der Aktivität des Nicht-Hefe-Surrogats für ein Protein des Pheromon-Systems, das ein Bereitstellen einer Kultur von Hefe-Zellen nach einem der Ansprüche 1 und 3 bis 32 umfaßt, wobei die Zellen das Surrogat und das heterologe Peptid funktionell exprimieren, und ein Bestimmen durch Nachweis einer Veränderung des selektierbaren oder screenbaren Ereignisses umfaßt, ob der Pheromon-Signalweg durch Interaktion mit dem Surrogat oder dem Peptid aktiviert oder gehemmt ist.

34. Verfahren nach Anspruch 33,
bei dem die Zellen einen pheromon-responsiven, selektierbaren Marker umfassen, und wobei die Zellen zur Expression eines unterschiedlichen Peptides selektiert sind, das die erwünschte aktivierende oder hemmende Wirkung aufweist.

35. Verfahren nach Anspruch 33,
bei dem die Zellen einen pheromon-responsiven, screenbaren Marker umfassen, und wobei die Zellen bezüglich einer Expression eines Peptids gescreent werden, das die gewünschte aktivierende oder hemmende Wirkung aufweist.

36. Verfahren der Untersuchung einer Peptid-Bibliothek bezüglich der Aktivität eines Protein-Surrogats des Nicht-Hefe-Pheromon-Systems, das ein Bereitstellen einer Hefe-Kultur nach Anspruch 1 oder einem der Ansprüche 3 bis 32 umfaßt, deren Zellen funktionell das Surrogat und ein heterologes Peptid der Bibliothek exprimieren, wobei die Kultur kollektiv die Peptid-Bibliothek exprimiert, und Bestimmen durch Nachweis einer Veränderung in dem selektierbaren oder screenbaren Ereignis, ob der Pheromon-Signalweg durch Interaktion des Surrogats und der Peptide in jeder der Zellen der Kultur aktiviert oder gehemmt wird.

37. Verfahren nach Anspruch 34,
bei dem das Surrogat menschliches Mdr1 ist, die Zellen auf histidin-freien Medien nur wachsen, wenn das Surrogat einen α-Faktor transportiert, wobei die Zellen nur galactosesensitiv sind, wenn das Surrogat einen α-Faktor transportiert, und bei dem die endogenen pleiotropen Arzneistoff-Resistenzgene inaktiviert wurden.

38. Zellkultur oder Verfahren nach einem der Ansprüche 1-32, bei dem das heterologe Peptid 5 bis 50 Aminosäuren lang ist.

## Revendications

1. Une culture cellulaire de levure comprenant une pluralité de cellules de levure, dont chaque cellule comprend:
(a) un premier gène hétérologue codant pour un substitut hétérologue d'une protéine du système de phéromones d'une levure, ce substitut capable de mettre en oeuvre dans le système de phéromones de la cellule de levure une fonction effectuée naturellement par la protéine du système de phéromones de la levure correspondante, et
(b) un second gène hétérologue codant pour un peptide hétérologue, cette culture de levure exprimant de manière collective une librairie de peptides hétérologues, dans laquelle une interaction d'un peptide hétérologue à l'intérieur de la librairie avec ce substitut module l'interaction de ce substitut avec ce système de phéromones et cette modulation est un événement sélectionnable ou capable d'être dépisté.

2. Procédé pour produire une cellule de levure possédant un système de phéromones, laquelle cellule comprend:
(a) un premier gène hétérologue codant pour un substitut hétérologue d'une protéine d'un système de phéromones de levure, ce substitut mettant en oeuvre dans le système de phéromones de la cellule de levure une fonction effectuée naturellement par la protéine correspondante du système de phéromones de levure, et
(b) un second gène hétérologue codant pour un peptide hétérologue, dans lequel ce peptide hétérologue module l'interaction de ce substitut avec ce système de phéromones dans la cellule de levure et cette modulation est un événement sélectionnable ou capable d'être dépisté, le procédé comprenant l'étape de dépistage ou de sélection de cette cellule à partir de la culture de cellule de la revendication 1.

3. Culture de cellules de levure selon la revendication 1 ou procédé selon la revendication 2, dans lequel la protéine du système de phéromones endogènes n'est pas produite sous forme fonctionnelle.

4. Culture de cellules de levure selon la revendication 1 ou procédé selon la revendication 2, dans lequel le peptide hétérologue est sécrété par la cellule dans l'espace périplasmique, à partir duquel il interagit avec ce substitut.

5. Culture de cellules de levure ou procédé selon la revendication 4 dans lequel le peptide hétérologue est exprimé sous forme d'un peptide précurseur comprenant un peptide leader de levure capable d'être clivé et un peptide mature, lequel peptide leader commande la sécrétion de ce peptide hétérologue.

6. Culture de cellules de levure ou procédé selon la revendication 5 dans lequel le peptide leader correspond à un peptide leader d'un facteur ou d'un facteur a de Saccharomyces cerevisiae.

7. Culture de cellules de levure ou procédé selon la revendication 5 dans lequel une phéromone de type sauvage du système de phéromones de levure n'est pas sécrétée.

8. Culture de cellules de levure ou procédé selon la revendication 4 dans lequel le peptide hétérologue est aussi exprimé sous une forme non sécrétoire.

9. Culture de cellules de levure selon la revendication 1 ou procédé selon la revendication 2 dans lequel la cellule est un brin mutant moins susceptible à la désensibilisation de son mécanisme à signal de phéromones par une stimulation répétée ou prolongée de celui-ci que le brin du type sauvage.

10. Culture de cellules de levure ou procédé selon la revendication 9 dans lequel le gène SST2 n'est pas exprimé de manière fonctionnelle.

11. Culture de cellules de levure selon la revendication 1 ou procédé selon la revendication 2, dans lequel le gène endogène FAR1 n'est pas exprimé de manière fonctionnelle.

12. Culture de cellules de levure selon la revendication 1 ou procédé selon la revendication 2, comprenant en outre un marqueur de sélection qui est activé par le mécanisme à signal de phéromones.

13. Culture de cellules de levure ou procédé selon la revendication 12, ce marqueur de sélection comprenant un gène rapporteur englobant un promoteur de réponse aux phéromones lié de manière opérationnelle à un gène de sélection étranger.

14. Culture de cellules de levure ou procédé selon la revendication 13, dans lequel le gène de sélection est un gène IGP déshydratase.

15. Culture de cellules de levure ou procédé selon la revendication 13, dans lequel le promoteur homologue de type sauvage est le promoteur FUS1.

16. Culture de cellules de levure selon la revendication 1 ou procédé selon la revendication 2 dans lequel la/les cellule(s) appartient/appartiennent à l'espèce Saccharomyces cerevisiae.

17. Culture de cellules de levure selon la revendication 1 ou procédé selon la revendication 2, dans lequel la protéine du système de phéromones est une farnésyltransférase.

18. Culture de cellules de levure selon la revendication 1 ou procédé selon la revendication 2, dans lequel la protéine du système de phéromones est une carboxyméthyltransférase.

19. Culture de cellules de levure selon la revendication 1 ou procédé selon la revendication 2, dans lequel la protéine du système de phéromones est une kinase.

20. Culture de cellules de levure selon la revendication 1 ou procédé selon la revendication 2, dans lequel la protéine du système de phéromones de levure est une protéase impliquée dans la production de la forme mature de la phéromone de levure, par le clivage d'une protéine précurseur, et le substitut est aussi une protéase.

21. Culture de cellules de levure ou procédé selon la revendication 20, dans lequel la protéine précurseur produite dans la/les cellule(s) est/sont elle(s)-même(s) un substitut de la protéine précurseur de phéromones de levure, et ce substitut de la protéine précurseur possède une séquence d'acides aminés comprenant un site de reconnaissance reconnu par la protéase substitut, ce site de reconnaissance différent de celui reconnu par la protéase du système de phéromones de levure, mais cette protéine précurseur du substitut est clivée par la protéase substitut pour produire la forme mature de la phéromone de levure.

22. Culture de cellules de levure ou procédé selon la revendication 21, dans lequel la protéine de précurseur de phéromones de levure du type sauvage n'est pas produite dans cette/ces cellule(s).

23. Culture de cellules de levure selon la revendication 1 ou procédé selon la revendication 2, dans lequel la protéine du système de phéromones de levure est un transporteur ABC impliqué dans le transport membranaire d'une phéromone de levure, et le substitut est aussi un transporteur ABC.

24. Culture de cellules de levure ou procédé selon la revendication 23, dans lequel le substitut transporte la phéromone de levure sans que le peptide n'interfère avec ce transport.

25. Culture de cellules de levure ou procédé selon la revendication 23, dans lequel le substitut transporte la phéromone de levure uniquement à l'aide de ce peptide.

26. Culture de cellules de levure selon la revendication 1 ou procédé selon la revendication 2, dans lequel la protéine du système de phéromones de levure est le récepteur d'une phéromone de levure.

27. Culture de cellules de levure ou procédé selon la revendication 26, dans lequel dans au moins une cellule le peptide est un agoniste pour le récepteur du substitut.

28. Culture de cellules de levure ou procédé selon la revendication 26, dans lequel dans au moins une cellule le peptide est un antagoniste pour récepteur du substitut.

29. Culture de cellules de levure ou procédé selon la revendication 26, dans lequel la/les cellule(s) de levure comprend/comprennent en outre une sous-unité chimère G d'une protéine G.

30. Culture de cellules de levure ou procédé selon la revendication 29, dans lequel la partie amino terminale de la sous-unité chimère G est homologue avec la sous-unité G d'une protéine G de levure et le reste est homologue à la partie correspondante d'une sous-unité G d'une protéine G hétérologue.

31. Culture de cellules de levure selon la revendication 1 ou procédé selon la revendication 2, dans lequel la protéine du système de phéromones est une cycline.

32. Culture de cellules de levure ou procédé selon la revendication 31, ou cette cellule le levure exprime en outre un marqueur non dépistable différent de ce peptide en réponse à une phéromone.

33. Méthode d'analyse d'un peptide pour moduler l'activité d'un substitut autre que de levure pour une protéine d'un système de phéromones qui comprend la fourniture d'une culture de cellules de levure selon l'une quelconque des revendications 1 et 3 à 32 lesquelles cellules expriment de manière fonctionnelle ce substitut et ce peptide hétérologue, et déterminent en détectant un changement dans cet événement sélectionnable ou capable d'être dépisté si le mécanisme à signal de phéromones est activé ou inhibé par l'interaction de ce substitut et ce peptide.

34. Méthode selon la revendication 33 dans laquelle les cellules comprennent un marqueur sélectionnable en réponse à une phéromone, et les cellules sont sélectionnées pour exprimer un peptide différent possédant l'effet d'activation ou d'inhibition souhaité.

35. Méthode selon la revendication 33 dans laquelle les cellules comprennent un marqueur sélectionnable en réponse à une phéromone, et les cellules sont dépistées pour exprimer un peptide possédant un effet d'activation ou d'inhibition souhaité.

36. Méthode d'analyse d'une librairie de peptides pour l'activité d'un substitut d'une protéine d'un système de phéromones autres que de levure qui comprend la fourniture d'une culture de levure selon la revendication 1 ou l'une quelconque des revendications 3 à 32, lesquelles cellules expriment de manière fonctionnelle ce substitut et un peptide hétérologue de cette librairie, cette culture exprime de manière collective cette librairie de peptides, et détermine en détectant un changement dans cet événement sélectionnable ou capable d'être dépisté si le mécanisme à signal de phéromones est activé ou inhibé par interaction de ce substitut et ces peptides dans chacune des cellules de cette culture.

37. Méthode selon la revendication 34, dans laquelle le substitut est du Mdrl humain, les cellules poussent sur un milieu sans histidine uniquement si le substitut transporte le facteur, les cellules sont sensibles au galactose uniquement si le substitut transporte le facteur, et les gènes endogènes pléiotropes de résistance aux drogues ont été inactivés.

38. Culture de cellule ou procédé selon l'une quelconque des revendications 1 à 32 dans lequel le peptide hétérologue possède une longueur de 5 à 50 acides aminés.
